(19) Europäisches Patentamt
European Patent Office
Office européen des brevets

(11) EP 0 873 998 A2

(12) # EUROPEAN PATENT APPLICATION

(43) Date of publication:
**28.10.1998 Bulletin 1998/44**

(51) Int Cl.⁶: **C07K 14/705**, C07K 16/28, C12N 15/12

(21) Application number: **98303190.7**

(22) Date of filing: **24.04.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **25.04.1997 JP 109798/97**
**17.09.1997 JP 251867/97**

(71) Applicant: **TAKEDA CHEMICAL INDUSTRIES, LTD.**
**Chuo-ku, Osaka 541 (JP)**

(72) Inventors:
- **Nishi, Kazunori**
**Chuo-ku, Osaka 541 (JP)**
- **Shintani, Atsushi**
**Chuo-ku, Osaka 541 (JP)**
- **Horiguchi, Takashi**
**Chuo-ku, Osaka 541 (JP)**

(74) Representative: **Giddings, Peter John, Dr.**
**SmithKline Beecham plc**
**Corporate Intellectual Property,**
**Two New Horizons Court**
**Brentford, Middlesex TW8 9EP (GB)**

(54) **Receptor protein and its use**

(57) A receptor protein derived from human dendritic cell, its partial peptide and their salts are disclosed. A production process of the receptor protein, an antibody against the receptor protein, a method for determination of a ligand to the receptor protein, a screening method and a screening kit for a compound which alters binding properties between a ligand and the receptor protein as well as a pharmaceutical composition of such compound are also disclosed. The receptor protein derived from human dendritic cell, its partial peptide and their salts are useful as reagents for screening ligands, agonists, antagonists and the like. The antibody is useful as a reagent for quantitative analysis of the receptor protein in a specimen fluid.

EP 0 873 998 A2

## Annex to the description

Sequence Listing

(1) GENERAL INFORMATION

(i) APPLICANT: Takeda Chemical Industries Ltd

(ii) TITLE OF THE INVENTION: Novel Receptor Protein and Its Use

(iii) NUMBER OF SEQUENCES: 18

(iv) CORRESPONDENCE ADDRESS:
(A) ADDRESSEE: SmithKline Beecham, Corporate Intellectual Property
(B) STREET: Two New Horizons Court
(C) CITY: Brentford
(D) COUNTY: Middlesex
(E) COUNTRY: United Kingdom
(F) POST CODE: TW8 9EP

(v) COMPUTER READABLE FORM:
(A) MEDIUM TYPE: Diskette
(B) COMPUTER: IBM Compatible

(vi) CURRENT APPLICATON DATA:
(A) APPLICATION NUMBER:
(B) FILING DATE:
(C) CLASSIFICATION

(vii) PRIOR APPLICATION DATA:
(A) APPLICATION NUMBER: 109798/1997
(B) FILING DATE: 25 April 1997
(A) APPLICATION NUMBER: 251867/1997
(B) FILING DATE: 17 September 1997

(viii) ATTORNEY/AGENT INFORMATION:
(A) NAME: GIDDINGS, Peter John
(B) GENERAL AUTHORISATION NUMBER:
(C) REFERENCE/DOCKET NUMBER: TAK50005

(ix) TELECOMMUNICATION INFORMATION:
(A) TLEEPHONE: +44 181 975 6331
(B) TELEFAX: +44 181 975 6294

(2) INFORMATION FOR SEQ ID NO: 1:
(i) SEQUENCE CHARACTERISTICS:
(A) LENGTH: 616
(B) TYPE: amino acid
(C) STRANDEDNESS:
(D) TOPOLOGY: linear
(ii) MOLECULAR TYPE: protein
(xi) SEQUENCE DESCRIPTION: SEQ ID NO: 1:

```
Met Ala Pro Arg Ala Arg Arg Arg Arg Pro Leu Phe Ala Leu Leu leu
 1               5                  10                  15
Leu Cys Ala Leu Leu Ala Arg Leu Gln Val Ala Leu Gln Ile Ala Pro
            20                  25                  30
Pro Cys Thr Ser Glu Lys His Tyr Glu His Leu Gly Arg Cys Cys Asn
        35                  40                  45
Lys Cys Glu Pro Gly Lys Tyr Met Ser Ser Lys Cys Thr Thr Thr Ser
    50                  55                  60
Asp Ser Val Cys Leu Pro Cys Gly Pro Asp Glu Tyr Leu Asp Ser Trp
65                  70                  75                  80
Asn Glu Glu Asp Lys Cys Leu Leu His Lys Val Cys Asp Thr Gly Lys
                85                  90                  95
Ala Leu Val Ala Val Val Ala Gly Asn Ser Thr Thr Pro Arg Arg Cys
            100                 105                 110
```

```
Ala Cys Thr Ala Gly Tyr His Trp Ser Gln Asp Cys Glu Cys Cys Arg
        115                 120                 125
Arg Asn Thr Glu Cys Ala Pro Gly Leu Gly Ala Gln His Pro Leu Gln
    130                 135                 140
Leu Asn Lys Asp Thr Val Cys Lys Pro Cys Leu Ala Gly Tyr Phe Ser
145                 150                 155                 160
Asp Ala Phe Ser Ser Thr Asp Lys Cys Arg Pro Trp Thr Asn Cys Thr
                165                 170                 175
Phe Leu Gly Lys Arg Val Glu His His Gly Thr Glu Lys Ser Asp Val
            180                 185                 190
Val Cys Ser Ser Ser Leu Pro Ala Arg Lys Pro Pro Asn Glu Pro His
        195                 200                 205
Val Tyr Leu Pro Gly Leu Ile Ile Leu Leu Leu Phe Ala Ser Val Ala
    210                 215                 220
Leu Val Ala Ala Ile Ile Phe Gly Val Cys Tyr Arg Lys Lys Gly Lys
225                 230                 235                 240
Ala Leu Thr Ala Asn Leu Trp His Trp Ile Asn Glu Ala Cys Gly Arg
                245                 250                 255
Leu Ser Gly Asp Lys Glu Ser Ser Gly Asp Ser Cys Val Ser Thr His
            260                 265                 270
Thr Ala Asn Phe Gly Gln Gln Gly Ala Cys Glu Gly Val Leu Leu Leu
        275                 280                 285
Thr Leu Glu Glu Lys Thr Phe Pro Glu Asp Met Cys Tyr Pro Asp Gln
    290                 295                 300
Gly Gly Val Cys Gln Gly Thr Cys Val Gly Gly Gly Pro Tyr Ala Gln
305                 310                 315                 320
Gly Glu Asp Ala Arg Met Leu Ser Leu Val Ser Lys Thr Glu Ile Glu
                325                 330                 335
Glu Asp Ser Phe Arg Gln Met Pro Thr Glu Asp Glu Tyr Met Asp Arg
            340                 345                 350
Pro Ser Gln Pro Thr Asp Gln Leu Leu Phe Leu Thr Glu Pro Gly Ser
        355                 360                 365
Lys Ser Thr Pro Pro Phe Ser Glu Pro Leu Glu Val Gly Glu Asn Asp
    370                 375                 380
Ser Leu Ser Gln Cys Phe Thr Gly Thr Gln Ser Thr Val Gly Ser Glu
385                 390                 395                 400
Ser Cys Asn Cys Thr Glu Pro Leu Cys Arg Thr Asp Trp Thr Pro Met
                405                 410                 415
Ser Ser Glu Asn Tyr Leu Gln Lys Glu Val Asp Ser Gly His Cys Pro
            420                 425                 430
His Trp Ala Ala Ser Pro Ser Pro Asn Trp Ala Asp Val Cys Thr Gly
        435                 440                 445
Cys Arg Asn Pro Pro Gly Glu Asp Cys Glu Pro Leu Val Gly Ser Pro
    450                 455                 460
Lys Arg Gly Pro Leu Pro Gln Cys Ala Tyr Gly Met Gly Leu Pro Pro
465                 470                 475                 480
Glu Glu Glu Ala Ser Arg Thr Glu Ala Arg Asp Gln Pro Glu Asp Gly
                485                 490                 495
Ala Asp Gly Arg Leu Pro Ser Ser Ala Arg Ala Gly Ala Gly Ser Gly
            500                 505                 510
Ser Ser Pro Gly Gly Gln Ser Pro Ala Ser Gly Asn Val Thr Gly Asn
        515                 520                 525
Ser Asn Ser Thr Phe Ile Ser Ser Gly Gln Val Met Asn Phe Lys Gly
    530                 535                 540
Asp Ile Ile Val Val Tyr Val Ser Gln Thr Ser Gln Glu Gly Ala Ala
545                 550                 555                 560
Ala Ala Ala Glu Pro Met Gly Arg Pro Val Gln Glu Glu Thr Leu Ala
                565                 570                 575
Arg Arg Asp Ser Phe Ala Gly Asn Gly Pro Arg Phe Pro Asp Pro Cys
            580                 585                 590
Gly Gly Pro Glu Gly Leu Arg Glu Pro Glu Lys Ala Ser Arg Pro Val
        595                 600                 605
Gln Glu Gln Gly Gly Ala Lys Ala
    610                 615
```

(2) INFORMATION FOR SEQ ID NO: 2
(i) SEQUENCE CHARACTERISTICS:
(A) LENGTH: 181
(B) TYPE: amino acid
(C) STRANDEDNESS:
(D) TOPOLOGY: linear
(ii) MOLECULAR TYPE: peptide
(xi) SEQUENCE DESCRIPTION: SEQ ID NO: 2:

```
Ile Ala Pro Pro Cys Thr Ser Glu Lys His Tyr Glu His Leu Gly Arg
1               5                   10                  15
Cys Cys Asn Lys Cys Glu Pro Gly Lys Tyr Met Ser Ser Lys Cys Thr
            20                  25                  30
Thr Thr Ser Asp Ser Val Cys Leu Pro Cys Gly Pro Asp Glu Tyr Leu
        35                  40                  45
Asp Ser Trp Asn Glu Glu Asp Lys Cys Leu Leu His Lys Val Cys Asp
    50                  55                  60
Thr Gly Lys Ala Leu Val Ala Val Val Ala Gly Asn Ser Thr Thr Pro
65                  70                  75                  80
Arg Arg Cys Ala Cys Thr Ala Gly Tyr His Trp Ser Gln Asp Cys Glu
                85                  90                  95
Cys Cys Arg Arg Asn Thr Glu Cys Ala Pro Gly Leu Gly Ala Gln His
            100                 105                 110
Pro Leu Gln Leu Asn Lys Asp Thr Val Cys Lys Pro Cys Leu Ala Gly
        115                 120                 125
Tyr Phe Ser Asp Ala Phe Ser Ser Thr Asp Lys Cys Arg Pro Trp Thr
    130                 135                 140
Asn Cys Thr Phe Leu Gly Lys Arg Val Glu His His Gly Thr Glu Lys
145                 150                 155                 160
Ser Asp Val Val Cys Ser Ser Ser Leu Pro Ala Arg Lys Pro Pro Asn
                165                 170                 175
Glu Pro His Val Tyr
            180
```

(2) INFORMATION FOR SEQ ID NO: 3
(i) SEQUENCE CHARACTERISTICS:
(A) LENGTH: 29
(B) TYPE: amino acid
(C) STRANDEDNESS:
(D) TOPOLOGY: linear
(ii) MOLECULAR TYPE: peptide
(xi) SEQUENCE DESCRIPTION: SEQ ID NO: 3:

```
Met Ala Pro Arg Ala Arg Arg Arg Arg Pro Leu Phe Ala Leu Leu leu
1               5                   10                  15
Leu Cys Ala Leu Leu Ala Arg Leu Gln Val Ala Leu Gln
            20                  25
```

(2) INFORMATION FOR SEQ ID NO: 4
(i) SEQUENCE CHARACTERISTICS:
(A) LENGTH: 1848
(B) TYPE: nucleic acid
(C) STRANDEDNESS: single
(D) TOPOLOGY: linear
(ii) MOLECULAR TYPE: cDNA
(xi) SEQUENCE DESCRIPTION: SEQ ID NO: 4:

```
ATGGCCCCGC GCGCCCGGCG GCGCCGCCCG CTGTTCGCGC TGCTGCTGCT CTGCGCGCTG   60
CTCGCCCCGC TGCAGGTGGC TTTGCAGATC GCTCCTCCAT GTACCAGTGA GAAGCATTAT  120
GAGCATCTGG GACGGTGCTG TAACAAATGT GAACCAGGAA AGTACATGTC TTCTAAATGC  180
ACTACTACCT CTGACAGTGT ATGTCTGCCC TGTGGCCCGG ATGAATACTT GGATAGCTGG  240
AATGAAGAAG ATAAATGCTT GCTGCATAAA GTTTGTGATA CAGGCAAGGC CCTGGTGGCC  300
GTGGTCGCCG GCAACAGCAC GACCCCCCGG CGCTGCGCGT GCACGGCTGG GTACCACTGG  360
AGCCAGGACT GCGAGTGCTG CCGCCGCAAC ACCGAGTGCG CGCCGGGCCT GGGCGCCCAG  420
CACCCGTTGC AGCTCAACAA GGACACAGTG TGCAAACCTT GCCTTGCAGG CTACTTCTCT  480
GATGCCTTTT CCTCCACGGA CAAATGCAGA CCCTGGACCA ACTGTACCTT CCTTGGAAAG  540
AGAGTAGAAC ATCATGGGAC AGAGAAATCC GATGTGGTTT GCAGTTCTTC TCTGCCAGCT  600
```

AGAAAACCAC CAAATGAACC CCATGTTTAC TTGCCCGGTT TAATAATTCT GCTTCTCTTC 660
GCGTCTGTGG CCCTGGTGGC TGCCATCATC TTTGGCGTTT GCTATAGGAA AAAAGGGAAA 720
GCACTCACAG CTAATTTGTG GCACTGGATC AATGAGGCTT GTGGCCGCCT AAGTGGAGAT 780
AAGGAGTCCT CAGGTGACAG TTGTGTCAGT ACACACACGG CAAACTTTGG TCAGCAGGGA 840
GCATGTGAAG GTGTCTTACT GCTGACTCTG GAGGAGAAGA CATTTCCAGA AGATATGTGC 900
TACCCAGATC AAGGTGGTGT CTGTCAGGGC ACGTGTGTAG GAGGTGGTCC CTACGCACAA 960
GGCGAAGATG CCAGGATGCT CTCATTGGTC AGCAAGACCG AGATAGAGGA AGACAGCTTC 1020
AGACAGATGC CCACAGAAGA TGAATACATG GACAGGCCCT CCCAGCCCAC AGACCAGTTA 1080
CTGTTCCTCA CTGAGCCTGG AAGCAAATCC ACACCTCCTT TCTCTGAACC CCTGGAGGTG 1140
GGGGAGAATG ACAGTTTAAG CCAGTGCTTC ACGGGGACAC AGAGCACAGT GGGTTCAGAA 1200
AGCTGCAACT GCACTGAGCC CCTGTGCAGG ACTGATTGGA CTCCCATGTC CTCTGAAAAC 1260
TACTTGCAAA AAGAGGTGGA CAGTGGCCAT TGCCCGCACT GGGCAGCCAG CCCCAGCCCC 1320
AACTGGGCAG ATGTCTGCAC AGGCTGCCGG AACCCTCCTG GGGAGGACTG TGAACCCCTC 1380
GTGGGTTCCC CAAAACGTGG ACCCTTGCCC CAGTGCGCCT ATGGCATGGG CCTTCCCCCT 1440
GAAGAAGAAG CCAGCAGGAC GGAGGCCAGA GACCAGCCCG AGGATGGGGC TGATGGGAGG 1500
CTCCCAAGCT CAGCGAGGGC AGGTGCCGGG TCTGGAAGCT CCCCTGGTGG CCAGTCCCCT 1560
GCATCTGGAA ATGTGACTGG AAACAGTAAC TCCACGTTCA TCTCCAGCGG GCAGGTGATG 1620
AACTTCAAGG GCGACATCAT CGTGGTCTAC GTCAGCCAGA CCTCGCAGGA GGGCGCGGCG 1680
GCGGCTGCGG AGCCCATGGG CCGCCCGGTG CAGGAGGAGA CCCTGGCGCG CCGAGACTCC 1740
TTCGCGGGGA ACGGCCCGCG CTTCCCGGAC CCGTGCGGCG GCCCCGAGGG GCTGCGGGAG 1800
CCGGAGAAGG CCTCGAGGCC GGTGCAGGAG CAAGGCGGGG CCAAGGCT 1848

(2) INFORMATION FOR SEQ ID NO: 5
(i) SEQUENCE CHARACTERISTICS:
(A) LENGTH: 543
(B) TYPE: nucleic acid
(C) STRANDEDNESS: double
(D) TOPOLOGY: linear
(ii) MOLECULAR TYPE: cDNA
(xi) SEQUENCE DESCRIPTION: SEQ ID NO: 5:

ATCGCTCCTC CATGTACCAG TGAGAAGCAT TATGAGCATC TGGGACGGTG CTGTAACAAA 60
TGTGAACCAG GAAAGTACAT GTCTTCTAAA TGCACTACTA CCTCTGACAG TGTATGTCTG 120
CCCTGTGGCC CGGATGAATA CTTGGATAGC TGGAATGAAG AAGATAAATG CTTGCTGCAT 180
AAAGTTTGTG ATACAGGCAA GGCCCTGGTG GCCGTGGTCG CCGGCAACAG CACGACCCCC 240
CGGCGCTGCG CGTGCACGGC TGGGTACCAC TGGAGCCAGG ACTGCGAGTG CTGCCGCCGC 300
AACACCGAGT GCGCGCCGGG CCTGGGCGCC CAGCACCCGT TGCAGCTCAA CAAGGACACA 360
GTGTGCAAAC CTTGCCTTGC AGGCTACTTC TCTGATGCCT TTTCCTCCAC GGACAAATGC 420
AGACCCTGGA CCAACTGTAC CTTCCTTGGA AAGAGAGTAG AACATCATGG GACAGAGAAA 480
TCCGATGTGG TTTGCAGTTC TTCTCTGCCA GCTAGAAAAC CACCAAATGA ACCCCATGTT 540
TAC 543

(2) INFORMATION FOR SEQ ID NO: 6
(i) SEQUENCE CHARACTERISTICS:
(A) LENGTH: 87
(B) TYPE: nucleic acid
(C) STRANDEDNESS: double
(D) TOPOLOGY: linear
(ii) MOLECULAR TYPE: cDNA
(xi) SEQUENCE DESCRIPTION: SEQ ID NO: 6:

ATGGCCCCGC GCGCCCGGCG GCGCCGCCCG CTGTTCGCGC TGCTGCTGCT CTGCGCGCTG 60
CTCGCCCGGC TGCAGGTGGC TTTGCAG 87

(2) INFORMATION FOR SEQ ID NO: 7
(i) SEQUENCE CHARACTERISTICS:
(A) LENGTH: 21
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 7:

GCCAGCCTGT CCCGCGCCAT G 21

(2) INFORMATION FOR SEQ ID NO: 8
(i) SEQUENCE CHARACTERISTICS:
(A) LENGTH: 24
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 8:

CAGAATATGA AAGTCCCACC ACAT 24

(2) INFORMATION FOR SEQ ID NO: 9
(i) SEQUENCE CHARACTERISTICS:
(A) LENGTH: 21
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 9:

GAATTCATGG CCCCGCGCGC C 21

(2) INFORMATION FOR SEQ ID NO: 10
(i) SEQUENCE CHARACTERISTICS:
(A) LENGTH: 21
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 10:

TTCTAGAGCC TTGGCCCCGC C 21

(2) INFORMATION FOR SEQ ID NO: 11
(i) SEQUENCE CHARACTERISTICS:
(A) LENGTH: 21
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 11:

TTCTAGAAAG TAAACATGGG G 21

(2) INFORMATION FOR SEQ ID NO: 12
(i) SEQUENCE CHARACTERISTICS:
(A) LENGTH: 21
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 12:

AAGCTTTCAA TGATGATGAT G 21

(2) INFORMATION FOR SEQ ID NO: 13
(i) SEQUENCE CHARACTERISTICS:
(A) LENGTH: 23
(B) TYPE: amino acid
(C) STRANDEDNESS:
(D) TOPOLOGY: linear
(ii) MOLECULAR TYPE: peptide
(xi) SEQUENCE DESCRIPTION: SEQ ID NO: 13:
Cys His Gly Thr Glu Lys Ser Asp Val Val Cys Ser Ser Ser Leu Pro

```
 1               5                  10                   15
Ala Arg Lys Pro Pro Asn Glu
             20
```

(2) INFORMATION FOR SEQ ID NO: 14
(i) SEQUENCE CHARACTERISTICS:
(A) LENGTH: 23
(B) TYPE: amino acid
(C) STRANDEDNESS:
(D) TOPOLOGY: linear
(ii) MOLECULAR TYPE: peptide
(xi) SEQUENCE DESCRIPTION: SEQ ID NO: 14:

```
Cys Ser Asp Ser Val Cys Leu Pro Cys Gly Pro Asp Glu Tyr Leu Asp
 1               5                  10                   15
Ser Trp Asn Glu Glu Asp Lys
             20
```

(2) INFORMATION FOR SEQ ID NO: 15
(i) SEQUENCE CHARACTERISTICS:
(A) LENGTH: 26
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 15:

AGGATCCCCT GTCCCGCGCC ATGGCC 26

(2) INFORMATION FOR SEQ ID NO: 16
(i) SEQUENCE CHARACTERISTICS:
(A) LENGTH: 30
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 16:

AAAGCTTAAG CCTTGGCCCC GCCTTGCTCC 30

(2) INFORMATION FOR SEQ ID NO: 17
(i) SEQUENCE CHARACTERISTICS:
(A) LENGTH: 30
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 17:

TTGCCCGGTT TTATAATTCT GCTTCTCTTC 30

(2) INFORMATION FOR SEQ ID NO: 18
(i) SEQUENCE CHARACTERISTICS:
(A) LENGTH: 29
(B) TYPE: nucleic acid
(C) STRANDEDNESS: single
(D) TOPOLOGY: linear
(ii) MOLECULAR TYPE: synthetic DNA
(xi) SEQUENCE DESCRIPTION: SEQ ID NO: 18:
TGTATTCATC TTCTGTGGGC ATCTGTCTG 29

SEQUENCE LISTING

NUMBER OF SEQUENCES:18

(1)INFORMATION FOR SEQ ID NO: 1

(i) SEQUENCE CHARACTERISTICS:

(A) LENGTH: 616

(B) TYPE: amino acid

(C) STRANDEDNESS:

(D) TOPOLOGY: linear

(ii) MOLECULAR TYPE: protein

(xi) SEQUENCE DESCRIPTION: SEQ ID NO: 1:

Met Ala Pro Arg Ala Arg Arg Arg Arg Pro Leu Phe Ala Leu Leu Leu
1 5 10 15

Leu Sys Ala Leu Leu Ala Arg Leu Gln Val Ala Leu Gln Ile Ala Pro
20 25 30

Pro Cys Thr Ser Glu Lys His Tyr Glu His Leu Gly Arg Cys Cys Asn
35 40 45

Lys Cys Glu Pro Gly Lys Tyr Met Ser Ser Lys Cys Thr Thr Thr Ser
50 55 60

Asp Ser Val Cys Leu Pro Cys Gly Pro Asp Glu Tyr Leu Asp Ser Trp
65 70 75 80

Asn Glu Glu Asp Lys Cys Leu Leu His Lys Val Cys Asp Thr Gly Lys

85 90 95

Ala Leu Val Ala Val Val Ala Gly Asn Ser Thr Thr Pro Arg Arg Cys

100 105 110

Ala Cys Thr Ala Gly Tyr His Trp Ser Gln Asp Cys Glu Cys Cys Arg

115 120 125

Arg Asn Thr Glu Cys Ala Pro Gly Leu Gly Ala Gln His Pro Leu Gln

130 135 140

Leu Asn Lys Asp Thr Val Cys Lys Pro Cys Leu Ala Gly Tyr Phe Ser

145 150 155 160

Asp Ala Phe Ser Ser Thr Asp Lys Cys Arg Pro Trp Thr Asn Cys Thr

165 170 175

Phe Leu Gly Lys Arg Val Glu His His Gly Thr Glu Lys Ser Asp Val

180 185 190

Val Cys Ser Ser Ser Leu Pro Ala Arg Lys Pro Pro Asn Glu Pro His

195 200 205

Val Tyr Leu Pro Gly Leu Ile Ile Leu Leu Leu Phe Ala Ser Val Ala

210 215 220

Leu Val Ala Ala Ile Ile Phe Gly Val Cys Tyr Arg Lys Lys Gly Lys

225 230 235 240

Ala Leu Thr Ala Asn Leu Trp His Trp Ile Asn Glu Ala Cys Gly Arg

245 250 255

Leu Ser Gly Asp Lys Glu Ser Ser Gly Asp Ser Cys Val Ser Thr His

260 265 270

Thr Ala Asn Phe Gly Gln Gln Gly Ala Cys Glu Gly Val Leu Leu Leu

275 280 285

Thr Leu Glu Glu Lys Thr Phe Pro Glu Asp Met Cys Tyr Pro Asp Gln

290 295 300

Gly Gly Val Cys Gln Gly Thr Cys Val Gly Gly Gly Pro Tyr Ala Gln

305 310 315 320

Gly Glu Asp Ala Arg Met Leu Ser Leu Val Ser Lys Thr Glu Ile Glu

325 330 335

Glu Asp Ser Phe Arg Gln Met Pro Thr Glu Asp Glu Tyr Met Asp Arg

340 345 350

Pro Ser Gln Pro Thr Asp Gln Leu Leu Phe Leu Thr Glu Pro Gly Ser

355 360 365

Lys Ser Thr Pro Pro Phe Ser Glu Pro Leu Glu Val Gly Glu Asn Asp

370 375 380

Ser Leu Ser Gln Cys Phe Thr Gly Thr Gln Ser Thr Val Gly Ser Glu

385 390 395 400

Ser Cys Asn Cys Thr Glu Pro Leu Cys Arg Thr Asp Trp Thr Pro Met

405 410 415

Ser Ser Glu Asn Tyr Leu Gln Lys Glu Val Asp Ser Gly His Cys Pro

420 425 430

His Trp Ala Ala Ser Pro Ser Pro Asn Trp Ala Asp Val Cys Thr Gly

435 440 445

Cys Arg Asn Pro Pro Gly Glu Asp Cys Glu Pro Leu Val Gly Ser Pro

450 455 460

Lys Arg Gly Pro Leu Pro Gln Cys Ala Tyr Gly Met Gly Leu Pro Pro

465 470 475 480

Glu Glu Glu Ala Ser Arg Thr Glu Ala Arg Asp Gln Pro Glu Asp Gly

485 490 495

Ala Asp Gly Arg Leu Pro Ser Ser Ala Arg Ala Gly Ala Gly Ser Gly

500 505 510

Ser Ser Pro Gly Gly Gln Ser Pro Ala Ser Gly Asn Val Thr Gly Asn

515 520 525

Ser Asn Ser Thr Phe Ile Ser Ser Gly Gln Val Met Asn Phe Lys Gly

530 535 540

Asp Ile Ile Val Val Tyr Val Ser GIn Thr Ser Gln Glu Gly Ala Ala

545 550 555 560

Ala Ala Ala Glu Pro Met Gly Arg Pro Val Gln Glu Glu Thr Leu Ala

565 570 575

Arg Arg Asp Ser Phe Ala Gly Asn Gly Pro Arg Phe Pro Asp Pro Cys

580 585 590

Gly Gly Pro Glu Gly Leu Arg Glu Pro Glu Lys Ala Ser Arg Pro Val

595 600 605

Gln Glu Gln Gly Gly Ala Lys Ala

610 615

(2)INFORMATION FOR SEQ ID NO: 2

(i) SEQUENCE CHARACTERISTICS:

(A) LENGTH: 181

(B) TYPE: amino acid

(C) STRANDEDNESS:

(D) TOPOLOGY: linear

(ii) MOLECULAR TYPE: peptide

(xi) SEQUENCE DESCRIPTION: SEQ ID NO: 2:

Ile AIa Pro Pro Cys Thr Ser Glu Lys His Tyr Glu His Leu Gly Arg

1 5 10 15

Cys Cys Asn Lys Cys Glu Pro Gly Lys Tyr Met Ser Ser Lys Cys Thr

20 25 30

Thr Thr Ser Asp Ser Val Cys Leu Pro Cys Gly Pro Asp Glu Tyr Leu

35 40 45

Asp Ser Trp Asn Glu Glu Asp Lys Cys Leu Leu His Lys Val Cys Asp

50 55 60

Thr Gly Lys Ala Leu Val Ala Val Val Ala Gly Asn Ser Thr Thr Pro

65 70 75 80

Arg Arg Cys Ala Cys Thr Ala Gly Tyr His Trp Ser Gln Asp Cys Glu

85 90 95

Cys Cys Arg Arg Asn Thr Glu Cys Ala Pro Gly Leu Gly Ala Gln His

100 105 110

Pro Leu Gln Leu Asn Lys Asp Thr Val Cys Lys Pro Cys Leu Ala Gly

115 120 125

Tyr Phe Ser Asp Ala Phe Ser Ser Thr Asp Lys Cys Arg Pro Trp Thr

130 135 140

Asn Cys Thr Phe Leu Gly Lys Arg Val Glu His His Gly Thr Glu Lys

145 150 155 160

Ser Asp Val Val Cys Ser Ser Ser Leu Pro Ala Arg Lys Pro Pro Asn

165 170 175

Glu Pro His Val Tyr

180

(3)INFORMATION FOR SEQ ID NO: 3

(i) SEQUENCE CHARACTERISTICS:

(A) LENGTH: 29

(B) TYPE: amino acid

(C) STRANDEDNESS:

(D) TOPOLOGY: linear

(ii) MOLECULAR TYPE: peptide

(xi) SEQUENCE DESCRIPTION: SEQ ID NO: 3:

Met Ala Pro Arg Ala Arg Arg Arg Arg Pro Leu Phe Ala Leu Leu leu

1 5 10 15

Leu Cys Ala Leu Leu Ala Arg Leu Gln Val Ala Leu Gln

20 25

(4)INFORMATION FOR SEQ ID NO: 4

(i) SEQUENCE CHARACTERISTICS:

(A) LENGTH: 1848

(B) TYPE: nucleic acid

(C) STRANDEDNESS: single

(D) TOPOLOGY: linear

(ii) MOLECULAR TYPE: cDNA

(xi) SEQUENCE DESCRIPTION: SEQ ID NO: 4:

ATGGCCCCGC GCGCCCGGCG GCGCCGCCCG CTGTTCGCGC TGCTGCTGCT CTGCGCGCTG 60

CTCGCCCGGC TGCAGGTGGC TTTGCAGATC GCTCCTCCAT GTACCAGTGA GAAGCATTAT 120

GAGCATCTGG GACGGTGCTG TAACAAATGT GAACCAGGAA AGTACATGTC TTCTAAATGC 180

ACTACTACCT CTGACAGTGT ATGTCTGCCC TGTGGCCCGG ATGAATACTT GGATAGCTGG 240

AATGAAGAAG ATAAATGCTT GCTGCATAAA GTTTGTGATA CAGGCAAGGC CCTGGTGGCC 300

GTGGTCGCCG GCAACAGCAC GACCCCCCGG CGCTGCGCGT GCACGGCTGG GTACCACTGG 360

AGCCAGGACT GCGAGTGCTG CCGCCGCAAC ACCGAGTGCG CGCCGGGCCT GGGCGCCCAG 420

CACCCGTTGC AGCTCAACAA GGACACAGTG TGCAAACCTT GCCTTGCAGG CTACTTCTCT 480

GATGCCTTTT CCTCCACGGA CAAATGCAGA CCCTGGACCA ACTGTACCTT CCTTGGAAAG 540

AGAGTAGAAC ATCATGGGAC AGAGAAATCC GATGTGGTTT GCAGTTCTTC TCTGCCAGCT 600

AGAAAACCAC CAAATGAACC CCATGTTTAC TTGCCCGGTT TAATAATTCT GCTTCTCTTC 660

GCGTCTGTGG CCCTGGTGGC TGCCATCATC TTTGGCGTTT GCTATAGGAA AAAAGGGAAA 720

GCACTCACAG CTAATTTGTG GCACTGGATC AATGAGGCTT GTGGCCGCCT AAGTGGAGAT 780

AAGGAGTCCT CAGGTGACAG TTGTGTCAGT ACACACACGG CAAACTTTGG TCAGCAGGGA 840

GCATGTGAAG GTGTCTTACT GCTGACTCTG GAGGAGAAGA CATTTCCAGA AGATATGTGC 900

TACCCAGATC AAGGTGGTGT CTGTCAGGGC ACGTGTGTAG GAGGTGGTCC CTACGCACAA 960

GGCGAAGATG CCAGGATGCT CTCATTGGTC AGCAAGACCG AGATAGAGGA AGACAGCTTC 1020

AGACAGATGC CCACAGAAGA TGAATACATG GACAGGCCCT CCCAGCCCAC AGACCAGTTA 1080

CTGTTCCTCA CTGAGCCTGG AAGCAAATCC ACACCTCCTT TCTCTGAACC CCTGGAGGTG 1140

GGGGAGAATG ACAGTTTAAG CCAGTGCTTC ACGGGGACAC AGAGCACAGT GGGTTCAGAA 1200

AGCTGCAACT GCACTGAGCC CCTGTGCAGG ACTGATTGGA CTCCCATGTC CTCTGAAAAC 1260

TACTTGCAAA AAGAGGTGGA CAGTGGCCAT TGCCCGCACT GGGCAGCCAG CCCCAGCCCC 1320

AACTGGGCAG ATGTCTGCAC AGGCTGCCGG AACCCTCCTG GGGAGGACTG TGAACCCCTC 1380

GTGGGTTCCC CAAAACGTGG ACCCTTGCCC CAGTGCGCCT ATGGCATGGG CCTTCCCCCT 1440

GAAGAAGAAG CCAGCAGGAC GGAGGCCAGA GACCAGCCCG AGGATGGGGC TGATGGGAGG 1500

CTCCCAAGCT CAGCGAGGGC AGGTGCCGGG TCTGGAAGCT CCCCTGGTGG CCAGTCCCCT 1560

GCATCTGGAA ATGTGACTGG AAACAGTAAC TCCACGTTCA TCTCCAGCGG GCAGGTGATG 1620

AACTTCAAGG GCGACATCAT CGTGGTCTAC GTCAGCCAGA CCTCGCAGGA GGGCGCGGCG 1680

GCGGCTGCGG AGCCCATGGG CCGCCCGGTG CAGGAGGAGA CCCTGGCGCG CCGAGACTCC 1740

TTCGCGGGGA ACGGCCCGCG CTTCCCGGAC CCGTGCGGCG GCCCCGAGGG GCTGCGGGAG 1800

CCGGAGAAGG CCTCGAGGCC GGTGCAGGAG CAAGGCGGGG CCAAGGCT 1848

(5)INFORMATION FOR SEQ ID NO: 5

(i) SEQUENCE CHARACTERISTICS:

(A) LENGTH: 543

(B) TYPE: nucleic acid

(C) STRANDEDNESS: double

(D) TOPOLOGY: linear

(ii) MOLECULAR TYPE: cDNA

(xi) SEQUENCE DESCRIPTION: SEQ ID NO: 5:

ATCGCTCCTC CATGTACCAG TGAGAAGCAT TATGAGCATC TGGGACGGTG CTGTAACAAA 60

TGTGAACCAG GAAAGTACAT GTCTTCTAAA TGCACTACTA CCTCTGACAG TGTATGTCTG 120

CCCTGTGGCC CGGATGAATA CTTGGATAGC TGGAATGAAG AAGATAAATG CTTGCTGCAT 180

AAAGTTTGTG ATACAGGCAA GGCCCTGGTG GCCGTGGTCG CCGGCAACAG CACGACCCCC 240

CGGCGCTGCG CGTGCACGGC TGGGTACCAC TGGAGCCAGG ACTGCGAGTG CTGCCGCCGC 300

AACACCGAGT GCGCGCCGGG CCTGGGCGCC CAGCACCCGT TGCAGCTCAA CAAGGACACA 360

GTGTGCAAAC CTTGCCTTGC AGGCTACTTC TCTGATGCCT TTTCCTCCAC GGACAAATGC 420

AGACCCTGGA CCAACTGTAC CTTCCTTGGA AAGAGAGTAG AACATCATGG GACAGAGAAA 480

TCCGATGTGG TTTGCAGTTC TTCTCTGCCA GCTAGAAAAC CACCAAATGA ACCCCATGTT 540

TAC 543

(6)INFORMATION FOR SEQ ID NO: 6

(i) SEQUENCE CHARACTERISTICS:

(A) LENGTH: 87

(B) TYPE: nucleic acid

(C) STRANDEDNESS: double

(D) TOPOLOGY: linear

(ii) MOLECULAR TYPE: cDNA

(xi) SEQUENCE DESCRIPTION: SEQ ID NO: 6:

ATGGCCCCGC GCGCCCGGCG GCGCCGCCCG CTGTTCGCGC TGCTGCTGCT CTGCGCGCTG 60

CTCGCCCGGC TGCAGGTGGC TTTGCAG 87

(7)INFORMATION FOR SEQ ID NO: 7

(i) SEQUENCE CHARACTERISTICS:

(A) LENGTH: 21

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 7:

GCCAGCCTGT CCCGCGCCAT G 21

(8)INFORMATION FOR SEQ ID NO: 8

(i) SEQUENCE CHARACTERISTICS:

(A) LENGTH: 24

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 8:

CAGAATATGA AAGTCCCACC ACAT 24

(9) INFORMATION FOR SEQ ID NO: 9

(i) SEQUENCE CHARACTERISTICS:

(A) LENGTH: 21

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 9:

GAATTCATGG CCCCGCGCGC C 21

(10) INFORMATION FOR SEQ ID NO: 10

(i) SEQUENCE CHARACTERISTICS:

(A) LENGTH: 21

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 10:

TTCTAGAGCC TTGGCCCCGC C 21

(11)INFORMATION FOR SEQ ID NO: 11

(i) SEQUENCE CHARACTERISTICS:

(A) LENGTH: 21

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 11:

TTCTAGAAAG TAAACATGGG G 21

(12)INFORMATION FOR SEQ ID NO: 12

(i) SEQUENCE CHARACTERISTICS:

(A) LENGTH: 21

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 12:

AAGCTTTCAA TGATGATGAT G 21

(13)INFORMATION FOR SEQ ID NO: 13

(i) SEQUENCE CHARACTERISTICS:

(A) LENGTH: 23

(B) TYPE: amino acid

(C) STRANDEDNESS:

(D) TOPOLOGY: linear

(ii) MOLECULAR TYPE: peptide

(xi) SEQUENCE DESCRIPTION: SEQ ID NO: 13:

Cys His Gly Thr Glu Lys Ser Asp Val Val Cys Ser Ser Ser Leu Pro
1 5 10 15

Ala Arg Lys Pro Pro Asn Glu
20

(14)INFORMATION FOR SEQ ID NO: 14

(i) SEQUENCE CHARACTERISTICS:

(A) LENGTH: 23

(B) TYPE: amino acid

(C) STRANDEDNESS:

(D) TOPOLOGY: linear

(ii) MOLECULAR TYPE: peptide

(xi) SEQUENCE DESCRIPTION: SEQ ID NO: 14:

Cys Ser Asp Ser Val Cys Leu Pro Cys Gly Pro Asp Glu Tyr Leu Asp
1 5 10 15

Ser Trp Asn Glu Glu Asp Lys
20

(15)INFORMATION FOR SEQ ID NO: 15

(i) SEQUENCE CHARACTERISTICS:

(A) LENGTH: 26

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 15:

AGGATCCCCT GTCCCGCGCC ATGGCC 26

(16) INFORMATION FOR SEQ ID NO: 16

(i) SEQUENCE CHARACTERISTICS:

(A) LENGTH: 30

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 16:

AAAGCTTAAG CCTTGGCCCC GCCTTGCTCC 30

(17) INFORMATION FOR SEQ ID NO: 17

(i) SEQUENCE CHARACTERISTICS:

(A) LENGTH: 30

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 17:

TTGCCCGGTT TTATAATTCT GCTTCTCTTC 30

(18) INFORMATION FOR SEQ ID NO: 18

(i) SEQUENCE CHARACTERISTICS:

(A) LENGTH: 29

(B) TYPE: nucleic acid

(C) STRANDEDNESS: single

(D) TOPOLOGY: linear

(ii) MOLECULAR TYPE: synthetic DNA

(xi) SEQUENCE DESCRIPTION: SEQ ID NO: 18:

TGTATTCATC TTCTGTGGGC

## Description

FIELD OF THE INVENTION

The present invention relates to a novel receptor protein derived from human dendritic cell and its use.

BACKGROUND OF THE INVENTION

Dendritic cells mature and differentiate from CD 34-positive progenitor cells present in bone marrow, and are distinguished from other monocytic cells in terms of morphology, motility and phagocytic activity. The cells reside in peripheral bloods and are widely distributed over skin tissues and non-lymphoid tissues such as heart, lung, etc. as well as lymphoid tissues such as lymph node, thymus, etc. They have important roles in the protection of a living body as antigen presenting cells of skin sensitizing materials, viral antigens, tumor related antigens, etc. to T cell. For example, it has been known that Langerhans cell, which is one member of cells belonging to this dendritic cell family, is widely distributed over skin tissues, in particular, epidermal cell layer, and profoundly participates in allergic inflammatory skin diseases such as allergic contact dermatitis, and atopic dermatitis [Rinshomeneki (Clinical Immune) 27, 1013-1018 (1995)]. In addition, recently, dendritic cells have been noted as a new material which can replace macrophages used for antitumor immunotherapy and immunopotentiation therapy in various infectious diseases due to their strong antigen presenting capabilities [Immunology Today, 18, 102-104 (1997)].

However, in comparison with other related cells such as macrophages and the like, study of dendritic cells has been remarkably delayed until quite recently because any technique for isolation and purification of a large amount of the cells, which is necessary for investigating cellular functions and biological activities of dendritic cells, have not been established.

At present, maturation of dendritic cells is hypothesized as "2-step model", initial maturation mediated through signals from exogenous antigens such as microorganisms and cytokines (e.g., IL-1$\beta$, GM-CSF, TNF-$\alpha$, etc.) and then terminal maturation mediated through cell cortex molecules (e.g., CD40L, etc.) and different cytokine signals (e.g., IFN-$\gamma$, etc.) [Immunology Today, 18, 102-104 (1997)]. It has also been reported that, upon activation of helper T cell by the above-described Langerhans cell as an antigen presenting cell, two signals are required, one being that mediated through a Class II antigen and a T cell receptor and the other being that by a so-called "costimulatory molecule" such as B7-1, B7-2 or the like [Immunology Today, 15, 464-469 (1994)].

Type 1 membrane proteins such as TNF receptor Types 1 and 2, low affinity NGF receptor, Fas antigen, CD27, CD30 and CD 40 are called as "TNF receptor family". Although their amino acid level-homology to one another is low, they have characteristic ligand binding domains having an abundance of cystein residue in their extracellular domains. Then, it is considered that a Type 1 membrane protein binds to a receptor specific ligand belonging to trimer forming Type 2 membrane proteins and crosslinking to each other to introduce signal transfer relating to various biological activities of a particular receptor. In addition, some of them have a common motif structure in their intracellular domains. For example, a domain called as "Death Doamin" is present in receptors having apoptosis introducing capability such as TNF receptor Type 1 and Fas antigen [Cell, 81, 479-482 (1995)].

CD40 is a member of TNA receptor family and has a molecular weight of about 50 kD. It was reported in 1986 as an antigen which transmits a B lymphocyte activating signal. A ligand of this receptor, CD40L (CD40 ligand), was isolated in 1993. This ligand is known to be that expressed on, for example, T cell surface as a Type 2 membrane protein having a molecular weight of about 35 kD. Recently, it has been clarified that a signal mediated through CD40 inhibits apoptosis of germ centre B lymphocyte, B lymphocyte cell strain and normal B lymphocyte. In addition, various functions of the molecule, for example, proliferation of B lymphocyte and introduction of class switch have been recognized. Then, the molecule is considered to play important roles in various steps of a T lymphocyte-dependent antibody reaction. Further, the signal is also mediated through CD40L and, therefore, participation of the signal in activation of T lymphocyte has been revealed. Since such expression of CD40 molecule is also observed on surfaces of the above-described dendritic cells, it has been suggested that dendritic cells also have similar activities to those of CD40 on B cell such as control of apoptosis, activating capability of T lymphocyte and the like. Nevertheless, if CD40L is deficient at the level of individuals, the production of isotypic antigens IgM are remarkably lowered, while serum IgM level is normal or higher, which causes, for example, immunodeficiency such as so-called sex-linked hyper-IgM syndrome. Then, regarding physiological significance of signals mediated through CD40 in dendritic cells, there are still many unknown points, though important roles of the signals in activation and differentiation of B lymphocyte have been recognized.

Accordingly, at present, it is difficult to explain physiological functions and, further, the relation thereof to various diseases, clearly, only by the functions of existing genes and the above-described presently available model of cell maturation, activation mechanism of T cell and the like. Then, it has been desired to isolate novel cell function-related genes derived from dendritic cells and to clarify its physiological functions from the viewpoint of not only further clarification of functions of dendritic cells in detail, but also developments of prophylactic and therapeutic medicines of various diseases targeting dendritic cells, or practice of effective immunotherapy with dendritic cells, from now.

## OBJECTS OF THE INVENTION

One object of the present invention is to provide a novel receptor protein derived from human dendritic cells, its partial peptide or their salts and a process for preparing the receptor protein or its salt.

Another object of the present invention is to provide an antibody against the receptor protein, its partial peptide or their salts.

A further object of the present invention is to provide a method for determination of a ligand to the receptor, the determined ligand to the receptor, a method for screening a compound which alters binding properties between the ligand and the receptor protein, a screening kit for the screening method, the compound obtained by the screening method or kit which alters binding properties between the ligand and the receptor protein and a pharmaceutical composition comprising the compound which alters binding properties between the ligand and the receptor protein.

These objects as well as other objects and advantages of the present invention will become apparent to those skilled in the art from the following description with reference to the accompanying drawings.

## BRIEF DESCRIPTION OF DRAWINGS

Fig. 1 is a nucleotide sequence encoding the novel receptor protein of the present invention obtained in Example 1 hereinafter and an amino acid sequence encoded by it.

Fig. 2 illustrates the results of hydrophobic plotting analysis of the novel receptor protein of the present invention.

## SUMMARY OF THE INVENTION

In order to accomplish the above objects of the present invention, the present inventors have studied intensively. As a result, the present inventors have succeeded in isolating a cDNA encoding a novel receptor protein derived from human dendritic cells, and analyzing its entire nucleotide sequence and the entire amino acid sequence of the receptor protein encoded by it. The present inventors have further studied based on these findings and completed the present invention.

That is, according to the present invention, among others, there are provided:

(1) A receptor protein which comprises the same or substantially the same amino acid sequence as that represented by SEQ ID NO: 1, or its salt;
(2) The protein of the above (1) which is a receptor protein derived from dendritic cell and belonging to the TNF receptor family;
(3) A partial peptide of the receptor protein of the above (1) or its salt;
(4) A process for producing the receptor protein of the above (1) or its salt which comprises cultivating a transformant transformed with a recombinant vector containing a DNA encoding the receptor protein of the above (1) to produce and accumulate the receptor protein and collecting it;
(5) An antibody against the receptor protein of the above (1), the partial peptide of the above (3) or their salts;
(6) A method for determining a ligand to the receptor protein of the above (1) or its salt which comprises using the receptor protein of the above (1), the partial peptide of the above (3) or their salts;
(7) A method for screening a compound which alters binding properties such as affinity or avidity between a ligand and the receptor protein of the above (1) or its salt, or a salt thereof, said method comprising using the receptor protein of the above (1), the partial peptide of the above (3) or their salts;
(8) A kit for screening a compound which alters binding properties between a ligand and the receptor protein of the above (1) or its salt, or a salt thereof, said kit comprising as an essential component the receptor protein of the above (1), the partial peptide of the above (3) or their salts;
(9) A compound which alters binding properties between a ligand and the receptor protein of the above (1) or its salt, or a salt thereof, whenever obtained by using the screening method of the above (7) or the screening kit of the above (8), or a salt thereof; and
(10) A pharmaceutical composition comprising a compound which alters binding properties between a ligand and the receptor protein of the above (1) or its salt, or a salt thereof, whenever obtained by using the screening method of the above (7) or the screening kit of the above (8).

More specifically, the present invention provides:

(11) The receptor protein of the above (1), wherein substantially the same amino acid sequence as that represented by SEQ ID NO: 1 (preferably the amino acid sequence from the 30th to 616th amino acids in the amino acid sequence represented by SEQ ID NO: 1) is that having about 40% or more (preferably about 50% or more, more preferably about 70% or more, more preferably about 80% or more, more preferably about 90% or more, most preferably about 95% or more) homology to the amino acid sequence represented by SEQ ID NO: 1 (preferably, the amino acid sequence from the 30th to 616th amino acids in the amino acid sequence represented by SEQ ID NO: 1);
(12) The receptor protein of the above (1), wherein substantially the same amino acid sequence as that represented by SEQ ID NO: 1 (preferably the amino acid sequence from the 30th to 616th amino acids in the amino acid sequence represented by SEQ ID NO: 1) is

(a) a variant of the amino acid sequence represented by SEQ ID NO: 1 (preferably the amino acid sequence from the 30th to 616th amino acids in the amino acid sequence represented by SEQ ID NO: 1) having a deletion of one or more amino acids (preferably about 1 - 30 amino acids),
(b) a variant of the amino acid sequence represented by SEQ ID NO: 1 (preferably the amino acid sequence from the 30th to 616th amino acids in the amino acid sequence represented by SEQ ID NO: 1) having an addition of one or more amino acids (preferably about 1 - 30 amino acids),
(c) a variant of the amino acid sequence represented by SEQ ID NO: 1 (preferably the amino acid sequence from the 30th to 616th amino acids in the amino acid sequence represented by SEQ ID NO: 1) having a substitution of one or more other amino acids (preferably about 1 - 30 amino acids), or
(d) a combination of the amino acid sequences of the above (a) to (c), or its salt;

(13) The partial peptide of the above (3) which comprises the same or substantially the same amino acid sequence as that represented by SEQ ID NO: 2;
(14) The partial peptide of the above (13) which is soluble;
(15) An isolated DNA comprising a DNA having a nucleotide sequence encoding the receptor protein of the above (1);
(16) The DNA of the above (15) having the nucleotide sequence represented by SEQ ID NO: 4 (preferably the nucleotide sequence from 88th to 1848th bases in the nucleotide sequence represented by SEQ ID NO: 4);
(17) A recombinant vector comprising the DNA of the above (16);
(18) A transformant transformed with the recombinant vector of the above (17);
(19) An isolated DNA comprising a DNA having a nucleotide sequence hybridizable with the nucleotide sequence represented by SEQ ID NO: 4 (preferably the nucleotide sequence from 88th to 1848th bases in the nucleotide sequence represented by SEQ ID NO: 4) under high stringent conditions;
(20) A recombinant vector comprising the DNA of the above (19);
(21) A transformant transformed with the recombinant vector of the above (20);
(22) A process for producing a receptor protein encoded by the DNA of the above (19), or its salt which comprises cultivating the transformant of the above (21) to produce and accumulate the receptor protein;
(23) The receptor protein produced by the process of the above (22), or its salt;
(24) An isolated DNA which comprises a DNA encoding the partial peptide of the above (3) or (13);
(25) The DNA of the above (24) having the nucleotide sequence of SEQ ID NO: 5;
(26) A recombinant vector comprising the DNA of the above (25);
(27) A transformant transformed with the recombinant vector of the above (26);
(28) A process for producing the partial peptide of the above (3) or (13), or its salt which comprises cultivating the transformant of the above (27) to produce and accumulate the peptide and collecting it;
(29) A signal peptide having the same or substantially the same amino acid sequence as that represented by SEQ ID NO: 3, or its salt;
(30) An isolated DNA comprising a DNA encoding the signal peptide of the above (29);
(31) The DNA of the above (30) having the nucleotide sequence represented by SEQ ID NO: 6;
(32) The method for determining a ligand of the above (6) which comprises allowing contact between a test compound, and the receptor protein of the above (1), the partial peptide of the above (3) or their salts;
(33) A ligand obtained by the ligand determination method of the above (6);
(34) The ligand of the above (33) which is TNF (e.g., TNF-$\alpha$), lymphotoxin (e.g., lymphotoxin-$\alpha$, lymphotoxin-$\beta$), Fas ligand, NGF, CD40 ligand, CD27 ligand, CD30 ligand, OX-40 ligand, 4-1BB ligand or Apo-2L (TRAIL);
(35) The method for screening of the above (7) which comprises comparing

(a) an index obtained upon allowing contact between the receptor protein of the above (1), the partial peptide of the above (3) or their salts and a ligand, and

(b) that obtained upon allowing contact among the receptor protein of the above (1), the partial peptide of the above (3) of theirs salts, the ligand, and a test compound;

(36) A method for screening a compound which alters binding properties between a ligand and the receptor protein of the above (1) or its salt, or a salt thereof, said method comprising measuring and comparing

(a) an amount of a labeled ligand bound to the receptor protein of the above (1), the partial peptide of the above (3) or their salts upon allowing contact between the labeled ligand and the receptor protein of the above (1), the partial peptide of the above (3) or their salts, and
(b) that upon allowing contact among the labeled ligand, a test compound and the receptor protein of the above (1), the partial peptide of the above (3) or their salts;

(37) A method for screening a compound which alters binding properties between a ligand and the receptor protein of the above (1) or its salt, or a salt thereof, said method comprising measuring and comparing

(a) an amount of a labeled ligand bound to cells containing the receptor protein of the above (1) upon allowing contact between the labeled ligand and the cells, and
(b) that upon allowing contact among the labeled ligand, a test compound and the cells;

(38) A method for screening a compound which alters binding properties between a ligand and the receptor protein of the above (1) or its salt, or a salt thereof, said method comprising measuring and comparing

(a) an amount of a labeled ligand bound to a membrane fraction of cells containing the receptor protein of the above (1) upon allowing contact between the labeled ligand and the membrane fraction of the cells, and
(b) that upon allowing contact among the labeled ligand, a test compound and the membrane fraction of the cells;

(39) A method for screening a compound which alters binding properties between a ligand and the receptor protein of the above (1) or its salt, or a salt thereof, said method comprising measuring and comparing

(a) an amount of a labeled ligand bound to the receptor protein upon allowing contact between the labeled ligand and the receptor protein expressed at the cell membrane of the transformant of the above (18) or (21) by cultivating the transformant, and
(b) that upon allowing contact among the labeled ligand, a test compound and the receptor protein expressed at the cell membrane of the transformant of the above (18) or (21) by cultivation of the transformant;

(40) A method for screening a compound which alters binding properties between a ligand and the receptor protein of the above (1) or its salt, or a salt thereof, said method comprising measuring and comparing

(a) a cell stimulating activity through the receptor protein upon allowing contact between a material activating the receptor protein of the above (1) or its salt and cells containing the receptor protein of the above (1), and
(b) that upon allowing contact among the material activating the receptor protein of the above (1), a test compound and cells containing the receptor protein of the above (1);

(41) A method for screening a compound which alters binding properties between the receptor protein of the above (1) or its salt, or a salt thereof, said method comprising measuring and comparing

(a) a cell stimulating activity through the receptor protein upon allowing contact between a material activating the receptor protein of the above (1) or its salt and the receptor protein expressed at the cell membrane of the transformant of the above (18) or (21) by cultivating the transformant, and
(b) that upon allowing contact among the material activating the receptor protein of the above (1), a test compound and the receptor protein expressed at the cell membrane of the transformant of the above (18) or (21) by cultivating the transformant;

(42) The screening method of the above (40) or (41), wherein the material activating the receptor protein of the above (1) is TNF (e.g., TNF-$\alpha$), lymphotoxin (e.g., lymphotoxin-$\alpha$, lymphotoxin-$\beta$) Fas ligand, NGF, CD40 ligand, CD27 ligand, CD30 ligand, OX-40 ligand, 4-1BB ligand or Apo-2L(TRAIL);
(43) A compound which alters binding properties between a ligand and the receptor protein of the above (1) or its salt, or a salt thereof, whenever obtained by any one of the screening methods of the above (35) to (41);

(44) A pharmaceutical composition which comprises a compound which alters binding properties between a ligand and the receptor protein of the above (1) or its salt, or a salt thereof, whenever obtained by any one of the screening methods of the above (35) to (41);

(45) The screening kit of the above (8) which comprises as an essential component cells containing the receptor protein of the above (1);

(46) The screening kit of the above (8) which comprises as an essential component a membrane fraction of cells containing the receptor protein of the above (1);

(47) The screening kit of the above (8) which comprises as an essential component the receptor protein expressed at the cell membrane of the transformant of the above (18) or (21) by cultivating the transformant;

(48) A compound which alters binding properties between a ligand and the receptor protein of the above (1) or its salt, or a salt thereof, whenever obtained by using the any one of the screening kits of the above (45) to (47);

(49) A pharmaceutical composition which comprises a compound which alters binding properties between a ligand and the receptor protein of the above (1) or its salt, or a salt thereof, whenever obtained by using any one of the screening kits of the above (45) to (47);

(50) A pharmaceutical composition which comprises an agonist to the receptor protein of the above (1) or its salt, whenever obtained by using any one of the screening methods of the above (7) and (35) to (41) or the screening kits of the above (8) and (45) to (47);

(51) The pharmaceutical composition of the above (50) for preventing or treating cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis ,etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others;

(52) A pharmaceutical composition which comprises an antagonist to the receptor protein of the above (1) or its salt, whenever obtained by using any one of the screening methods of the above (7) and (35) to (41) or the screening kits of the above (8) and (45) to (47);

(53) The pharmaceutical composition of the above (52) for preventing or treating allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others;

(54) A method for quantitative assay of the receptor protein of the above (1), the partial peptide of the above (3) or their salts which comprises allowing contact between the antibody of the above (5), and the receptor protein of the above (1), the partial peptide of the above (3) or their salts;

(55) A method for quantitative assay of the receptor protein of the above (1), the partial peptide of the above (3) or their salts in a specimen fluid which comprises reacting the antibody of the above (5) with the specimen fluid solution to be tested, and the receptor protein of the above (1), the partial peptide of the above (3) or their salts which has been labeled, competitively, and then measuring the ratio of the labeled receptor protein of the above (1), the partial peptide of the above (3) or their salts bound to the antibody;

(56) A method for quantitative assay of the receptor protein of the above (1), the partial peptide of the above (3) or their salts in a specimen fluid which comprises reacting the specimen fluid to be tested with one antibody of the above (5) which has been insolubilized on a carrier and another antibody of the above (5) which has been labeled, simultaneously or continuously, and then measuring the activity of the labeling agent on the insolubilized carrier;

(57) A pharmaceutical composition which comprises the antibody of the above (5);

(58) A pharmaceutical composition which comprises the antibody of the above (5) having an agonistic activity;

(59) The pharmaceutical composition of the above (58) for preventing or treating cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others;

(60) A pharmaceutical composition which comprises the antibody of the above (5) having an antagonistic activity;

(61) The pharmaceutical composition of the above (60) for preventing or treating allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others;
(62) A pharmaceutical composition which comprises the soluble partial peptide of the above (14);
(63) The pharmaceutical composition of the above (62) for preventing or treating allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others;
(64) An antisense DNA comprising a complementary or substantially complementary nucleotide sequence to the DNA of the above (15) or (19) and having an inhibitory activity of the expression of the DNA;
(65) The antisense DNA of the above (64), wherein the substantially complementary nucleotide sequence to the DNA of the above (15) or (19) is a nucleotide sequence having about 70% or more (preferably about 80% or more, more preferably about 90% or more, most preferably about 95% or more) homology to the entire or partial nucleotide sequence complementary to the DNA;
(66) A pharmaceutical composition comprising the antisense DNA of the above (64); and
(67) The pharmaceutical composition of the above (66) for preventing or treating allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others.

## DETAILED DESCRIPTION OF THE INVENTION

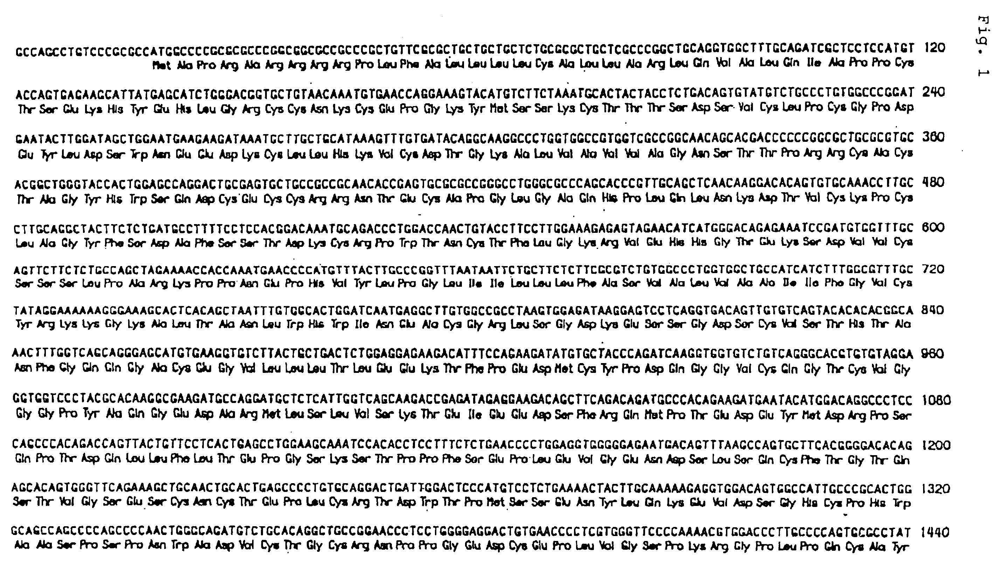

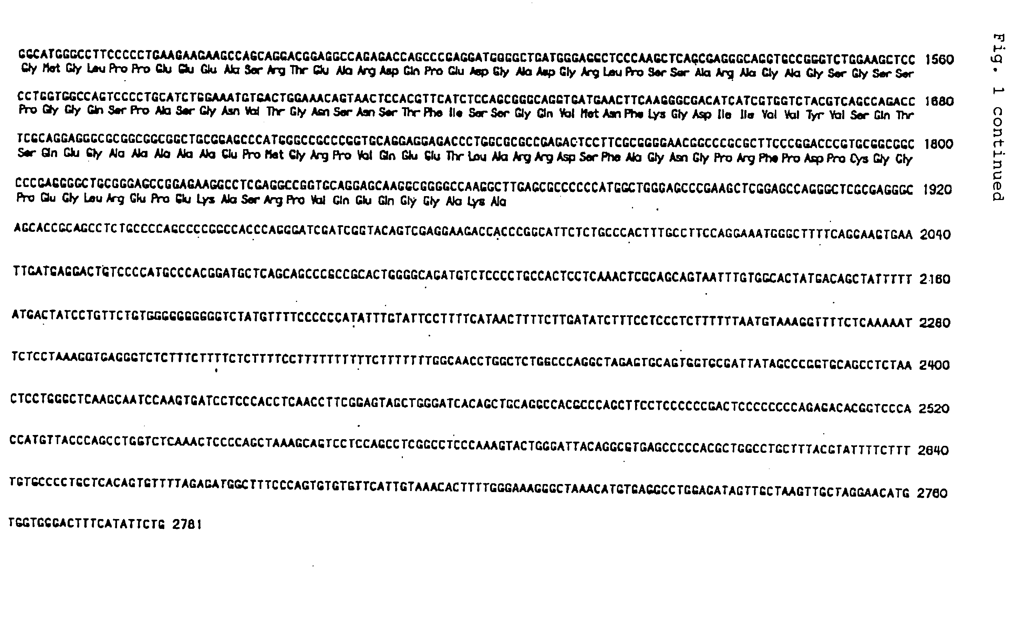

The receptor protein of the present invention is that having the same or substantially the same amino acid sequence as that represented by SEQ ID NO: 1 [Fig. 1] (preferably the amino acid sequence from the 30th to 616th amino acids in the amino acid sequence represented by SEQ ID NO: 1).

The receptor protein of the present invention may be any protein derived from any cells of human beings and another mammal (e.g., guinea pig, rat, mouse, rabbit, pig, sheep, cattle, monkey, etc.), for example, dendritic cell (e. g., Langerhans' cell, etc.), splenocyte, nerve cell, glia cell, pancreatic β cell, bone marrow cell, mesangial cell, epidermic cell, epithelial cell, endothelial cell, fibroblast, fibrocyte, muscular cell, fat cell, immunocyte (e.g., macrophage, T cell, B cell, natural killer cell, mast cell, neutrophil, basophil, eosinophil, monocyte, etc.), megakaryocyte, synovial cell, chondrocyte, osteocyte, osteoblast, osteoclast, mammary gland cell, hepatocyte, or interstitial cell or their precursor cells, stem cells or cancer cells thereof and the like, preferably dendritic cell; hemocyte (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-1, etc.) and any tissues containing such cells, for example, brain, various parts of brain (e.g., olfactory bulb, amygdala, cerebral basal qanglia, hippocampus, thalamus, hypothalamus, substhanlamic nucleus, cerebral cortex, medulla, cerebellum, occipital lobe, temporal lobe, peridium, caudate nucleus, cerebral gland, substantia nigra), spinal cord, pituitary, stomach, pancreas, kidney, liver, genital organs, thyroid gland, gall bladder, bone marrow, adrenal gland, skin, muscle, lung, digestive tract (e.g., large and small intestines), blood vessel, heart, thymus, spleen, submandibular gland, peripheral blood, intestinal tract, prostate, testicle, testis, ovarium, placenta, uterus, bone, joint, skeletal muscle and the like. And, the protein may be a synthetic one.

Examples of "substantially the same amino acid sequence as that represented by SEQ ID NO: 1" (preferably that of from the 30th to 616th amino acids in the amino acid sequence represented by SEQ ID NO:1) include an amino acid sequence having about 40% or more homology, preferably about 50% or more, more preferably about 70% or more, more preferably about 80% or more, more preferably about 90% or more and most preferably about 95% or more homology to the amino acid sequence represented by SEQ ID NO: 1, among others.

Specific preferred examples of "substantially the same amino acid sequence as that represented by SEQ ID NO: 1" (preferably that of from the 30th to 616th amino acids in the amino acid sequence represented by SEQ ID NO: 1) include an amino acid sequence having that of from the 30th to 210th amino acids in the amino acid sequence represented by SEQ ID NO: 1 and having about 40% or more homology, preferably about 50% or more, more preferably about 70% or more, more preferably about 80% or more, more preferably about 90% or more, and most preferably about 95% or more homology to the amino acid sequence represented by SEQ ID NO: 1 (preferably that of from the 30th to 616th amino acids in the amino acid sequence represented by SEQ ID NO:1), among others.

Examples of the receptor protein of the present invention which has substantially the same amino acid sequence as that represented by SEQ ID NO: 1 (preferably that of from 30th to 616th amino acids in the amino acid sequence represented by SEQ ID NO: 1) include that having the above-described substantially the same amino acid sequence as that represented by SEQ ID NO: 1 (preferably that of from the 30th to 616th amino acids in the amino acid sequence represented by SEQ ID NO:1), and has an activity of substantially the same quality as that of the protein comprising the amino acid sequence represented by SEQ ID NO: 1 (preferably that of from the 30th to 616th amino acids in the amino acid sequence represented by SEQ ID NO:1).

Examples of "an activity of substantially the same quality" include a ligand binding activity, a signal information transfer activity and the like. The wording "substantially the same" means that the natures (e.g., physiological chemical or pharmacological) of their activities are equal to one another, qualitatively. Therefore, although, preferably, the receptor proteins of the present invention have equal activities such as ligand binding activities, signal transfer ring activities and the like to one another (e.g., about 0.01 - 100 times, preferably about 0.5 - 20 times, more preferably about 0.5 - 2 times), degrees of their activities and quantitative factors such as molecular weights may differ from one another.

The activities such as the ligand binding activity, signal transfer ring activity and the like can be determined according to per se known method and, for example, can be determined according to the ligand determination method or screening method as described hereinafter.

Further, the receptor protein of the present invention may be a protein comprising

(a) a variant of the amino acid sequence represented by SEQ ID NO: 1 (preferably that of from the 30th to 616th amino acids in the amino acid sequence of SEQ ID NO: 1) having a deletion of one or more amino acids (preferably about 1 - 30, more preferably about 1 - 10, more preferably about 1 - 5 amino acids),
(b) a variant of the amino acid sequence represented by SEQ ID NO: 1 (preferably that of from the 30th to 616th amino acids in the amino acid sequence of SEQ ID NO: 1) having an addition of one or more amino acids (preferably about 1 to 30, more preferably about 1 - 10, more preferably about 1 - 5 amino acids),
(c) a variant of the amino acid sequence represented by SEQ ID NO: 1 (preferably that of from the 30th to 616th amino acids in the amino acid sequence of SEQ ID NO: 1) having a substitution of one or more amino acids (preferably about 1 - 30, more preferably about 1 - 10, more preferably about 1 - 5 amino acids), or
(d) a combination of the above (a) to (c).

When the amino acid sequence has the above-described deletion, addition or substitution, the position of such deletion, addition or substitution is not specifically limited, but an example thereof is a position other than the amino acid sequence of from the 30th to 210th amino acids in SEQ ID NO: 1.

In this respect, the amino acid sequence of from the 30th to 210th amino acids in SEQ ID NO: 1 (i.e., the amino acid sequence represented by SEQ ID NO: 2) represents that of the extracellular domain of the receptor protein having the amino acid sequence represented by SEQ ID NO: 1 of the present invention, preferably the ligand binding site.

Further, the amino acid sequence of from the 30th to 616th amino acids in SEQ ID NO: 1 corresponds to the amino acid sequence of SEQ ID NO: 1 from the amino terminus of which the amino acid sequence of SEQ ID NO: 3 (the amino acid sequence of the signal peptide of the present invention) has been removed and represents the amino acid sequence of the mature receptor protein of the present invention.

The receptor protein is represented herein by a conventional manner in the peptide art. That is, the left-hand end (amino terminus) is the N-terminus and the right-hand end (carboxyl terminus) is the C-terminus. And, in the receptor protein of the present invention, a representative example thereof being the protein comprising the amino acid sequence represented by SEQ ID NO: 1, normally, the C-terminus is carboxyl group (-COOH) or carboxylate ($-COO^-$), but the C-terminus may be the amide ($-CONH_2$) or an ester (-COOR).

Examples of R of the ester group include a $C_{1-6}$ alkyl group such as methyl, ethyl, n-propyl, isopropyl, n-butyl, etc., a $C_{3-8}$ cycloalkyl group such as cyclopentyl, cyclohexyl, etc., a $C_{6-12}$ aryl group such as phenyl, $\alpha$-naphthyl, etc., a $C_{7-14}$ aralkyl group such as a phenyl-$C_{1-2}$ alkyl group (e.g., benzyl, phenethyl, etc.), an $\alpha$-naphthyl-$C_{1-2}$ alkyl group (e.g., $\alpha$-naphthylmethyl, etc.) and the like. In addition, pivaloyloxymethyl ester or the like which is used widely as an ester for oral administration can also be used.

When the protein of the present invention has a carboxyl group (or carboxylate) at a position other than the C-terminus, it may be amidated or esterified and such amide or ester is also included in the scope of the receptor protein of the present invention. The ester group may be the same group as that described with respect to the above C-terminus.

Furthermore, examples of the receptor protein of the present invention include variants of the above receptor protein, wherein the amino group of the N-terminal methionine residue of the above receptor protein is protected with a protecting group (e.g., acyl group having 1 to 6 carbon atoms such as formyl group, acetyl group, etc.); the N-terminal region of the above protein is cleaved in a living body and the glutamyl group formed is pyroglutaminated; or a substituent (e.g., -OH, -COOH, amino group, imidazole group, indole group, guanidine group, etc.) on the side chain of an amino acid in the molecule of the above receptor protein is protected with a suitable protecting group (e.g., acyl group having 1 to 6 carbon atoms such as formyl group, acetyl group, etc.), or conjugated receptor proteins of the above receptor protein such as glycoproteins having sugar chains.

Specific examples of the receptor protein of the present invention include that derived from human dendritic cells comprising the amino acid sequence represented by SEQ ID NO: 1 (Fig. 1) and that derived from human dendritic cells comprising the amino acid sequence of from the 30th to 616th amino acids of the amino acid sequence represented by SEQ ID NO: 1.

The partial peptide of the receptor protein of the present invention (hereinafter sometimes abbreviated as the partial peptide of the present invention) may be any partial peptide of the above-described receptor proteins of the present invention. However, among molecules of the receptor proteins of the present invention, that having, for example, a part, which is exposed to outside of cell membranes and has a ligand binding activity, is preferred. Further, preferably, the partial peptide of the present invention is soluble.

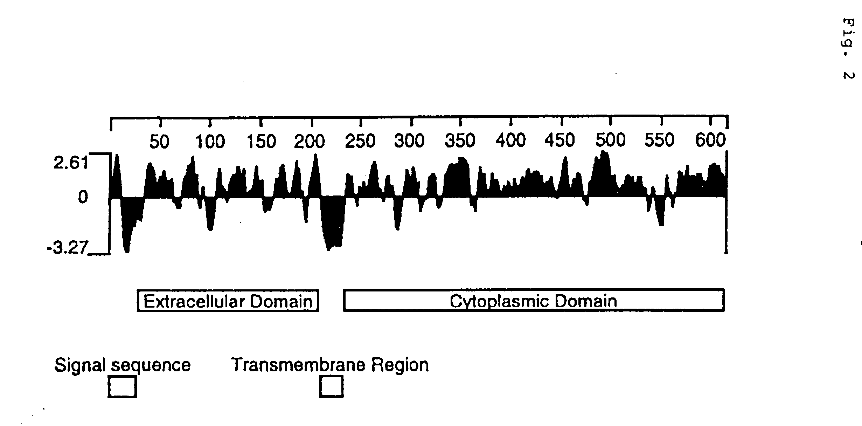

Specifically, the partial peptide of the receptor protein having the amino acid sequence represented by SEQ ID NO: 1 includes a peptide containing the part which is analyzed to be an extracellular domain from a hydrophobic plotting analysis (Fig. 2). Further, a peptide containing a hydrophobic part as a part thereof is also included in the partial peptide of the present invention. The partial peptide of the present invention may contain the respective domains separately. Or, the partial peptide of the present invention may contain plural domains together.

The partial peptide of the present invention has an amino acid sequence composed of at least about 20 amino acids, preferably about 50 or more amino acids, more preferably about 100 or more amino acids, more preferably about 200 or more amino acids, but less than about 300 amino acids of the constituent the amino acid sequence of the receptor protein of the present invention as described above.

Specific examples of the partial peptide of the present invention is that having the same or substantially the same amino acid sequence as that represented by SEQ ID NO: 2 and, preferably, having an activity of the same or substantially the same quality as that of the receptor protein of the present invention. Suitably, the peptide is soluble.

Substantially the same amino acid sequence as that represented by SEQ ID NO: 2 is an amino acid sequence having about 40% or more homology, preferably about 50% or more, more preferably about 70% or more, more preferably about 80% or more, more preferably about 90% or more, most preferably about 95% or more homology to the amino acid sequence represented by SEQ ID NO: 2.

The wording "an activity of substantially the same quality" is as defined above. The "activity of substantially the same quality" can be determined according to the ligand determination method or screening method as described hereinafter.

Further, the partial peptide of the present invention may be a variant of the above amino acid sequence having a deletion of one or more amino acids (preferably about 1 - 10, more preferably about 1 - 5 amino acids); a variant of the above amino acid sequence having an addition of one or more amino acids (preferably about 1 - 20, more preferably about 1 - 10, more preferably about 1 - 5 amino acids) ; or a variant of the above amino acid sequence having a substitution of one or more amino acids (preferably about 1 - 10, more preferably about 1 - 5 amino acids).

Like the above receptor protein, in the partial peptide of the present invention, normally, the C-terminus is carboxyl group (-COOH) or carboxylate ($-COO^-$). The C-terminus may also be the amide ($-CONH_2$) or an ester (-COOR).

Furthermore, like the above receptor protein, examples of the partial peptide of the present invention include its variants, wherein the amino group of the N-terminal methionine residue of the above receptor protein is protected with a protecting group; the N-terminal region is cleaved in a living body and the glutamyl group formed is pyroglutaminated; or a substituent on the side chain of an amino acid in the molecule of the above receptor protein is protected with a suitable protecting group; or conjugated peptides such as glycopeptides having sugar chains.

The partial peptide of the present invention can be used as an antigen for production of an antibody. Then, its receptor activity is not always required.

The signal peptide of the present invention is, for example, a peptide having the same or substantially the same amino acid sequence as that represented by SEQ ID NO: 3.

The signal peptide of the present invention may be any peptide derived from any cells of the above-described human beings and other warm-blooded animals (e.g., guinea pig, rat, mouse, chicken, rabbit, pig, sheep, cattle, monkey, etc.), or tissues wherein the cells are present. Or, the signal peptide may be a synthetic peptide.

Substantially the same amino acid sequence as that represented by SEQ ID NO: 3 is an amino acid sequence having about 70% or more homology, preferably about 80% or more, more preferably about 90% or more, most preferably about 95% or more homology to the amino acid sequence represented by SEQ ID NO: 3. More specifically, the signal peptide of the present invention may be any peptide in so far as it has substantially the same amino acid sequence as that represented by SEQ ID NO: 3 and can exhibit the function as a signal peptide. Therefore, quantitative factors such as molecular weight, etc. may be different from one another.

Further, the signal peptide of the present invention may be a variant of the above amino acid sequence having a deletion of one or more amino acids (preferably about 1 - 10, more preferably about 1 - 5, more preferably about 1 - 3 amino acids); a variant of the above amino acid sequence having an addition of one or more amino acids (preferably about 1 - 10, more preferably about 1 - 5, more preferably about 1 - 3 amino acids); or a variant of the above amino acid sequence having a substitution of one or more amino acids (preferably about 1 - 10, more preferably about 1 - 5, more preferably about 1 - 3 amino acids).

Like the above receptor protein, in the signal peptide of the present invention, normally, the C-terminus is carboxyl group (-COOH) or carboxylate ($-COO^-$). The C-terminus may also be the amide ($-CONH_2$) or an ester (-COOR).

Furthermore, like the above receptor protein, examples of the signal peptide of the present invention include its variants, wherein the amino group of the N-terminal methionine residue of the above receptor protein is protected with a protecting group; or a substituent on the side chain of an amino acid in the molecule of the above receptor protein is protected with a suitable protecting group; or conjugated peptides such as glycopeptides having sugar chains.

Specific examples of the signal peptide of the present invention include a peptide having the amino acid sequence represented by SEQ ID NO: 3 (i.e., the 1st to 29th amino acids of the amino acid sequence represented by SEQ ID NO: 1).

The signal peptide of the present invention can secrete various receptor proteins including the receptor protein of the present invention from cells extracellularly and efficiently.

As the salt of the receptor protein, partial peptide or signal peptide of the present invention, in particular, a physiologically acceptable acid addition salt is preferred. Examples of the salt include those with inorganic acids (e.g., hydrochloric acid, phosphoric acid, hydrobromic acid, sulfuric acid, etc.) and those with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.).

The receptor protein, partial peptide, signal peptide or their salts of the present invention can be produced from the above-described cells and tissues of human beings or other mammals by a per se known purification method of receptor proteins or peptides. Alternatively, they can be prepared by cultivating a transformant containing a DNA encoding the receptor protein, partial peptide or signal peptide of the present invention as described hereinafter, or according to a peptide synthesis method or its modification as described hereinafter.

When the receptor protein or the peptide is produced from cells or tissues of a human being or another mammal, the cells or tissues are homogenized and then extracted with, for example, an acid. The extract can be purified and isolated by combining chromatographies such as reverse phase chromatography, ion exchange chromatography and the like.

For synthesizing the receptor protein, partial peptide, signal peptide or their salts or amide derivatives of the present invention, normally, a commercially available synthetic resin for receptor protein synthesis can be used. Examples of the resin include chloromethyl resin, hydroxymethyl resin, benzhydrylamine resin, aminomethyl resin, 4-benzyloxybenzyl alcohol resin, 4-methyl-benzhydrylamine resin, PAM resin, 4-hydroxymethyl-methylphenylacetamidomethyl resin, polyacrylamide resin, 4-(2',4'-dimethoxyphenylhydroxymethyl)phenoxy resin, 4-(2',4'-dimethoxyphenyl-Fmoc aminoethyl)phenoxy resin and the like. By using such a resin and following the amino acid sequence of the desired receptor protein, amino acids, wherein $\alpha$-amino group and a functional group on a side chain are suitably protected, are condensed on the resin according to a per se known condensation method. After the condensation reaction, the protein is cleaved from the resin and, at the same time, various protecting groups are removed. Further, an intramolecular disulfide bond formation reaction is carried out in a highly diluted solution to obtain the desired receptor protein (partial peptide or signal peptide) or its amide derivative.

As for condensation of the above-described protected amino acids, various activating reagents for protein synthesis can be used. In particular, carbodiimides are preferred. Examples thereof include DCC, N,N'-di-isopropylcarbodiimide, N-ethyl-N'-(3-dimethylamino-prolyl)carbodiimide and the like. For activation with the reagent, the protected amino acid can be added the resin together with a racemization-inhibiting additive (e.g., HOBt, HOOBt), directly. Alternatively, the protected amino acid is activated in advance in the form of a symmetric acid anhydride, HOBt ester or HOOBt ester and then the activated amino acid derivative can be added to the resin.

As a solvent to be used for activation of protected amino acids and condensation with the resin, it can be appropriately selected from known solvents for protein condensation reactions. Examples thereof include acid amides such as N,N-dimethylformamide, N,N-dimethylacetamide, N-methylpyrrolidone and the like; halogenated hydrocarbons such as methylene chloride, chloroform and the like; alcohols such as trifluoroethanol and the like; sulfoxides such as dimethylsulfoxide and the like; ethers such as pyridine, dioxane, tetrahydrofuran and the like; nitriles such as acetonitrile, propionitrile and the like; esters such as methyl acetate, ethyl acetate and the like; suitable mixture of these solvents; and the like. The reaction temperature can be selected from those employed for peptide bond formation reactions, normally, within the range of from about -20°C to about 50°C. Normally, the activated amino acid derivative is used in 1.5 times to 4 times excess amount. When insufficient condensation is indicated by ninhydrin reaction, condensation can be repeated without removal of protecting groups to obtain sufficient condensation. If sufficient condensation is not obtained by repetition of the condensation reaction, unreacted amino acids can be acetylated with acetic anhydride or acetylimidazole not to affect on the following reaction.

Examples of the protecting group for the amino group of the starting material include Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl, Fmoc and the like.

The carboxyl group can be protected, for example, by alkyl esterification (esterification with straight, branched or cyclic alkyl such as methyl, ethyl, propyl, butyl, tert-butyl, cylcopentyl, cyclohexyl, cycloheptyl, cyclooctyl, 2-adamantyl, etc.), aralkyl esterification (formation of benzyl ester, 4-nitorbenzyl ester, 4-methoxybenzyl ester, 4-chlorobenzyl ester, benzhydril ester, etc.), phenacyl esterification, formation of benzyloxycarbonylhydrazide, formation of tert-butoxycarbonyl-hydrazide, formation of tritylhydrazide and the like.

The hydroxy group of serine can be protected, for example, by esterification or etherification. Suitable groups to be used for the esterification include, for example, lower alkanoyl groups such as acetyl group, aroyl groups such as benzoyl group, groups derived from carboxy acid such as benzyloxycarbonyl and ethoxycarbonyl, and the like. Suitable groups to be used for the etherification include, for example, benzyl, tetrahydropyranyl, t-butyl and the like.

As the protecting group for the phenolic hydroxy group of tyrosine, for example, Tos, 4-methoxy-2,3,6-trimethylbenzenesulfonyl, DNP, benzyloxymethyl, Bum, Boc, Trt, Fmoc and the like can be used.

Examples of the activated carboxyl group of the starting material include the corresponding acid anhydride, azide, activated ester (ester with an alcohol, e.g., pentachlorophenol, 2,4,5-trichlorophenol, 2,4-dinitro-phenol, cyanomethylalcohol, p-nitrophenol, HONB, N-hydroxysuccinimide, N-hydroxyphthalimide, HOBt) and the like. Examples of the activated amino group of the starting material include the corresponding phosphoric acid amide.

Removal of the protecting group (deprotection) can be carried out, for example, by catalytic reduction in a stream of hydrogen in the presence of a catalyst such as Pd black or Pd-C; treatment with an acid, for example, anhydrous hydrogen fluoride, methanesulfonic acid, trifluoro-methanesulfonic acid, trifluoroacetic acid, a mixture thereof and the like; by treatment with a base, for example, diisopropylethylamine, triethylamine, piperidine, piperazine and the like; reduction with sodium in liquid ammonia; and the like. The above treatment with an acid is generally carried out at about -20°C to about 40°C and, in the treatment with an acid, addition of a cation scavenger such as anisole; phenol, thioanisole, m-cresol, p-cresol, dimethylsulfide, 1,4-butanediol, 1,2-ethanediol or the like is effective. Also, 2,4-dinitrophenyl group used for protecting the imidazole protecting group of histidine is removed by treatment with thiophenol, and formyl group used for protecting the indole protecting group of tryptophan can also be removed by treatment with an alkali such as a dilute sodium hydroxide solution, dilute ammonia, etc. in addition to the above acid treatment in the presence of 1,2-ethanediol, 1,4-butanediol, etc.

Known groups and means can be appropriately selected for protection of a functional group which should not participate in the reaction of the starting material and its protecting group as well as such conditions as removal of the protecting group, activation of a functional group which participates in the reaction, and the like.

Alternatively, the amide derivative of the receptor protein or peptide of the present invention can be obtained, for example, by, first, protecting the α-carboxyl group of the C-terminal amino acid by amidation and extending a peptide (protein) chain towards the N-terminal to obtain the desired length of the chain. Then, only the protecting group of α-amino group of the N-terminal of the resultant peptide is removed to obtain a protein. Likewise, another protein is produced by removing only the protecting group of the C-terminal carboxyl group. Both proteins are condensed in the above-described mixed solvent. The condensation can be carried out as described above. The protected protein thus produced is purified and all the protecting groups can be deprotected as described above to obtain the desired crude protein. The crude protein can be purified by employing various known purification means and the main fraction is lyophilized to obtain the amide derivative of the desired protein or peptide.

For obtaining an ester of the receptor protein or peptide of the present invention, for example, the α-carboxyl group of the C-terminal amino acid is esterified by condensation of the desired alcohol to obtain the corresponding amino acid ester. Then, according to the same manner as in the amide derivative of the receptor protein, the desired ester of the receptor protein or peptide can be produced.

The partial peptide, signal peptide or their salts of the present invention can be produced by a per se known method for peptide synthesis. Alternatively, they can be produced by cleaving the receptor protein of the present invention with a suitable peptidase.

As the peptide synthesis method, for example, any of solid phase synthesis and liquid phase synthesis can be employed. That is, the desired peptide can be produced by condensing a partial peptide or amino acid sequence which can compose of the desired peptide with the remaining part and deprotecting a protecting group, if any. Conventional condensing methods and deprotecting methods can be employed and they are described by, for example, M. Bodanszky and M.A. Ondetti, Peptide Synthesis, Interscience Publishers, New York (1966); Schroeder and Luebke, The Peptide, Academic Press, New York (1965); Nobuo Izumi et al., Fundamental and Experiment of Peptide Synthesis, Maruzen (1975); Haruaki Yazima and Syunpei Sakakibara, Biochemistry Experiment Lecture, Protein Chemistry IV, 205 (1977); Haruaki Yazima, Second Series Drug Development Vol. 14, Peptide Synthesis, Hirokawa Shoten.

After completion of the reaction, the desired peptide can be purified and isolated by combining conventional purification methods such as solvent extraction, distillation, column chromatography, liquid chromatography, recrystallization and the like. In case the peptide thus obtained is a free peptide, it can be converted into its appropriate salt according to a known method. On the other hand, the peptide obtained is in the form of a salt, it can be converted into the corresponding free peptide.

The DNA encoding the receptor protein of the present invention may be any DNA in so far as it contains the nucleotide sequence encoding the above-described receptor protein of the present invention. The DNA may be any of genomic DNA, genomic DNA library, cDNA derived from the above-described cells and tissues, cDNA library derived from the above-described cells and tissues and synthetic DNA. The vector to be used for the library may be any of bacteriophage, plasmid, cosmid, phagemid and the like. In addition, the DNA can be amplified directly by reverse transcriptase polymerase chain reaction (hereinafter abbreviated to RT-PCR) with the total RNA or a mRNA fraction prepared from the above-described cells and tissues.

Specifically, the DNA encoding the receptor protein of the present invention may be, for example, the DNA having the nucleotide sequence represented by SEQ ID NO: 4 or any DNA having a nucleotide sequence hybridizable to the nucleotide sequence represented by SEQ ID NO: 4 under high stringent conditions and encoding a protein which has activities of the same quality (e.g., the ligand binding activity, signal transferring activity, etc.) as those of the receptor protein of the present invention.

Examples of the DNA hybridizable to the nucleotide sequence represented by SEQ ID NO: 4 include a DNA containing a nucleotide sequence having at least about 70%, preferably about 80% or more, more preferably about 90% or more, most preferably about 95% or more homology to the nucleotide sequence represented by SEQ ID NO: 4.

Hybridization can be carried out by a per se known method or its modification, for example, according to the method described in Molecular Cloning, 2nd ed, J. Sambrook et al., Cold Spring Harbor Lab. Press, 1989 or the like. A commercially available library can be used according to the direction of the attached manufacturer's protocol. More preferably, it can be carried out under high stringent conditions.

The high stringent conditions used herein are, for example, those of a sodium concentration at about 19 mM to about 40 mM, preferably about 19 mM to about 20 mM and a temperature at about 50°C to about 70°C, preferably about 60°C to about 65°C. In particular, most preferred hybridization conditions are at a sodium concentration of about 19 mM and a temperature of about 65°C.

Specifically, the DNA encoding the receptor protein containing the amino acid sequence represented by SEQ ID NO: 1 may be, for example, the DNA having the nucleotide sequence represented by SEQ ID NO: 2. The DNA encoding the receptor protein containing the amino acid sequence of from the 30th to 616th amino acids of the sequence represented by SEQ ID NO: 1 may be, for example, the DNA having the nucleotide sequence of from the 88th to 1848th bases of the sequence represented by SEQ ID NO: 2.

The DNA encoding the partial peptide of the present invention may be any DNA in so far as it contains the nucleotide sequence encoding the above-described partial peptide of the present invention. The DNA may be any of genomic DNA, genomic DNA library, cDNA derived from the above-described cells and tissues, cDNA library derived from the above-described cells and tissues and synthetic DNA. The vector to be used for the library may be any of bacteriophage, plasmid, cosmid, phagemid and the like. In addition, the DNA can be amplified directly by RT-PCR with a mRNA fraction prepared from the above-described cells and tissues.

Specifically, the DNA encoding the partial peptide of the present invention may be, for example, (a) a DNA having a partial nucleotide sequence of the DNA having the sequence represented by SEQ ID NO: 4, (b) a DNA having a partial nucleotide sequence of the DNA having a nucleotide sequence hybridizable to the nucleotide sequence represented by SEQ ID NO: 4 under high stringent conditions and encoding a receptor protein which has activities of the same quality (e.g., the ligand binding activity, signal transferring activity, etc.) as those of the receptor protein peptide of the present invention, or the like.

Examples of the DNA hybridizable to the nucleotide sequence represented by SEQ ID NO: 4 include a DNA containing a nucleotide sequence having at least about 70%, preferably about 80% or more, more preferably about 90% or more, most preferably about 95% or more homology to the nucleotide sequence represented by SEQ ID NO: 4.

More specifically, the DNA encoding the partial peptide having the amino acid sequence represented by SEQ ID NO: 2 may be, for example, the DNA having the nucleotide sequence represented by SEQ ID NO: 5, a DNA having a nucleotide sequence hybridizable to such nucleotide sequence under high stringent conditions, or the like.

Examples of the DNA hybridizable to the nucleotide sequence represented by SEQ ID NO: 5 include a DNA containing a nucleotide sequence having at least about 70%, preferably about 80% or more, more preferably about 90% or more, most preferably about 95% or more homology to the nucleotide sequence represented by SEQ ID NO: 5.

The same hybridization method and high stringent conditions as those described above can be employed.

The DNA encoding the signal peptide of the present invention may be any DNA in so far as it contains the nucleotide sequence encoding the above-described signal peptide of the present invention. The DNA may be any of genomic DNA, genomic DNA library, cDNA derived from the above-described cells and tissues, cDNA library derived from the above-described cells and tissues and synthetic DNA.

The DNA encoding the signal peptide of the present invention may be, for example, a DNA having the nucleotide sequence represented by SEQ ID NO: 6, a DNA having a nucleotide sequence hybridizable to the nucleotide sequence represented by SEQ ID NO: 6 under high stringent conditions and encoding a peptide which can exhibit the function as a signal peptide, or the like.

Examples of the DNA hybridizable to the nucleotide sequence represented by SEQ ID NO: 6 include a DNA containing a nucleotide sequence having at least about 70%, preferably about 80% or more, more preferably about 90% or more, most preferably about 95% or more homology to the nucleotide sequence represented by SEQ ID NO: 6.

The same hybridization method and high stringent conditions as those described above can be employed.

More specifically, as a DNA encoding the signal peptide having the amino acid sequence represented by SEQ ID: 3, a DNA comprising the DNA having the nucleotide sequence represented by SEQ ID NO: 6 or the like.

As a means of cloning the DNA encoding the receptor protein or its partial peptide of the present invention (hereinafter sometimes abbreviated as the receptor protein, etc. of the present invention), completely, there are (1) amplification of the desired DNA by PCR using synthetic DNA primers containing partial nucleotide sequences of the receptor protein, etc. of the present invention, (2) selection of a DNA integrated into a suitable vector by hybridization with a labeled DNA fragment or a synthetic DNA encoding a part or entire region of the receptor protein, etc. of the present invention. The hybridization is carried out, for example, according to the method described in Molecular Cloning 2nd ed.; J. Samrook et al., Cold Spring Harbor Lab. Press, (1989). A commercially available library can be used according to the direction of the attached manufacturer's protocol.

The DNA encoding the signal peptide of the present invention can be produced according to a per se known oligonucleotide synthetic method.

Modification (deletion, addition, substitution) of the nucleotide sequence of the DNA can be carried out by using a known kit, for example, Mutan™-G (Takara Shuzo Co., Ltd.) or Mutan™-K (Takara Shuzo Co., Ltd.) according to a per se known method such as Gapped duplex method or Kunkel method or its modification.

The cloned DNA encoding the receptor protein, etc. of the present invention can be used as such according to a particular purpose. Alternatively, if desired, it can be used after digestion with one or more restriction enzymes, or a linker can be added. The DNA may have the codon, ATG, as a translation initiation codon at its 5' terminal side and the codon, TAA, TGA or TAG as a translation termination codon at its 3' terminal side. These translation initiation and termination codons can be added by using a suitable synthetic DNA adapter.

The expression vector of the receptor protein, etc. of the present invention can be prepared, for example, by (a) cutting out the desired DNA fragment from the DNA encoding the receptor protein, etc. of the present invention and (b) joining the DNA fragment to a suitable expression vector at the downstream from a promoter in the vector.

Examples of the vector include plasmids derived from *Escherichia coli* (e.g., pBR322, pBR325, pUC12, pUC13), plasmids derived from *Bacillus subtilis* (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter in so far as it matches with a host to be used for gene expression. In case of using animal cells as the host, examples of the promoter include SRα promoter, SV40 promoter, LTR promoter, CMV (cytomegalovirus) promoter, HSV-TKpromoter, etc. Among them, CMV promoter or SRα promoter is preferred. In case of using bacteria of the genus *Escherichia* as the host, preferred examples of the promoter include trp promoter, lac promoter, recA promoter, $\lambda P_L$ promoter, lpp promoter, etc. In case of using bacteria of the genus *Bacillus* as the host, preferably, SPO1 promoter, SPO2 promoter, penP promoter, etc. can be used. In case of using yeast as the host, preferred examples of the promoter include PHO5 promoter, PGK promoter, GAP promoter, ADH promoter, etc. In case of using insect cells as the host, preferred examples of the promoter include polyhedrin prompter, P10 promoter, etc.

In addition to the above, optionally, the expression vector may further contains enhancer, splicing signal, poly A adenylation signal, selection marker, SV40 replication origin (hereinafter sometimes abbreviated to SV40 ori), etc. Examples of the selection marker include dihydrofolate reductase (hereinafter sometimes abbreviated to dhfr) gene [methotrexate (MTX) resistance], ampicillin resistant gene (hereinafter sometimes abbreviated to $Amp^r$), neomycin resistant gene (hereinafter sometimes abbreviated to Neo, G418 resistance), etc. In particular, when CHO ($dhfr^-$) cell is used together with dhfr gene as a selection marker, selection can also be carried out by using a thymidine free medium.

If necessary, a signal sequence which matches with a host is added to the N-terminal side of the receptor protein, etc. of the present invention. As the signal sequence, there may be mentioned PhoA signal sequence, OmpA signal sequence, etc. in case of using bacteria of the genus *Escherichia* as the host;α-amylase signal sequence, subtilisin signal sequence, etc. in case of using bacteria of the genus *Bacillus* as the host; MFα signal sequence, SUC2 signal sequence, etc. in case of using yeast as the host; insulin signal sequence, α-interferon signal sequence, antibody molecule signal sequence, etc. in case of using animal cells as the host, respectively.

The DNA encoding the receptor protein, etc. of the present invention thus constructed can be introduced into a host to produce a transformant.

As the host, for example, there may be mentioned bacteria of the genus *Escherichia,* bacteria of the genus *Bacillus,* yeast, insect cells, insects and animal cells, etc.

Specific examples of bacteria of the genus *Escherichia* include *Escherichia coli* K12·DH1 [Proc. Natl. Acad. Sci. USA, 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of bacteria of the genus *Bacillus* include *Bacillus subtilis* MI114 [Gene, 24, 255 (1983)], 207-21 [Journal of Biochemistry, 95, 87 (1984)], etc.

Examples of yeast include *Saccharomyces cereviseae* AH22, AH22$^-$, NA87-11A, DKD-5D and 20B-12, *Schizosaccharomyces pombe* NCYC1913 and NCYC2036, *Pichia pastoris,* etc.

Examples of insect cells include *Spodoptera frugiperda* cell (Sf cell), MG1 cell derived from mid-intestine of *Trichoplusia ni,* High Five™ cell derived from egg of *Trichoplusia ni,* cells derived from *Mamestra brassicae,* cells derived from *Estigmena acrea,* etc. for the virus, AcNPV; and *Bombyx mori* N cell (BmN cell), etc. for the virus, BmNPV. As the Sf cell, for example, Sf9 cell (ATCC CRL1711) and Sf21 cell described by Vaughn, J. L., in Vitro, 13, 213-217 (1977) can be used.

As the insect, for example, a larva of *Bombyx mori* can be used [Maeda et al., Nature, 315, 592 (1985)].

Examples of animal cells include monkey cell COS-7, Vero cell, Chinese hamster cell CHO (hereinafter abbreviated to CHO cell), dhfr gene deficient Chinese hamster cell CHO (hereinafter abbreviated to CHO(dhfr$^-$) cell), mouse L cell, mouse myeloma cell, rat GH3 cell, human FL cell, 293 cell, C127 cell, mouse cell, BALB3T3 cell, Sp-2/O cell, etc. Among them, CHO cell, CHO(dhfr$^-$) cell, 293 cell, etc. are preferred.

Transformation of bacteria of the genus *Escherichia* is carried out, for example, according to the method described in Proc. Natl. Acad. Sci. USA, 69, 2110 (1972) or Gene, 17, 107 (1982).

Transformation of bacteria of the genus *Bacillus* is carried out, for example, according to the method described in Molecular & General Genetics, 168, 111 (1979).

Transformation of yeast is carried out, for example, the method described in Methods in Enzymology, 194, 182-187 (1991); Proc. Natl. Acad. Sci. USA, 75, 1929 (1978); etc.

Transformation of insect cells or insect is carried out, for example, according to the method described in Bio/Technology, 6, 47-55 (1988).

Transformation of animal cells is carried out, for example, according to the methods described in Saiboukogaku (Cell Technology) Separate-Volume 8, New Cell Technology Experimental Protocol, 263-267 (1995), published by Syuzyun-sha; Virology, 52, 456 (1973); etc.

Introduction of the expression vector into cells can be carried out, for example, by calcium phosphate method (Graham, F.L. and van der Eb, A. J., Virology, 52, 456-467 (1973)), electroporation (Nuemann, E. et al., EMBO J., 1, 841-845 (1982)) or the like.

Thus, the transformant transformed with the expression vector containing the DNA encoding the receptor protein, etc. of the present invention can be obtained.

As a method for stable expression of the receptor protein, etc. of the present invention by using an animal cell, there may be mentioned clone selection for selecting an animal cell wherein the expression vector introduced is integrated in its chromosome. More specifically, transformants are selected by utilizing the above selection marker as an indicator. Further, clone selection of thus-obtained animal cells by using the above selection marker can be carried out repeatedly to obtain a stable animal cell line highly expressing the receptor protein, etc. of the present invention. When using a dhfr gene as the selection marker, an animal cell line more higher expressing the receptor protein can be obtained by cultivating the cell with gradually increasing MTX concentration to select a resistant strain, thereby amplifying the DNA encoding the protein, etc. of the present invention together with dhfr gene in the cell.

The receptor protein, etc. of the present invention can be produced by cultivating the above-described transformant under such conditions that the DNA encoding the receptor protein, etc. of the present invention can be expressed to form and accumulate the receptor protein, etc. of the present invention.

In case of the bacterial host of the genus *Escherichia* or *Bacillus,* the transformant can be suitably cultivated in a liquid culture medium and materials required for growth of the transformant such as carbon sources, nitrogen sources, inorganic materials, etc. are added to the medium. Examples of the carbon sources include glucose, dextrin, soluble starch, sucrose, etc. The nitrogen sources include, for example, inorganic or organic materials such as ammonium salts, nitrate salts, corn steep liquor, peptone, casein, meat extract, soybean meal, potato extract, etc. The inorganic materials include, for example, calcium chloride, sodium dihydrogen phosphate, magnesium chloride, etc. In addition, yeast extract, vitamins, growth promoting factors etc. can be added. Preferably, the medium is adjusted to pH about 5 to about 8.

Preferably, the medium for cultivating the bacteria of the genus *Escherichia* is, for example, M9 medium containing glucose and casamino acids (Miller, Journal of Experiments in Molecular Genetics, 431-433, Cold Spring Harbor Laboratory, New York, 1972). If necessary, in order to activate the promoter efficiently, for example, an agent such as 3β-indolyl acrylic acid can be added to the medium.

In case of the bacterial host of the genus *Escherichia,* normally, the transformant is cultivated at about 15°C to about 43°C for about 3 hours to about 24 hours. If necessary, the culture can be aerated or stirred.

In case of the bacterial host of the genus *Bacillus,* normally, the transformant is cultivated at about 30°C to about 40°C for about 6 hours to about 24 hours. If necessary, the culture can be aerated or stirred.

In case of the yeast host, the transformant is cultivated in, for example, Burkholder's minimal medium [Bostian, K. L. et al., Proc. Natl. Acad. Sci. USA, 77, 4505 (1980) and SD medium containing 0.5% casamino acids [Bitter, G. A., Proc. Natl. Acad. Sci. USA, 81, 5330 (1984)]. Preferably, the medium is adjusted to pH about 5 to about 8. Normally, the transformant is cultivated at about 20°C to about 35°C for about 24 hours to about 72 hours. If necessary, the culture can be aerated or stirred.

In case of the insect cell host or insect host, the transformant is cultivated in, for example, Grace's insect medium [Grace, T. C. C.,. Nature, 195, 788 (1962)] to which an appropriate additive such as inactivated 10% bovine serum is added. Preferably, the medium is adjusted to pH about 6.2 to about 6.4. Normally, the transformant is cultivated at about 27° C for 3 days to 5 days and, if necessary, the culture can be aerated or stirred.

In case of the animal cell host, the transformant is cultivated in, for example, MEM medium containing about 5% to about 20% fetal bovine serum [Science, 122, 501 (1952)], DMEM medium [Virology, 8, 396 81959)], RPMI 1640 medium [The Journal of the American Medical Association, 199, 519 (1967)], 199 medium [Proceeding of the Society for the Biological Medicine, 73, 1 (1950)], etc. Preferably, the medium is adjusted to pH about 6 to about 8. Normally, the transformant is cultivated at about 30° C to about 40° C for about 15 hours to about 70 hours and, if necessary, the culture can be aerated or stirred.

In particular, when using CHO ($dhfr^-$) cell and dhfr gene as a selection marker, it is preferred to use DMEM medium which contains dialyzed fetal bovine serum almost free from thymidine.

As described above, the receptor protein, etc. of the present invention can be produced by the transformant.

Separation and purification of the receptor protein, etc. of the present invention from the above culture can be carried out, for example, as follows.

For extraction of the receptor protein, etc. of the present invention from the transformant culture such as microbial cells, insect and animal cells, a known method can be appropriately employed. For example, after cultivation, the transformant is recovered by a per se known method and suspended in a suitable buffer. Then, the transformant is disrupted by ultrasonication, treatment with lysozyme and/or freeze-thaw cycling, followed by separating a crude extract of the receptor protein, etc. of the present invention by centrifugation, filtration, etc. The buffer may contain a protein modifier such as urea, guanine hydrochloride or a surfactant such as Triton X-100™ (TM means the registered trademark), etc.

When the partial peptide is secreted in the culture broth, after completion of cultivation, its supernatant can be separated from the transformant cells by a per se known method to collect the supernatant.

Purification of the receptor protein, etc. of the present invention contained in the culture supernatant thus obtained or the extract can be carried out by combining per se known separation and purification methods appropriately. As the per se known separation and purification methods, there may be mentioned a method utilizing difference in solubilities such as salting out, solvent precipitation, etc.; a method mainly utilizing difference in molecular weights such as dialysis, ultrafiltration, gel filtration, SDS-polyacrylamide gel electrophoresis, etc.; a method utilizing difference in electric charges such as ion exchange chromatography, etc.; a method utilizing difference in specific affinities such as affinity chromatography, etc.; a method utilizing difference in hydrophobic properties such as reverse phase high performance liquid chromatography, etc.; a method utilizing difference in isoelectric points such as isoelectric point electrophoresis: and the like.

When the receptor protein, etc. of the present invention is obtained in its free form, it can be converted into its salt by a per se known method or its modification. On the other hand, when the receptor protein, etc. of the present invention is obtained in the form of a salt, it can be converted into the free form or a different salt by a per se known method or its modification.

The receptor protein, etc. of the present invention produced by the recombinant can be treated with an appropriate protein modifying enzyme prior to or after purification to appropriately modify the receptor protein or to partially remove a polypeptide. Examples of the protein modifying enzyme include trypsin, chymotrypsin, arginyl endopeptidase, protein kinase, glycosidase and the like.

The presence or activity of thus-produced receptor protein, etc. of the present invention or their salts can be determined by a binding test to a ligand or an enzyme immunoassay using a specific antibody.

An antibody against the receptor protein, etc. of the present invention or their salts may be any monoclonal or polyclonal antibody in so far as it can recognize the receptor protein, etc. of the present invention. Further, an antibody against the receptor protein, etc. of the present invention may be that having such activity that it can bind to the receptor protein, etc. of the present invention to neutralize the activity of the receptor protein, etc. (antagonistic activity), or that having such activity that it can bind to the receptor protein, etc. of the present invention to induce the activity of the receptor protein, etc. (agonistic activity).

The antibody against the receptor protein, etc. of the present invention can be produced by using the receptor protein, etc. of the present invention as the antigen according to a conventional antibody or antiserum preparation process.

Preparation of monoclonal antibody

(a) Preparation of a monoclonal antibody-producing hybridoma

The receptor protein, etc. of the present invention as such or together with a suitable carrier or diluent is administered to a site of a mammal which permits the antibody production. For enhancing the antibody productivity, complete Freund's adjuvant or incomplete Freund's adjuvant may be administered. Normally, the receptor protein, etc. is administered once every 2 weeks to 6 weeks, in total, about 2 times to about 10 times. The mammal to be used include monkey, rabbit, dog, guinea pig, mouse, rat, sheep, goat and the like and mouse or rat is preferred.

For preparing monoclonal antibody-producing cells, an individual whose antibody titer has been confirmed is selected from warm-blooded animals immunized with the antigen, for example, mouse and, 2 days to 5 days after the final immunization, its spleen or lymph node is collected and antibody-producing cells contained therein are fused with myeloma cells of the same or a different kind of animal to prepare the desired monoclonal antibody producer hybridoma. Measurement of the antibody titer in an antiserum can be carried out, for example, by reacting the labeled receptor protein, etc. as described hereinafter and an antiserum and then measuring the activity of the labeling agent bound to the antibody. The cell fusion can be carried out according to a known method, for example, the method described by Köhler and Milstein, Nature, 256, 495 (1975). As a fusion promoter, for example, polyethylene glycol (PEG) or Sendai virus (HVJ), preferably PEG can be used.

Examples of myeloma cells include those of warm-blooded animals such as NS-1, P3U1, SP2/0, AP-1 and the like, and P3U1 is preferred. The proportion of the number of antibody producer cells (spleen cells) and the number of myeloma cells to be used is preferably about 1 : 1 to about 20 : 1 and PEG (preferably PEG 1000 - PEG 6000) is added in concentration of about 10% to about 80%. The cell fusion can be carried out efficiently by incubating a mixture of both cells at about 20°C to about 40°C, preferably about 30°C to about 37°C for about 1 minute to about 10 minutes.

Various methods can be used for screening for a hybridoma producing the antibody against the receptor protein, etc. For example, there may be mentioned a method wherein a supernatant of the hybridoma is added to a solid phase (e.g., microplate) to which the receptor protein, etc. antibody is adsorbed directly or together with a carrier, and then an anti-immunoglobulin antibody (if mouse cells are used in cell fusion, anti-mouse immunoglobulin antibody is used) or Protein A labeled with a radioactive substance or an enzyme is added to detect the monoclonal antibody against the receptor protein, etc. bound to the solid phase; and a method wherein a supernatant of the hybridoma is added to a solid phase to which an anti-immunoglobulin antibody or Protein A is adsorbed, and then the receptor protein, etc. labeled with a radioactive substance or an enzyme is added to detect the monoclonal antibody against the receptor protein, etc. bound to the solid phase.

Selection of the monoclonal antibody can be carried out according to a per se known method or its modification. Normally, a medium for animal cells to which HAT (hypoxanthine, aminopterin, thymidine) are added is employed. Any selection and growth medium can be employed in so far as the hybridoma can grow. For example, RPMI 1640 medium containing 1% to 20%, preferably 10% to 20% fetal bovine serum, GIT medium containing 1% to 10% fetal bovine serum (Wako Pure Chemical Industries, Ltd.), a serum-free medium for cultivation of a hybridoma (SFM-101, Nissui Seiyaku) and the like can be used. Normally, the cultivation is carried out at 20°C to 40°C, preferably 37°C for 5 days to 3 weeks, preferably 1 week to 2 weeks under 5% $CO_2$ gas. The antibody titer of the supernatant of a hybridoma culture can be measured according to the same manner as described above with respect to the antibody titer of the anti-protein, etc. in the antiserum.

(b) Purification of monoclonal antibody

Separation and purification of a monoclonal antibody against the receptor protein, etc. can be carried out according the same manner as those of conventional polyclonal antibodies such as separation and purification of immunoglobulins, for example, salting-out, alcoholic precipitation, isoelectric point precipitation, electrophoresis, adsorption and desorption with ion exchangers (e.g., DEAE), ultracentrifugation, gel filtration, or a specific purification method wherein only an antibody is collected with an active adsorbent such as an antigen-binding solid phase, Protein A or Protein G and dissociating the binding to obtain the antibody.

Preparation of polyclonal antibody

A polyclonal antibody against the receptor protein, etc. of the present invention can be prepared by a per se known method or its modification. For example, an immunogen (an antigen against the receptor protein, etc.) itself or a complex thereof with a carrier protein is prepared and a mammal is immunized according to the same manner as that described with respect to the above monoclonal antibody preparation. A material containing the antibody against the receptor protein, etc. of the present invention is recovered from the immunized animal and the antibody is separated and purified.

As to the complex of the immunogen and the carrier protein to be used for immunization of a mammal, any carrier protein and any mixing proportion of the carrier and a hapten can be employed in so far as an antibody against the hapten, which is crosslinked on the carrier and used for immunization, is produced efficiently. For example, bovine serum albumin, bovine cycloglobulin, keyhole limpet hemocyanin, etc. can be coupled to an hapten in a weight ratio of about 0.1 part to about 20 parts, preferably, about 1 part to about 5 parts per 1 part of the hapten.

In addition, various condensing agents can be used for coupling of a hapten and a carrier. For example, there may be mentioned glutaraldehyde, carbodiimide, maleimide activated ester, activated ester reagents containing thiol group or dithiopyridyl group, and the like.

The condensation product as such or together with a suitable carrier or diluent is administered to a site of a mammal which permits the antibody production. For enhancing the antibody productivity, complete Freund's adjuvant or incomplete Freund's adjuvant may be administered. Normally, the protein, etc. is administered once every 2 weeks to 6 weeks, in total, about 3 times to about 10 times.

The polyclonal antibody is recovered from blood, ascites and the like, preferably, blood of an animal immunized by the above method.

The antibody titer of the polyclonal antibody in the antiserum can be measured according to the same manner as that described above with respect to the supernatant of the hybridoma culture. Separation and purification of the antibody can be carried out according to the same separation and purification method of immunoglobulin as that described with respect to the above monoclonal antibody.

The antisense DNA having a nucleotide sequence complementary or substantially complementary to the DNA encoding the receptor protein or partial peptide of the present invention (hereinafter sometimes abbreviated as the DNA of the present invention) may be any antisense DNA in so far as it has a nucleotide sequence complementary or substantially complementary to the DNA of the present invention and has a capability to inhibit expression of the DNA.

Examples of a nucleotide sequence substantially complementary to the DNA of the present invention include a nucleotide sequence having about 70% or more, more preferably about 80% or more, more preferably about 90% or more, most preferably about 95% or more homology to the entire or partial nucleotide sequence of the complementary nucleotide sequence of the DNA of the present invention (i.e., the complementary strand of the DNA of the present invention). In particular, a preferred antisense DNA is that having about 70% or more, preferably about 80% or more, more preferably about 90% or more, most preferably about 95% or more homology to a complementary nucleotide sequence of the nucleotide sequence encoding the N-terminal portion of the receptor protein or its partial peptide in the entire nucleotide sequence of the complementary strand of the DNA of the present invention. These antisense DNA can be prepared by using a known DNA synthesizer, etc.

In view of the structural characteristics, the receptor protein of the present invention is a member of TNF receptor family (e.g., type 1 TNF receptor, type 2 TNF receptor, Fas, CD40, CD30, etc.). Proteins belonging to this family relate to various physiological functions such as differentiation, proliferation and activation of cells, cell death and the like [Cell, 76, 959-962 (1994)]. Since the receptor protein of the present invention is expressed by dendritic cells and plays important roles in transferring signals relating to physiological functions (e.g., cell differentiation, proliferation, activation, etc.) in cells having antigen presenting capability whose representative example is a dendritic cell, the receptor protein of the present invention is considered to be involved in diseases caused by abnormal immune function such as allergic diseases, autoimmune diseases, or diseases accompanied with abnormal immune functions.

The receptor protein, etc. of the present invention or the DNA encoding it can be used for (1) determination of a ligand to the receptor protein of the present invention, (2) obtaining an antibody and antiserum, (3) construction of a expression system of a recombinant receptor protein, (4) development of a receptor binding assay system utilizing the expression system and screening of candidate compounds for medicines, (5) practice of drug design based on comparison with ligands and receptors having structural similarity, (6) preparation of probes or PCR primers in gene diagnosis (7) production of transgenic animals and (8) gene prophylactic or therapeutic drugs, among others.

In particular, a compounds which can alter binding properties of a ligand to a specific receptor in a human being or another mammal (e.g., agonist, antagonist, etc.) can be screened by using a receptor binding assay system utilizing an expression system of the recombinant receptor protein of the present invention and the agonist or antagonist can be used for prophylactic or therapeutic medicines of various diseases.

Hereinafter, utilities of the receptor protein, etc. of the present invention, the DNA of the present invention and the antibody against the receptor protein, etc. of the present invention (hereinafter sometimes abbreviated as the antibody of the present invention) will be specifically illustrated.

(1) Determination of a ligand to the receptor protein of the present invention

The receptor protein, etc. of the present invention is useful as a reagent for determining or investigating a ligand (agonist) to the receptor protein of the present invention.

That is, the present invention provides a method for determination of a ligand to the receptor protein of the present invention comprising allowing contact between the receptor protein, etc. of the present invention and a test compound.

Specifically, the ligand determination method of the present invention is a method for determining a compound (e.g., peptide, protein, non-peptide compound, synthetic compound, fermented product, etc.) which can bind to the receptor protein of the present invention to show a cell-stimulative (stimulating) activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) or its salt by using the receptor protein, etc. of the present invention as such, or by constructing an expression system of the recombinant receptor protein, etc. and using a receptor binding assay system utilizing the expression system.

The ligand determination method of the present invention is characterized by measurement of, for example, an amount of a test compound bound to the receptor protein, etc., or a cell stimulation activity to cells containing the receptor protein, etc. upon allowing contact between the receptor protein, etc. of the present invention and the test compound.

More specifically, the present invention provides:

(a) a method for determination of a ligand to the receptor protein of the present invention or its salt which comprises measuring an amount of a labeled test compound bound to the receptor protein, etc. of the present invention upon allowing contact between the labeled test compound and the receptor protein, etc. of the present invention;
(b) a method for determination of a ligand to the receptor protein to the receptor protein of the present invention or its salt which comprises measuring an amount of a labeled test compound bound to cells containing the receptor protein, etc. of the present invention or a membrane fraction thereof upon allowing contact between the labeled test compound and the cells or the membrane fraction;
(c) a method for determination of a ligand to the receptor protein of the present invention which comprises measuring an amount of a labeled test compound bound to the receptor protein of the present invention or its salt upon allowing contact between the labeled test compound and the receptor protein expressed on a cell membrane by cultivating a transformant comprising the DNA encoding the receptor protein of the present invention;
(d) a method for determination of a ligand to the receptor protein or its salt which comprises measuring a cell-stimulative activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through the receptor protein upon allowing contact between a test compound and cells containing the receptor protein of the present invention; and
(e) a method for determination of a ligand to the receptor protein of the present invention or its salt which comprises measuring a cell-stimulative activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through the receptor protein upon allowing contact between a test compound and the receptor protein expressed on a cell membrane by cultivating the transformant containing the DNA encoding the receptor protein of the present invention.

In particular, preferably, the above test (a)-(c) is carried out to confirm that a test compound binds to the receptor protein of the present invention, followed by the above test (d)-(e).

The receptor protein standard to be used in the ligand determination method may be any of the above-described receptor protein, etc. of the present invention, or cells (e.g., dendritic cell, etc.) containing the receptor protein, etc. of the present invention. Suitably, the receptor protein is expressed in a large amount by using animal cells.

For producing the receptor protein, etc. of the present invention, the above-described expression method is employed. Preferably, the production is carried out by expressing the DNA encoding the receptor protein in mammalian or insect cells. Normally, cDNA is used as a DNA fragment encoding the desired receptor protein region, but the present invention is not necessarily limited thereto. For example, a gene fragment or a synthetic DNA can be used. For introducing the DNA encoding the receptor protein, etc. of the present invention into a host animal cell and expressing it efficiently, it is preferred to integrate the DNA at the downstream from polyhedron promoter of nuclear polyherosis virus (NPV), a member of baculovirus whose host is an insect, a promoter derived from SV40, a promoter of retrovirus, metallothionein promoter, human heat shock promoter, cytomegalovirus promoter, SRa promoter, or the like. A test for the amount and quality of the expressed receptor can be carried out according to a per se known method, for example, that described by Nabmi, P. et al., J. Biol. Chem., 267, 19555-19559 (1992).

Accordingly, the receptor protein, etc. of the present invention to be used in the ligand determination method of the present invention may be the receptor protein, etc. per se purified according to a per se known method, or cells containing the receptor protein, etc. or a membrane fraction thereof may be used.

In the ligand determination method of the present invention, when cells containing the receptor protein, etc. of the present invention are used, the cells can be immobilized with glutaraldehyde, formalin or the like. Immobilization can be carried out according to a per se known method.

The cells containing the receptor protein, etc. of the present invention are host cells which express the receptor protein, etc. of the prevent invention, including cells of *Escherichia coli, Bacillus subtilus,* yeast, insect cells and animal cells.

The cell membrane fraction is a fraction containing a large amount of cell membrane components obtained according to a per se known method after disruption of cells. Disruption of cells can be carried out by, for example, crushing cells with Potter-Elvehjem homogenizer, disrupting cells with Waring blender or Polytoron (manufactured by Kinemtaica), disrupting with ultrasonication or disrupting cells with French press, etc. For fractionation of the cell fraction, a fractionation method utilizing centrifugal force such as differential centrifugation or density gradient centrifugation is mainly employed. For example, a cell disruption mixture is centrifuged at a low rate (500 rpm - 3,000 rpm) for a short period of time (normally, about 1 minute - 10 minutes) and the resultant supernatant is further centrifuged at a high rate (15,000 rpm - 30,000 rpm) for, normally, 30 minutes to 2 hours to obtain the desired cell fraction as a precipitate. The cell membrane fraction contains the expressed receptor protein, etc. and many membrane components derived from cells such as phospholipids, membrane proteins and the like.

The amount of the receptor protein, etc. to be contained in cells or a membrane faction thereof is preferably $10^3$ to $10^8$, more preferably $10^5$ to $10^7$ molecules per one cell. As the expression amount is increased, the ligand binding activity (specific activity) per the membrane fraction is higher, which makes possible not only to construct a high sensitive screening system but also to measure a lot of samples in a single lot.

For carrying out the above methods (a) to (c) for determination of a ligand to the receptor protein of the present invention, a suitable receptor protein fraction and a labeled test compound are required.

The receptor protein faction is desirably a naturally occurring receptor protein fraction or a recombinant receptor fraction having an equivalent activity thereof. The wording "an equivalent activity" means an equivalent (e.g., about 0.01 - 100 times, preferably about 0.5 - 20 times, more preferably about 0.2 - 2 times) ligand binding activity, signal transferring activity or the like.

Examples of the test compounds includes extracts from tissues of a human being and another mammal (e.g., mouse, rat, pig, cattle, sheep, monkey, etc.), cell culture supernatants and the like, in addition to known ligands [e.g., TNF (e.g., TNF-$\alpha$), lymphotoxin (e.g., lymphotoxin-$\alpha$, lymphotoxin-$\beta$), Fas ligand, NGF, CD40 ligand, CD27 ligand, CD30 ligand, OX-40 ligand, 4-1BB ligand, Apo-2L (TRAIL), etc.]. For example, the tissue extract or cell culture supernatant is added to cells containing the receptor protein, etc. of the present invention and fractionation is carried out, while measuring a ligand binding activity, a cell-stimulative activity or the like to obtain a single ligand as an end product.

For labeling the test compound, radioisotopes include [$^3$H], [$^{125}$I], [$^{14}$C], [$^{35}$S] and the like.

Specifically, for carrying out the methods (a) to (c) for determination of a ligand to the receptor protein or its salt of the present invention, for example, cells or a membrane fraction thereof containing the receptor protein of the present invention is suspended in a buffer suitable for the determination method to prepare a receptor standard.

Any buffer can be used in so far as it does not interfere with binding between a ligand and the receptor protein. Examples of the buffer include phosphate buffer of pH about 4 to about 10 (preferably pH about 6 - 8), Tris-HCl buffer and the like. In order to reduce non-specific binding, a surfactant such as CHAPS, Tween-80™ (Kao-Atlas), digitonin, deoxycholate and various proteins such as bovine serum albumin, gelatin and the like can be added to the buffer. Further, in order to prevent degradation of a ligand and the receptor with a protease, a protease inhibitor such as PMSF, leupeptin, E-64 (Peptide Laboratory), pepstatin or the like can also be added. Then, a test compound labeled with a given amount (5,000 cpm - 500,000 cpm) of [$^3$H], [$^{125}$I], [$^{14}$C], [$^{35}$S] or the like is included in 0.01 ml to 10 ml of the solution of the receptor. For counting a non-specific binding (NSB) count, a reaction tube containing an excess amount of an unlabeled test compound is also provided. The reaction is carried out at about 0°C to about 50°C, preferably about 4°C to about 37°C for about 20 minutes to about 24 hours, preferably about 30 minutes to about 3 hours.

After the reaction, the reaction mixture is filtered through a glass fiber filter, etc. and washed with the same buffer and the radioactivity remained on the glass fiber filter is counted with a liquid scintillation counter or $\gamma$-counter. A count (B-NCB) is calculated by subtracting NSB count from the total binding (B) count. The test compound having B-NSB count of excess 0 cpm can be selected as a ligand (agonist) to the receptor protein or its salt of the present invention.

For carrying out the above methods (d) - (e) for determination of a ligand to the receptor protein or its salt of the present invention, a cell-stimulative activity (e.g., activity for promoting or inhibiting activation of NF-$\kappa$B, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through the receptor protein can be measured by a known method or by using a commercially available measuring kit.

Specifically, cells containing the receptor protein, etc. is cultivated in a multi-well plate or the like. On determination of a ligand, a culture medium is changed to a fresh medium or a suitable buffer which is not toxic to the cells in advance and then a test compound and the like are added thereto. The mixture is incubated, and the cells are extracted or the supernatant is recovered, followed by measurement of a product formed according to a method suitable for the product, quantitatively.

Where it is difficult to detect an indicative product of the cell stimulating activity (e.g., arachidonic acid, etc.) to be assayed due to a degradation enzyme contained in the cells, the assay can also be carried out with addition of an inhibitor of the degradation enzyme.

The kit to be used for determination of a ligand to the receptor protein of the present invention comprises as an essential component, for example, the receptor protein, etc. of the present invention, cells containing the receptor protein, etc. of the present invention, or a membrane fraction thereof.

An example of the ligand determination kit of the present invention is as follows.

(i) Ligand determination reagents

(i-a) Measurement buffer and washing buffer

0.05% bovine serum albumin (Sigma) is added to Hank's Balanced Salt Solution (Gibco).

The mixture is filtered aseptically with a filter having a pore size of 0.45 μm and stored at 4°C or it can be prepared when it is used.

(i-b) Receptor protein standard

CHO cells wherein the receptor protein has been expressed is subcultured in a 12-well plate at concentration of $5\times10^5$ cells/well and cultivated at 37°C in 5% $CO_2$-95% air for 2 days.

(i-c) Labeled test compound

A compound labeled with a commercially available [$^3H$], [$^{125}I$], [$^{14}C$], [$^{35}S$] or the like or labeled with another suitable method.

An aqueous solution of the labeled test compound is stored at 4°C or -20°C and is diluted to concentration of 1 μM with the measurement buffer when it is used. In case of a test compound which is slightly soluble in water, it is dissolved in dimethylformamide, DMSO, methanol, etc.

(i-d) Unlabeled test compound

The same compound as that of the labeled test compound is prepared in 100-fold to 1,000-fold higher concentration.

(ii) Measurement

(ii-a) CHO cells expressing the receptor protein of the present invention is cultivated in a 12-well tissue culture plate and washed twice with 1 ml of the measurement buffer, and then 490 μl of the measurement buffer is added to each well.

(ii-b) 5 μl of a labeled test compound was added and reacted at room temperature for 1 hour. For measuring the non-specific binding amount, 5 μl of the unlabeled test compound is added.

(ii-c) The reaction mixture is removed and the well is washed tree times with lml of the washing buffer. The labeled test compound bound to cells is dissolved with 0.2 N NaOH-1% SDS and mixed with 4 ml of liquid scintillator A (Wako Pure Chemical Industries, Limited).

(ii-d) Radioactivity is measured with a liquid scintillation counter (Beckman).

(ii-e) When the radioactivity is reduced, the test compound can be selected as a candidate ligand compound.

(ii-f) The above methods (d)-(e) are carried out by using the candidate ligand compound and a compound having a cell stimulating activity can be selected as a ligand (agonist)

Examples of the ligand which can bind to the receptor protein of the present invention include TNF (e.g., TNF-a), lymphotoxin (e.g., lymphotoxin-α, lymphotoxin-β), Fas ligand, NGF, CD40 ligand, CD27 ligand, CD30 ligand, OX-40 ligand, 4-1BB ligand and Apo-2L (TRAIL), among others.

(2) Therapeutic or prophylactic medicines of deficiency diseases of the receptor protein of the present invention

The DNA encoding the receptor protein or its partial peptide of the present invention (DNA of the present invention) can be used as a medicine for prophylaxis and therapy of deficiency diseases of the receptor protein of the present invention.

For example, where a physiological activity of a ligand is not expected because of deficiency of the receptor protein of the present invention in a living body, an amount of the receptor protein in the body of a patient can be increased by (i) administrating the DNA of the present invention to the patient to express the protein, or (ii) inserting the DNA of the present invention into subject cells to express the protein and implanting the cell into the patient, thereby exhibiting the activity of the ligand sufficiently. Since the DNA of the present invention is safe and low toxic, it is useful as a prophylactic or therapeutic medicine of deficiency diseases of the receptor protein, for example, cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others.

For using the DNA of the present invention as the above-described prophylactic or therapeutic medicine, the DNA of the present invention can be administered to a human being or other warm-blooded animals as it is or, after introduction into a suitable vector such as retroviral vector, adenoviral vector, adenovirus associated viral vector, etc., according to a conventional method. The DNA of the present invention can be administered by a gene gur, or a catheter such as a hydrogel catheter as it is or together with a physiologically acceptable carrier such as an auxiliary for promoting ingestion.

The vector containing the DNA of the present invention can be used in the form of, for example, tablets, if necessary, providing sugar coating, capsules, elixirs, microcapsules, etc. for oral administration, or in the form of injectable preparations such as aseptic solutions or suspensions in water or other pharmaceutically acceptable solutions for parenteral administration. A pharmaceutical composition in a unit dosage form can be prepared by mixing the DNA of the present invention with, for example, one or more pharmaceutically acceptable carriers, flavors, excipients, vehicles, preservatives, stabilizers, binders, etc. according to generally acceptable manner. The effective component is contained in the composition in such an amount that a dose in the intended desired range can be obtained.

Examples of additives to be mixed in tablets, capsules, etc. include binders such as gelatin, corn starch, tragacanth gum and gum arabic, excipients such as crystalline cellulose, swelling agents such as corn starch, gelatin and alginic acid, lubricants such as magnesium stearate, sweeteners such as sucrose, lactose and saccharin, flavors such as peppermint, akamono oil and cherry, and the like. In case of the capsule dosage unit form, in addition to the above component, it can contain a liquid carrier such as fat or oil. An injectable aseptic composition can be prepared according to a conventional manner, for example, by dissolving or suspending the active component in a vehicle such as injectable water and a natural vegetable oil such as sesame oil, coconut oil, etc. Examples of the injectable aqueous solution include physiological saline, isotonic solutions containing glucose and other adjuvants (e.g., D-sorbitol, D-mannitol, sodium chloride, etc.) and suitable dissolution aids, for example, alcohols (e.g., ethanol), polyalcohols (e.g., propylene glycol, polyethylene glycol), nonionic surfactants (e.g., Polysorbate 80™, HCO-50) may be further added. As an oily solution, for example, sesame oil, soybean oil, etc. can be used and a dissolution aid such as benzyl benzoate or benzyl alcohol, etc. can be further added.

The above prophylactic and therapeutic medicines can further contain, for example, buffers (e.g., phosphate buffer, sodium acetate buffer), smoothing agents (e.g., benzalkonium chloride, procaine hydrochloride, etc.), stabilizers (e.g., human serum albumin, polyethylene glycol, etc.), preservatives (e.g., benzyl alcohol, phenol, etc.), antioxidants, and the like. The injectable preparation thus produced is normally filled in a suitable ampoule.

Since the pharmaceutical composition thus obtained is safe and low toxic, it can be administer to a human being and another mammal (e.g., rat, rabbit, sheep, pig, cattle, horse, cat, dog, monkey, etc.).

Although the amount of the DNA of the present invention to be administered is varied according to a particular disease, patient, administration route, etc., in general, for oral administration to an adult human being (as 60 kg body weight) for treatment of cancer, the DNA of the present invention is administered in an amount of about 0.1 mg/day to about 100 mg/day, preferably about 1.0 mg/day to about 50 mg/day, more preferably about 1.0 mg/day to about 20 mg/day. For parenteral administration to an adult human patient (as 60 kg body weight) for treatment of cancer, the DNA of the present invention is advantageous to administer the composition in the form of an injectable preparation in an amount of about 0.01 mg/day to about 30 mg/day, preferably about 0.1 mg/day to about 20 mg/day, more preferably about 0.1 mg/day to about 10 mg/day, though the single dosage is varied according to particular diseases, routes of administration, etc. As to other animals, the composition can be administered in the above amount with converting it into that for the body weight of 60 kg.

(3) Gene diagnosing agent

The DNA of the present invention can be used as a probe for detecting an abnormality of a DNA or mRNA encoding the receptor protein, etc. of the present invention (abnormal gene) in a human being or another mammal (e.g., rat, rabbit, sheep, pig, cattle, horse, cat, dog, monkey, etc.). Therefore, the DNA of the present invention is useful as a gene diagnosing agent for detecting, for example, damage, mutation or lowering of expression of the DNA or mRNA, or increased or excess expression of the DNA or mRNA, and the like.

The gene diagnosis using the DNA of the present invention can be carried out, for example, by a per se known Northern hybridization, PCR-SSCPmethod (Genomics, 5, 874-879 (1989); Proc. Natl. Acad. Sci. USA, 86, 2766-2770 (1989)), or the like. For example, when lowering of expression of the mRNA encoding the receptor protein, etc. of the present invention is detected by Northern hybridization, one can diagnose that a patient is or has high probability of suffering from cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others.

On the other hand, when excess expression of the mRNA is detected by hybridization, one can diagnose that a patient is or has high possibility of suffering from allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others. In addition, when mutation of the DNA is detected by PCR-SSCP method, one can diagnose that a patient is or has high probability of suffering from cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others.

(4) Quantitative analysis of a ligand to the receptor protein of the present invention

Since the receptor protein, etc. of the present invention can bind to a ligand, concentration of the ligand in a living body can be determined at high sensitivity, quantitatively.

The quantitative analysis of the present invention can be used in combination with, for example, a competitive analysis. That is, concentration of a ligand in a specimen to be tested can be determined by allowing contact between the specimen and the receptor protein, etc. of the present invention. Specifically, for example, the quantitative analysis of the present invention can be used according to the methods described in the following (a) and (b) or their modification.

(a) Hiroshi Irie, Ed., Radioimmunoassay, Kodan-sha (1974);
(b) Hiroshi Irie, Ed, Second Series, Radioimmunoassay, Kodan-sha (1979).

(5) Screening of a compound which alters binding properties between the receptor protein of the present invention and a ligand

The screening of a compound which alters binding properties between a ligand and the receptor protein of the present invention (e.g., peptide, protein, non-peptide compound, synthetic compound, fermented product, etc.), or its salt can be carried out efficiently by using the receptor protein, etc. of the present invention as such, or by constructing an expression system of the recombinant receptor protein, etc. and using a receptor protein assay system utilizing the expression system.

Examples of these compounds include (a) a compound having a cell-stimulative activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through the receptor protein (so-called agonist to the receptor protein of the present invention); (b) a compound without the cell-stimulative activity (so-called antagonist to the receptor protein of the present invention); (c) a compound increasing the affinity or avidity between a ligand and the receptor protein of the present invention; or (d) a compound decreasing the affinity or avidity between a ligand and the receptor protein of the present invention. The screening of the above compound (a) is preferably carried out by the above-described ligand determination method.

That is, the present invention provide a method for screening a compound which alters binding properties between a ligand and the receptor protein of the present invention which comprises comparing

(i) an index obtained upon allowing contact between the receptor protein, etc. of the present invention and the ligand, and
(ii) that obtained upon allowing contact among the receptor protein, etc of the present invention, the ligand and a test compound.

The screening method of the present invention is characterized in that an index, for example, an amount of the ligand bound to the receptor protein, etc. of the present invention, or a cell-stimulative activity on cells containing the receptor protein, etc. of the present invention in case of the above (i) and that in case of the above (ii) are measured and they are compared with each other.

More specifically, the present invention provides:

(a) a method for screening a compound which alters binding properties between a ligand and the receptor protein of the present invention, or its salt which comprises measuring and comparing (i) an amount of a labeled ligand bound to the receptor protein, etc. of the present invention upon allowing contact between the labeled ligand and the receptor protein, etc. of the present invention, and (ii) that upon allowing contact among the labeled ligand, a test compound and the receptor protein, etc. of the present invention;
(b) a method for screening a compound which alters binding properties between a ligand and the receptor protein of the present invention, or its salt which comprises measuring and comparing (i) an amount of a labeled ligand bound to cells containing the receptor protein, etc. of the present invention or a membrane fraction thereof upon allowing contact between the labeled ligand and the cells or the membrane fraction, and (ii) that upon allowing contact among the labeled ligand, a test compound and the cells or the membrane fraction;
(c) a method for screening a compound which alters binding properties between a ligand and the receptor protein of the present invention, or its salt which comprises measuring and comparing (i) an amount of a labeled ligand bound to the receptor protein of the present invention or its salt upon allowing contact between the labeled ligand and the receptor protein, etc. expressed on a cell membrane by cultivating a transformant comprising the DNA of the present invention, and (ii) that upon allowing contact among the labeled ligand, a test compound and the receptor protein, etc. expressed on the cell membrane;
(d) a method for screening a compound which alters binding properties between a ligand and the receptor protein of the present invention, or its salt which comprises comparing and measuring (i) a cell-stimulative activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through a receptor upon allowing contact between a material which activates the receptor protein, etc. of the present invention (e.g., a ligand to the receptor protein, etc. of the present invention) and cells containing the receptor protein, etc. of the present invention and (ii) that upon allowing contact among the material which activates the receptor protein, etc. of the present invention, a test compound and the cells; and
(e) a method for screening a compound which alters binding properties between a ligand and the receptor protein of the present invention, or its salt which comprises measuring and comparing (i) a cell-stimulative activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through a receptor upon allowing contact between a material which activates the receptor protein, etc. of the present invention (e.g., a ligand to the receptor protein, etc. of the present invention) and the receptor protein, etc. of the present invention expressed on a cell membrane by cultivating the transformant containing the DNA of the present invention, and (ii) that upon allowing contact among the material which activate the receptor protein, etc. of the present invention, a test compound and the receptor protein etc. of the present invention expressed on the cell membrane.

In particular, before the human receptor protein, etc. of the present invention is available, for screening a receptor agonist or antagonist, it has been required to, first, obtain candidate compounds by using cells, tissues or their cell membrane fractions containing the receptor protein of rat, etc. (primary screening) and, second, carry out a confirmation test for whether the candidate compounds actually inhibit binding between the human receptor protein and a ligand (secondary screening). Since there are other receptor proteins when using cells, tissues or cell membrane fractions as such, it is difficult to screen an agonist or antagonist to the desired receptor protein, actually.

However, by using the human receptor protein of the present invention, for example, the primary screening is not required, which make possible to carry out screening of a compound which inhibits the binding between a ligand and the receptor protein, efficiently. In addition, whether the compound screened is an agonist or an antagonist can be readily evaluated.

The screening method of the present invention will be illustrated specifically.

The receptor protein standard to be used in the screening method of the present invention may be any of the above-described receptor protein, etc. of the present invention, or cells containing it and, cell membrane fractions of mammalian organs containing the receptor protein, etc. of the present invention are preferred. However, since availability of human organs is very low, for example, the human receptor protein, etc. expressed in a large amount by using recombinant techniques is suitable.

For producing the receptor protein, etc. of the present invention, the above-described method is employed. Preferably, expressing the DNA of the present invention in mammalian or insect cells carries out the production. Normally, a cDNA is used as a DNA fragment encoding the desired receptor protein region, but the present invention is not necessarily limited thereto. For example, a gene fragment or a synthetic DNA can be used. For introducing the DNA encoding the receptor protein, etc. of the present invention into host animal cells and expressing it efficiently, it is preferred to integrate the DNA at the downstream from polyhedron promoter of nuclear polyherosis virus (NPV), a member of baculovirus whose host is an insect, a promoter derived from SV40, promoter of retrovirus, metallothionein promoter, human heat shock promoter, CMV (cytomegaloviruspromoter), SRapromoter, or the like. A test for the amount and quality of the expressed receptor can be carried out according to a per se known method, for example, that described by Nabmi, P. et al., J. Biol. Chem., 267, 19555-19559 (1992).

Accordingly, the receptor protein standard to be used in the screening method of the present invention may be the receptor protein, etc. per se purified according to per se known method, or cells containing the receptor protein, etc. or a membrane fraction thereof may also be used.

In the screening method of the present invention, when cells containing the receptor protein, etc. of the present invention are used, the cells can be immobilized with glutaraldehyde, formalin or the like. Immobilization can be carried out according to a per se known method.

The cells containing the receptor protein, etc. of the present invention are host cells which express the receptor protein, etc. of the prevent invention, including cells of *Escherichia coli, Bacillus subtilus,* yeast, insect cells and animal cells.

The cell membrane fraction is a fraction containing a large amount of a cell membrane obtained according to a per se known method after disruption of the cells. Disruption of cells can be carried out by, for example, crushing cells with Potter-Elvehjem homogenizer, disrupting cells with Waring blender or Polytoron (manufactured by Kinemtaica), disrupting with ultrasonication or disrupting cells with French press, etc. For the fractionation, a method utilizing centrifugal force such as differential centrifugation or density gradient centrifugation is mainly employed. For example, a cell disruption mixture is centrifuged at a low rate (500 rpm - 3,000 rpm) for a short period of time (normally, about 1 minute - about 10 minutes) and the resultant supernatant is further centrifuged at a high rate (15,000 rpm - 30,000 rpm) for, normally, about 30 minutes to about 2 hours to obtain the desired cell fraction as a precipitate. The cell membrane fraction contains the expressed receptor protein, etc. and many membrane components derived from the cells such as phospholipids, membrane proteins and the like.

The amount of the receptor protein, etc. to be contained in cells or a membrane faction thereof is preferably about $10^3$ to about $10^8$, more preferably about $10^5$ to about $10^7$ molecules per one cell. As the expression amount is increased, the ligand binding activity (specific activity) per the membrane fraction is higher, which makes possible not only to construct a high sensitive screening system but also to measure a lot of samples in a single lot.

For carrying out the above screening methods (a) to (c) of a compound which alters binding properties between a ligand and the receptor protein, etc. of the present invention, a suitable receptor protein fraction and a labeled ligand are required.

The receptor protein fraction is desirably a naturally occurring receptor protein fraction or a recombinant receptor fraction having an equivalent activity thereof. The wording "an equivalent activity" means an equivalent (e.g., about 0.01 - 100 times, preferably about 0.5 - 20 times, more preferably about 0.2 - 2 times) ligand binding activity, signal transferring activity or the like.

Examples of ligands include those obtained by the above ligand determination method and their analogues.

For labeling a ligand, radioisotopes include [$^3$H], [$^{125}$I], [$^{14}$C], [$^{35}$S] and the like.

Specifically, for carrying out the method for screening a compound which alters binding properties between a ligand and the receptor protein of the present invention, first, cells or a membrane fraction thereof containing the receptor protein of the present invention are suspended in a buffer suitable for the screening to prepare a receptor protein standard.

Any buffer can be used in so far as it does not interfere with binding between a ligand and the receptor protein, including, for example, phosphate buffer of pH about 4-10 (preferably pH about 6-8), Tris-HCl buffer and the like. In order to reduce non-specific binding, a surfactant such as CHAPS, Tween-80™ (Kao-Atlas), digitonin, deoxycholate or the like can be added to the buffer. Further, in order to prevent degradation of a ligand and the receptor with a protease, a protease inhibitor such as PMSF, leupeptin, E-64 (Peptide Laboratory), pepstatin or the like can also be added. Then, a ligand labeled with a given amount (5,000 cpm - 500,000 cpm) added to 0.01 ml to 10 ml of the solution of the receptor. At the same time, about $10^{-4}$ M to about $10^{-10}$ M of a test compound is included in the solution. For counting a non-specific binding (NSB) count, a reaction tube containing an excess amount of an unlabeled ligand is also provided. The reaction is carried out at about 0°C to about 50°C, preferably about 4°C to about 37°C for about 20 minutes to about 24 hours, preferably about 30 minutes to about 3 hours.

After reaction, the reaction mixture is filtered through a glass fiber filter, etc. and washed with the same buffer and the radioactivity remained on the glass fiber filter is counted with a liquid scintillation counter or $\gamma$-counter. A count ($B_0$-NCB) is calculated by subtracting NSB count from the count obtained without any antagonistic material ($B_0$). By taking the count (Bo-NSB) as 100%, the test compound having B-NSB count of, for example, about 80% or less, preferably about 70% or less, more preferably about 50% or less can- be selected as a candidate material having antagonistic activity.

For carrying out the above methods (d)-(e) for screening a compound which alters binding properties between a ligand and the receptor protein or its salt of the present invention, a cell-stimulative activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through the receptor protein can be measured by a known method or by using a commercially available measuring kit.

Specifically, cells containing the receptor protein, etc. ,is cultivated in a multi-well plate or the like. On screening, a culture medium is changed to a fresh medium or a suitable buffer which is not toxic to the cells in advance and then a test compound and the like are added thereto. The mixture is incubated for a given period of time, and the cells are extracted or the supernatant is recovered, followed by measurement of a product formed according to a method suitable for the product, quantitatively.

Where it is difficult for formation of an indicative product of the cell-stimulative activity (e.g., arachidonic acid, etc.) to be assayed due to a degradation enzyme contained in the cells, the assay can also be carried out with addition of an inhibitor of the degradation enzyme.

For screening by measuring a cell-stimulative activity, cells expressing a suitable receptor protein are required. As cells expressing the receptor protein of the present invention, for example, a cell line having the intrinsic receptor protein of the present invention or a cell line expressing the above recombinant receptor protein is preferred.

The test compound to be used in the screening method of the present invention includes, for example, peptide, protein, non-peptide compound, synthetic compound, fermented product, cell extract, plant extract, animal tissue extract and the like. It may be a novel compound or a known compound.

The kit to be used for screening a compound which alters binding properties between a ligand and the receptor protein of the present invention comprises as an essential component the receptor protein, etc. of the present invention, cells containing the receptor protein, etc. of the present invention, or a membrane fraction thereof.

An example of the screening kit of the present invention is as follows.

(i) Screening reagents

(i-a) Measurement buffer and washing buffer

0.05% bovine serum albumin (Sigma) is added to Hank's Balanced Salt Solution (Gibco).

The mixture is filtered aseptically with a filter having a pore size of 0.45 μm and stored at 4°C or it can be prepared when it is used.

(i-b) Receptor protein standard

CHO cells wherein the receptor protein of the present invention has been expressed is subcultured in a 12-well plate at concentration of $5 \times 10^5$ cells/well and cultivated at 37°C in 5% $CO_2$-95% air for 2 days.

(i-c) Labeled ligand

A ligand labeled with a commercially available [$^3$H], [$^{125}$I], [$^{14}$C], [$^{35}$S] or the like.

An aqueous solution thereof is stored at 4°C or -20°C and is diluted to concentration of 1 μM with the measurement buffer when it is used.

(i-d) Ligand standard

The ligand is dissolved in PBS containing 0.1% bovine serum albumin (Sigma) so that the final volume is 1 mM and stored at -20°C.

(ii) Measurement

(ii-a) CHO cells expressing the receptor protein of the present invention is cultivated in a 12-well tissue culture plate and washed twice with 1 ml of the measurement buffer, and then 490 µl of the measurement buffer is added to each well.

(ii-b) 5 µl of a $10^{-3}$ to $10^{-10}$ M test compound solution was added and then 5 µl of a labeled ligand was added. Then the mixture was reacted at room temperature for 1 hour. For measuring the non-specific binding amount, 5 µl of $10^{-3}$ M ligand is added instead of the test compound.

(ii-c) The reaction mixture is removed and the well is washed tree times with 1 ml of the washing buffer. The labeled ligand bound to cells is dissolved with 0.2 N NaOH-1% SDS and mixed with 4 ml of liquid scintillator A (Wako Pure Chemical Industries, Limited).

(ii-d) Radioactivity is measured with a liquid scintillation counter (Beckman). Percent Maximum Binding (PMB) is calculated from the following equation:

$$PMB=[(B-NSB)/(B_0-NSB)] \times 100$$

wherein PMB is Percent Maximum Binding, B is the value obtained by addition of a sample, NSB is non-specific binding and Bo is the maximum binding value.

The compound obtained by the screening method and the kit for screening of the present invention, or its salt is an active compound in altering binding properties between a ligand and the receptor protein of the present invention. Specifically, the compound is (a) a compound having a cell-stimulative activity (e.g., activity for promoting or inhibiting activation of NF-κB, activation of TRAF molecule binding, release of arachidonic acid, release of acetylcholine, release of intracellular $Ca^{2+}$, intracellular formation of cAMP, intracelluler formation of cGMP, production of inositol triphosphate, change in cell membrane potential, phosphorylation of intracellular proteins, activation of c-fos, lowering of pH, etc.) mediated through the receptor protein (so-called agonist to the receptor protein of the present invention); (b) a compound without the cell stimulation activity (so-called antagonist to the receptor protein of the present invention); (c) a compound increasing the affinity or avidity between a ligand and the receptor protein of the present invention; or (d) a compound decreasing the affinity or avidity between a ligand and the receptor protein of the present invention.

The compound can be obtained from the above-described test compound and includes peptide, protein, non-peptide compound, synthetic compound, fermented product or the like. The compound may be a novel compound or a known compound.

An agonist to the receptor protein of the present invention has a similar activity to the physiological activity of a ligand to the receptor protein of the present invention, and there, it is useful as a safe and low toxic medicine according to a particular activity of the ligand. The agonist is useful as medicine for prophylaxis and therapy of cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others.

An antagonist to the receptor protein of the present invention can inhibit physiological activities of a ligand to the receptor protein of the present invention. Therefore, it is useful as safe and low toxic medicine. The antagonist is useful as medicine for prophylaxis or therapy of allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others.

A compound increasing the affinity of avidity between a ligand and the receptor protein of the present invention is a safe and low toxic medicine for increasing physiological activity having the ligand to the receptor protein of the present invention. The compound is useful as medicine for prophylaxis or therapy of cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others.

A compound which decreases the affinity of avidity between a ligand and the receptor protein of the present invention is a safe and low toxic medicine for decreasing physiological activity having the ligand to the receptor protein of the present invention. The compound is useful as medicine for prophylaxis or therapy of allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others.

The compound obtained by using the screening method or the kit for screening of the present invention can be used as the above-described prophylactic or therapeutic medicine according to a conventional method. For example, it can be formulated into, for example, tablets, capsules, elixirs, microcapsules, aseptic solution, suspension, etc. according to the same manner as that containing the DNA of the present invention.

Since the pharmaceutical composition thus obtained is safe and low toxic, it can be administer to a human being and another mammal (e.g., rat, rabbit, sheep, pig, cattle, horse, cat, dog, monkey, etc.).

Although the amount of the compound or its salt to be administered is varied according to a particular disease, patient, administration route, etc., in general, for oral administration to an adult human being (as 60 kg body weight) for treatment of cancer, the agonist is administered in an amount of about 0.1 mg/day to about 100 mg/day, preferably about 1.0 mg/day to about 50 mg/day, more preferably about 1.0 mg/day to about 20 mg/day. For parenteral administration to an adult human patient (as 60 kg body weight) for treatment of cancer, the agonist is advantageous to administer the composition in the form of an injectable preparation in an amount of about 0.01 mg/day to about 30 mg/day, preferably about 0.1 mg/day to about 20 mg/day, more preferably about 0.1 mg/day to about 10 mg/day, though the single dosage is varied according to particular diseases, routes of administration, etc. As to other animals, the composition can be administered in the above amount with converting it into that for the body weight of 60 kg.

Further, for oral administration to an adult human being (as 60 kg body weight) for treatment of allergic immunological diseases, the antagonist is administered in an amount of about 0.1 mg/day to about 100 mg/day, preferably about 1.0 mg/day to about 50 mg/day, more preferably about 1.0 mg/day to about 20 mg/day. For parenteral administration to an adult human patient (as 60 kg body weight) for treatment of allergic immunological diseases, the antagonist is advantageous to administer the composition in the form of an injectable preparation in an amount of about 0.01 mg/day to about 30 mg/day, preferably about 0.1 mg/day to about 20 mg/day, more preferably about 0.1 mg/day to about 10 mg/day, though the single dosage is varied according to particular diseases, routes of administration, etc. As to other animals, the composition can be administered in the above amount with converting it into that for the body weight of 60 kg.

(6) Quantitative assay of the receptor protein, its partial peptide or their salts

Since the antibody against the protein, etc. of the present invention can recognize the receptor protein, etc. of the present invention specifically, it can be used for quantitative assay of the protein, etc. of the present invention in a specimen fluid, in particular, determination by a sandwich immunoassay or the like. That is, the present invention provides, for example,

(a) a quantitative assay method of the receptor protein, etc. of the present invention in a specimen fluid which comprises reacting the antibody against the protein, etc. of the present invention with the specimen fluid and the labeled protein, etc. of the present invention, competitively, and measuring the proportion of the labeled protein, etc. of the present invention bound to the antibody; and

(b) a quantitative assay method of the receptor protein, etc. of the present invention in a specimen fluid which comprises reacting the specimen fluid with one antibody of the present invention insolubilized on a carrier and another labeled antibody of the present invention, simultaneously or continuously and measuring the activity of the labeling agent on the insolubilized carrier.

In the above method (b), preferably, one antibody recognizes the N-terminal of the receptor protein, etc. of the present invention and the other antibody reacts with the C-terminal of the receptor protein, etc. of the present invention.

In the present invention, the monoclonal antibody against the receptor protein, etc. of the present invention (hereinafter sometimes referred to as the monoclonal antibody of the present invention) can be used for assaying the receptor protein, etc. of the present invention and further it can be used for detection by histological stains and the like. For these purposes, the antibody molecule as such can be used or $F(ab')_2$, Fab' or Fab fraction of the antibody molecule can also be used.

An assay using an antibody against the receptor protein, etc. of the present invention is not specifically limited and any assay method can be used in so far as an amount of an antigen (e.g., an amount of the receptor protein), antibody or antibody-antigen complex corresponding to an amount of an antigen in a fluid to be determined can be detected by a chemical or physical means and calculated based on a calibration curve prepared by using standard solutions containing known amounts of the antigen. For example, nephelometry, competitive method, immunometric method and sandwich method are suitably employed. In particular, in view of sensitivity, specificity and the like, a sandwich method as described hereinafter is preferred.

As a labeling agent used in the assay method using a labeled reagent, radioisotopes, enzymes, fluorescent materials, luminous materials and the like can be used. Examples of radioisotopes include [$^{125}$I], [$^{131}$I], [$^{3}$H], [$^{14}$C] and the like. As the above enzymes, that having good stability and high specific activities is preferred and, for example, there are β-galactosidase, β-glucosidase, alkaline phosphatase, peroxidase, malate dehydrogenase and the like. As the fluorescent materials, for example, there are fluorescamine, fluorescein isothiocyanate and the like. As the luminous materials, there are luminol, luminol derivatives, luciferin, lucigenin and the like. In addition, biotin-avidin system can be used for binding of an antibody or antigen to a labeling agent.

For insolubilization of an antigen or antibody, physical adsorption can be used or, normally, a method using a chemical bond for insolubilizing or immobilizing a protein or an enzyme can be used. Examples of the carrier include insoluble polysaccharides such as agarose, dextran, cellulose and the like, synthetic resins such as polystyrene, polyacrylamide, silicone and the like, glass and the like.

In a sandwich method, a specimen fluid to be tested is reacted with an insolubilized anti-protein antibody (primary reaction) and further reacting a labeled anti-protein antibody (secondary reaction), followed by measuring the labeling agent on the insoluble carrier to determine the amount of the receptor protein, etc. of the present invention in the specimen fluid. The order of the primary and secondary reactions can be reversed and they can be carried out simultaneously or separately at different times. The above-described labeling agent and insolubilization can be applied to this method. In addition, in an immunoassay by a sandwich method, an antibody to be used as the solid phase antibody or labeled antibody is not necessary one kind of antibodies and, in order to improve measuring sensitivity, etc., a mixture of two or more kinds of antibodies can be used.

In the assay method of the receptor protein, etc. by the sandwich method of the present invention, preferably, the antibodies against the receptor protein, etc. used in the primary and secondary reactions are those having different binding sites for the receptor protein. For example, when the antibody used in the secondary reaction is that recognizing the C-terminal region of the receptor protein, etc., preferably, the antibodies used in the primary reaction is that recognizing an region other than the C-terminal region, for example, the N-terminal region.

The monoclonal antibody of the present invention can also be used for a measuring system other than a sandwich method, for example, a competitive method, immunometric method, nephelometry and the like. In a competitive method, an antigen in a specimen fluid and a labeled antigen are reacted with the antibody competitively and, after separation of the unreacted labeled antigen (F) from the labeled antigen bound to the antibody (B), measuring the amount of the labeling agent of either B or F to determine the amount of the antigen in the specimen fluid. In this reaction, both liquid phase method and solid phase method can be employed. In the liquid phase method, a soluble antibody is used as the antibody and B/F separation can be carried out by using polyethylene glycol, a second antibody against the above antibody. In the solid phase method, an immobilized solid phase antibody is used as the first antibody, or a soluble antibody is used as the first antibody and an immobilized solid phase antibody is used as the second antibody.

In the immunometric method, an antigen in a specimen fluid to be tested and an immobilized solid phase antigen are reacted with a given amount of a labeled antibody, competitively and then the solid phase is separated from the liquid phase. Alternatively, an antigen in a specimen fluid to be tested is reacted with an excess amount of a labeled antibody and an immobilized solid phase antigen is added to permit the unreacted labeled antibody to bind to the solid phase, followed by the separation of the solid phase from the liquid phase. Then, the amount of the labeling agent of either phase is measured to determine the amount of the antigen in the specimen fluid.

In nephelometry, an antigen-antibody reaction is carried out in a gel or solution and the amount of an insoluble precipitate formed is measured. Even when the amount of an antigen in a specimen fluid to be tested is small and the amount of a precipitate formed is small, laser nephelometry wherein diffusion of laser is utilized can be suitably employed.

When employing these immunoassay methods in the determination method of the present invention, to set any special conditions, procedures and the like is not required. That is, the determination system of the receptor protein, etc. of the present invention can be constructed based on conventional conditions and procedures in respective methods together with conventional artisan's technical consideration.

As for details of these general technical means, reference can be made to various reviews, texts and the like, for example, Hiroshi Irie, Ed., Radioimmunoassay, Kodan-sha (1974); Hiroshi Irie, Ed, Second Series, Radioimmunoassay, Kodan-sha (1979); Eizi Ishikawa et al., Ed., Enzyme Immunoassay, Igaku-shoin (1978); Eizi Ishikawa et al., Ed., Second Series, Enzyme Immunoassay, Igaku-shoin (1982); Eizi Ishikawa et al., Third Series, Enzyme Immunoassay, Igaku-shoin (1987); Method in Enzymology, Vol. 70, Immunochemical Techniques (Part A)), Academic Press; ibid., Vol. 73, Immunochemical Techniques (Part B); ibid., Vol. 74, Immunochemical Techniques (Part C); ibid., Vol. 84, Immunochemical Techniques (Part D: Selected Immunoassays); ibid., Vol. 92, Immunochemical Techniques (Part E: Monoclonal Antibodies and General Immunoassay Methods); ibid., Vol. 121, Immunochemical Techniques (Part I: Hybridoma Technology and Monoclonal Antibodies) and the like.

As described hereinabove, the receptor protein, etc. of the present invention can be determined at high sensitivity by using the antibody of the present invention. In addition, the antibody of the present invention can be used for detecting the protein, etc. present in a specimen fluid such as a body fluid, tissue, etc. Further, detection of the receptor protein, etc. of the present invention by using the antibody of the present invention can be utilized in diagnosis of various diseases involving the receptor protein of the present invention. Specifically, when decrease in concentration of the receptor protein, etc. of the present invention is detected, one can diagnose that a patient is or has high probability of suffering from cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others.

On the other hand, when increase in concentration of the receptor protein, etc. is detected, one can diagnose that a patient is or has high possibility of suffering from allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others.

Thus, the antibody of the present invention is useful for a diagnosing agent of the above diseases.

In addition, the antibody of the present invention can be used for detecting the receptor protein, etc. of the present invention present in a specimen such as a body fluid, tissue or the like. Further, it can be used for preparation of an antibody column to be used for purification of the receptor protein, etc. of the present invention, detection of the receptor protein, etc. of the present invention in each fraction obtained during purification thereof, analyzing behavior of the receptor protein, etc. of the present invention in cells to be tested, and the like.

(7) Medicines containing a soluble partial peptide of the present invention

The soluble partial peptide of the present invention can bind to a ligand to inhibit binding between the ligand and the receptor protein of the present invention to inhibit physiological activities of a ligand to the receptor protein of the present invention. Therefore, the soluble partial peptide of the present invention is a useful medicine for prophylaxis or therapy of allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others.

For using the soluble partial peptide of the present invention as medicine, it can be prepared according to the same manner as a medicine containing the above DNA of the present invention and can be administered to a human being or another mammal.

Since the pharmaceutical composition thus obtained is safe and low toxic, it can be administer to a human being and another mammal (e.g., rat, rabbit, sheep, pig, cattle, horse, cat, dog, monkey, etc.).

Although the amount of the soluble partial peptide of the present invention to be administered is varied according to a particular disease, patient, administration route, etc., in general, for oral administration to an adult human being (as 60 kg body weight) for treatment of allergic immunological diseases, the soluble partial peptide of the present invention is administered in an amount of about 0.1 mg/day to about 100 mg/day, preferably about 1.0 mg/day to about 50 mg/day, more preferably about 1.0 mg/day to about 20 mg/day. For parenteral administration to an adult human patient (as 60 kg body weight) for treatment of autoimmune diseases, the soluble partial peptide of the present invention is advantageous to administer the composition in the form of an injectable preparation in an amount of about 0.01 mg/day to about 30 mg/day, preferably about 0.1 mg/day to about 20 mg/day, more preferably about 0.1 mg/day to about 10 mg/day, though the single dosage is varied according to particular diseases, routes of administration, etc. As to other animals, the composition can be administered in the above amount with converting it into that for the body weight of 60 kg.

(8) Medicines containing the antibody of the present invention

The antibody which functions to bring about the receptor activity of the receptor protein, etc. of the present invention (agonistic activity) is useful as a medicine for prophylaxis or therapy of, for example, cancer (e.g., breast cancer, prostate cancer, ovarian cancer, follicular lymphoma, cancer accompanied by p53 mutation, brain tumor, bladder carcinoma, cancer of uterine carvix, cancer of large intestine (carcinoma of colon and rectum), non-small cell lung cancer, small cell lung cancer, gastric cancer, etc.), AIDS, infections (e.g., herpesvirus infection, adenovirus infection, poxvirus infection, *Helicobacter pylori* infection, varicella-zoster virus infection, human papillomavirus infection, active staphylococcal infection, influenza virus infection, severe systemic mycosis, etc.), acute bacterial periostitis, acute viral encephalitis, adult respiratory distress syndrome, bacterial pneumonia, chronic lymphocytic leukemia, chronic myelogenous leukemia, insulin-dependent diabetes mellitus (type I), malignant melanoma, multiple myeloma, non-Hodgkin lymphoma, peptic ulcer, sepsis, septic shock and tuberculosis, among others.

On the other hand, the antibody of the present invention which functions to neutralize the biological activity of the receptor protein, etc. of the present invention (antagonistic activity) is useful as a medicine for prophylaxis or therapy of, for example, allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others.

The above prophylactic or therapeutic medicine containing the antibody of the present invention can be administered as such in the form of a liquid preparation or a suitable pharmaceutical composition to a human being or another mammal (e.g., rat, rabbit, sheep, pig, cattle, cat, dog, monkey, etc.), orally or parenterally. Although the amount of the antibody of the present invention to be administered is varied according to a particular disease, patient, administration route, etc., for example, for preventing and treating cancer of an adult human being, normally, advantageous results can be obtained by administering the antibody which brings about the biological activity of the receptor protein of the present invention in an amount of about 0.01 mg/kg body weight to about 20 mg/kg body weight, preferably about 0.1 mg/kg body weight to about 10 mg/kg body weight, more preferably about 0.1 mg/kg body weight to about 5 mg/kg body weight as a single dose and once to about 5 times/day, preferably once to about 3 times/day by an intravenous injection. And, for example, for preventing and treating allergic immunological diseases of an adult human being, normally, advantageous results can be obtained by administering the antibody which neutralizes the biological activity of the receptor protein of the present invention in an amount of about 0.01 mg/kg body weight to about 20 mg/kg body weight, preferably about 0.1 mg/kg body weight to about 10 mg/kg body weight, more preferably about 0.1 mg/kg body weight to about 5 mg/kg body weight as a single dose and once to about 5 times/day, preferably one to about 3 times/day by an intravenous injection. As to other oral and parenteral administration, the antibody of the present invention can be administered according to the above doses. In case of particularly severe conditions, the doses can be increased according to the conditions.

The antibody of the present invention having agonistic or antagonistic activities can be administered as such or in the form of suitable pharmaceutical compositions. The pharmaceutical composition to be used for the above administration comprises the above antibody or its salt and a pharmacologically acceptable carrier, diluent or excipient. Such composition can be provided as a composition suitable for oral or parenteral administration.

That is, examples of pharmaceutical compositions suitable for oral administration are tablets (including sugar coated tablets, film coated tablets), pills, granules, powders, capsules (including soft capsules), syrups, emulsions, suspensions and the like. Such compositions can be produced according to a per se known method and contains a conventional carrier, diluent or excipient. For example, carriers or excipients for tablets includes lactose, starch, sugar, magnesium stearate and the like.

Examples of pharmaceutical compositions suitable for parenteral administration are injectable preparations, suppositories and the like. The injectable preparations include preparations for intravenous injection, subcutaneous injection, intracutaneous injection, intramuscular injection, instillation and the like. Such injectable preparations can be produced according to a per se known method, for example, by dissolving, suspending or emulsifying the above antibody or its salt in aseptic aqueous or oily solutions to be used in conventional injectable preparations. Examples of the injectable aqueous solution include physiological saline, isotonic solutions containing glucose and other adjuvants and suitable dissolution aids, for example, alcohols (e.g., ethanol), polyalcohols (e.g., propylene glycol, polyethylene glycol), nonionic surfactants (e.g., Polysorbate 80™, HCO-50 (polyoxyethylene (50 mol) adduct of hydrogenated caster oil)) may be further added. As an oily solution, for example, sesame oil, soybean oil, etc. can be used and a dissolution aid such as benzyl benzoate or benzyl alcohol, etc. can be further added. The injectable preparations thus produced is normally filled in a suitable ampoule. Suppositories used for rectal administration can be prepared by mixing the above antibody or its salt with conventional carriers for suppositories.

Advantageously, the above pharmaceutical compositions for oral or parenteral administration are prepared in the form of unit dosage forms suitable for particular amounts of the active components to be administered. Such compositions can be prepared in the form of, for example, tablets, pills, capsules, injectable preparations (filled in ampoules), suppositories and the like. Normally, the above antibody is contained in the composition in an amount of 5 to 500 mg/unit dose, in particular, 5 to 100 mg/unit dose for an injectable preparation and 10 to 250 mg/unit dose for other composition.

The above compositions may contain other components in so far as they are undesired for combining the above antibody.

(9) Medicines containing the antisense DNA

An antisense DNA which can inhibit expression of the DNA of the present invention by complementary binding to the DNA of the present invention can inhibit the functions of the receptor protein, etc. or DNA (mRNA) of the present invention. Then, it can be used as medicines for prophylaxis or therapy of various diseases, for example, allergic immunological diseases (e.g., atopic dermatitis, contact dermatitis, allergic rhinitis, pollen allergy, etc.), inflammation (e.g., arthritis, hepatitis, etc.), autoimmune diseases (e.g., rheumatoid arthritis, disseminated lupuserythematodes, Sjögren's disease, etc.), AIDS, glomerulonephritis and bronchial asthma, among others.

For using the above antisense DNA as medicines, they can be formulated into pharmaceutical compositions according to the same manner as the compositions containing the DNA of the present invention and administer to a human being or other mammals. The pharmaceutical compositions thus obtained are safe and low toxic and they can be administered to (e.g., rat, rabbit, sheep, pig, cattle, cat, dog, monkey, etc.).

Although the amount of the antisense DNA of the present invention to be administered is varied according to a particular disease, patient, administration route, etc., in general, for preventing or treating allergic immunological disease by oral administration to an adult human being (as 60 kg body weight), the DNA is administered in an amount of about 0. 1 mg/day to about 100 mg/day, preferably about 1.0 mg/day to about 50 mg/day, more preferably about 1.0 mg/day to about 20 mg/day. For preventing or treating autoimmune diseases by parenteral administration to an adult human being (as 60 kg body weight), for example, in the form of an injectable preparation, the DNA can be administered in an amount of about 0.01 mg/day to about 30 mg/day, preferably about 0.1 mg/day to about 20 mg/day, more preferably about 0.1 mg/day to 10 mg/day intravenously, though the single dose of the DNA is varied according to particular diseases, routes of administration, etc. As for other animals, it can be administered in the above amount with converting it into that for the body weight of 60 kg.

(10) Creation of non-human animal having the DNA of the present invention

A transgenic non-human animal can be created by using the DNA of the present invention. Examples of non-human animals include mammals (e.g., rat, mouse, rabbit, sheep, pig, cattle, cat, dog, monkey, etc.) (hereinafter simply referred to as animal) and, among others, mouse and rabbit are preferred.

For transferring the DNA of the present invention into a subject animal, in general, it is advantageous to use the DNA as a gene construct wherein the DNA is joined to the downstream from a promoter which can express the DNA in animal cells. For example, for transferring the DNA of the present invention derived from rabbit, a gene construct wherein the DNA is joined to the downstream from a promoter which is derived from an animal having high homology thereto and can express the DNA in the animal cells can be transferred into rabbit fertilized egg by microinjection to create the tarnsgenic animal of the present invention which can produce the receptor protein, etc. of the present invention in a high yield. As the promoter, for example, ubiquitous expression promoters such as those derived from viruses, metallothioneine, and the like can also be used.

Transfer during a fertilized egg cell stage secures the DNA of the present invention so that the DNA exists in all the embryo cells and somatic cells of the subject animal. The presence of the receptor protein, etc. of the present invention in the embryo cells of the created animal after DNA transfer means that all the descendants of the created animal have the receptor protein, etc. of the present invention in their embryo cells and somatic cell. Descendants of this kind of animals that have succeeded to the gene have the receptor protein, etc. of the present invention in all their embryo cells and somatic cell.

Upon confirming that a gene can be maintained stably by mating, the DNA transfer animal of the present invention can be subjected to passage breeding in a normal breeding environment. In addition, homozygote animals that have the introduced gene on both homologous chromosomes can be obtained by mating of male and female animals having the desired DNA and proliferation passage can be carried out by mating of male and female homozygote animals so that all the descendants have the DNA.

In the DNA-transferred animal of the present invention, the receptor protein, etc. of the present invention is highly expressed and then the animal is useful for screening of agonists or antagonists to the receptor protein, etc. of the present invention.

The DNA-transferred animal of the present invention can also be used as a cell source of a tissue culture. For example, an analysis for the receptor protein, etc. of the present invention can be carried out by analyzing DNA or RNA in a tissue of the DNA-transferred mouse of the present invention, directly, or by analyzing tissue having the receptor protein, etc. of the present invention expressed by a gene. Tissues having the receptor protein, etc. of the present invention are cultivated according a standard tissue culture technique and, by using them, functions of cells derived from a tissue such as brain and peripheral tissues (e.g., skin, etc.) which is generally difficult to cultivate can be studied. In addition, by using such cells, for example, it is possible to select medicines which can increase functions of various tissues. Further, when a cell line highly expresses the receptor protein, etc. of the present invention, it can be isolated and purified therefrom.

In the specification and drawings, the abbreviations of bases, amino acids and the like are those according to IUPAC-IUB Commission on Biochemical Nomenclature or those conventionally used in the art. The examples are as follows. When the amino acid has an optical isomer, the amino acid is L-isomer unless otherwise stated.

| | |
|---|---|
| DNA : | deoxyribonucleic acid |
| CDNA : | complementary deoxyribonucleic acid |
| A : | adenine |
| T : | thymine |
| G : | guanine |
| C : | cvtosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecylsulfate |
| Gly : | glycine |
| Ala : | alanine |
| Val : | valine |
| Leu : | leucine |
| Ile : | isoleucine |
| Ser : | serine |
| Thr : | threonine |
| Cys : | cysteine |
| Met : | methionine |
| Glu : | glutamic acid |
| Asp : | aspartic acid |
| Lys : | lysine |
| Arg : | arginine |
| His : | histidine |
| Phe : | phenylalanine |
| Tyr : | tyrosine |
| Trp : | tryptophan |
| Pro : | proline |
| Asn : | asparagine |
| Gln : | glutamine |
| pGlu : | pyroglutamic acid |
| Me : | methyl group |
| Et : | ethyl group |
| Bu : | butyl group |
| Ph : | phenyl group |
| TC : | thiazolidin-4 (R) -carboxamide group |

The substituents, protecting groups and reagents often used herein are shown by the following abbreviations.

| | |
|---|---|
| Tos : | p-toluenesulfonyl |
| CHO : | formyl |
| Bzl : | benzyl |

$Cl_2Bzl$ : 2,6-dichlorobenzyl
Bom : benzyloxymethyl
Z : benzyloxycarbonyl
Cl-Z : 2-chlorobenzyloxycarbonvl
Br-Z : 2-bromobenzyloxycarbonyl
Boc : t-butoxycarbonyl
DNP : dinitrophenol
Trt : trityl
Bum : t-butoxymethyl
Fmoc : N-9-fluorenylmethoxycarbonyl
HoBt : 1-hydroxybenztriazole
HOOBt : 3,4-dihydro-3-hydroxy-4-oxo-1,2,3-benzotriazine
HONB : 1-hydroxy-5-norbornen-2,3-dicarboxyimide
DCC : N,N'-dicylcohexylcarbodiimide

The sequences in the Sequence Listing of the present specification are as follows.

SEQ ID NO: 1

This sequence represents an amino acid sequence of the receptor protein of the present invention derived from human dendritic cell.

SEQ ID NO: 2

This sequence represents an amino acid sequence of the soluble partial peptide of the present invention.

SEQ ID NO: 3

This sequence represents an amino acid sequence of the signal peptide of the present invention.

SEQ ID NO: 4

This sequence represents a nucleotide sequence of DNA encoding the receptor protein of the prevent invention derived from human dendritic cells and having the amino acid sequence represented by SEQ ID NO: 1.

SEQ ID NO: 5

This sequence represents a nucleotide sequence of DNA encoding the partial peptide having the amino acid sequence represented by SEQ ID NO: 2.

SEQ ID NO: 6

This sequence represents a nucleotide sequence of DNA encoding the signal peptide having the amino acid sequence represented by SEQ ID NO: 3.

SEQ ID NO: 7

This sequence represents a nucleotide sequence of a synthetic primer used in Example 1 hereinafter for amplification of cDNA encoding the receptor protein of the present invention derived from human dendritic cell.

SEQ ID NO: 8

This sequence represents a nucleotide sequence of a synthetic primer used in Example 1 hereinafter for amplification of cDNA encoding the receptor protein of the present invention derived from human dendritic cell.

SEQ ID NO: 9

This sequence represents a nucleotide sequence of a synthetic primer used in Examples 3 and 4 hereinafter for amplification of cDNA encoding the receptor protein of the present invention derived from human dendritic cell.

SEQ ID NO: 10

This sequence represents a nucleotide sequence of a synthetic primer used in Example 3 hereinafter for amplification of cDNA encoding the receptor protein of the present invention derived from human dendritic cell.

SEQ ID NO: 11

This sequence represents a nucleotide sequence of a synthetic primer used in Example 3 hereinafter for amplification of cDNA encoding the receptor protein of the present invention derived from human dendritic cell.

SEQ ID NO: 12

This sequence represents a nucleotide sequence of a synthetic primer used in Example 4 hereinafter for amplification of cDNA encoding the receptor protein of the present invention derived from human dendritic cell.

SEQ ID NO: 13

This sequence represents an amino acid sequence of a synthetic peptide used in Example 2 hereinafter for determination of antibody titer.

SEQ ID NO: 14

This sequence represents an amino acid sequence of a synthetic peptide used in Example 2 hereinafter for determination of antibody titer.

SEQ ID NO: 15

This sequence represents a nucleotide sequence of a synthetic primer used in Example 5 hereinafter.

SEQ ID NO: 16

This sequence represents a nucleotide sequence of a synthetic primer used in Example 5 hereinafter.

SEQ ID NO: 17

This sequence represents a nucleotide sequence of a synthetic primer used in Example 5 hereinafter.

SEQ ID NO: 18

This sequence represents a nucleotide sequence of a synthetic primer used in Example 5 hereinafter.

The transformant *Escherichia coli* DH 10B/pTB1984 obtained in Example 1 hereinafter has been deposited with National Institute of Bioscience and Human-Technology (NIBH), Agency of Industrial Science & Technology Ministry of International Trade & Industry (1-3, Higasi 1-chome, Tsukuba-shi Ibaraki, 305 Japan) according to the Budapest Treaty under the accession number of FERM BP-6102 since September 16, 1997 and also deposited with Institute for Fermentation Osaka (IFO, 17-85, Juso-honmachi 2-chome Yodogawa-ku, Osaka, 532 Japan) under accession number of IFO 16113 since September 17, 1997.

The following examples further illustrate the present invention in detail but are not to be construed to limit the scope thereof. Gene manipulation using *Escherichia coli* was carried out according to the method described in Molecular Cloning, 2nd Edition (1989).

Example 1

Cloning of cDNA encoding a receptor protein derived from human dendritic cell and determination of its nucleotide sequence

DNA encoding the receptor protein of the present invention was obtained by the following PCR method. First, *Escherichia coli* DH10B of a human dendritic cell cDNA library (Human Genome Science, Library ID H0521) stored in a frozen state was incubated with shaking in Terrific Broth (12 g/l of bacto-tryptone (Difco), 24 g/l of bacto-yeast extract (Difco), 2.3 g/l of potassium dihydrogen phosphate and 12.5 g/l of potassium monohydrogen phosphate) containing 100 μg/ml of ampicillin sodium (Wako Pure Chemical Industries, Limited) at 30° C for 16 hours to amplify the plasmid DNA. Then, the plasmid DNAs were purified by Qiagen Plasmid Kit (Quiagen) to obtain the DNA solution. Two oligo DNA:

5'-GCCAGCCTGTCCCGCGCCATG-3' (SEQ ID NO: 7)

5'-CAGAATATGAAAGTCCCACCACAT-3' (SEQ ID NO: 8)

were synthesized by using a DNA synthesizer (Oligo 1000M, Beckman). PCR was carried out by preparing 50 μl of a mixture containing 0.63 μg of the library DNA, each 3.2 pmol of these two oligo DNA, 8 μl of dNTP mixture contained in Takara LA PCR™ Kit Ver. 2 (Takara Shuzo), 5 μl of 10×LA PCR Buffer II ($Mg^{2+}$ plus) and 5.0 Unit of Takara LA Taq™ and by using a thermalcycler GeneAmp™ PCR system 2400 (Perkin Elmer). The temperature cycle program was first 1 minute at 94°C; and then 20 seconds at 98°C and 15 minutes at 68°C, for 30 cycles. After completion of the reaction, the reaction mixture was subjected to electrophoresis with 0.8% agarose to identify a single band of the PCR amplified product at the position of 2.8 kb.

The DNA was recovered by using Qia Quick Gel Extraction Kit (Qiagen) and subcloned into T-cloning site of pT7Blue T-vector (Novagen) to determine its nulceotide sequence. By using the thus-obtained plasmid DNA (pTB1984) as a template, and by using oligo DNA synthesized with a DNA synthesizer (Oligo 1000M, Beckman) in addition to two commercially available primer DNA (PRM-007 and PRM-008) (Toyobo) as primer DNA, sequence reactions were carried out with ABI PRISM™ Dye Terminator Cycle Sequencing FS Ready Reaction Kit (Prekin Elmer) and GeneAmp$^{R}$ PCR System 2400 under the conditions prescribed in the protocol attached to the kit. Then, the sample was analyzed by a DNA Sequencer 373A (Perkin Elmer). The nucleotide sequence obtained was further analyzed by a gene analyzing soft, Lasergene (DNASTAR). As a result, a DNA fragment of a nucleotide sequence composed of 2781 bases containing an open reading frame of the nucleotide sequence composed of 1842 bases represented by SEQ ID NO: 2 which encodes the novel receptor protein composed of 616 amino acids represented by SEQ ID NO: 1 was contained at the T-cloning site of the clone (Fig. 1). According to the hydrophobic plotting analysis (Fig. 2), in this receptor protein, it has been deduced that the region of up to the 29th amino acid (Gln) is the secretion signal and the hydrophobic region of from the 211th amino acid (Leu) to the 234th amino acid (Cys) is a hydrophobic transmembrane region, i.e., so-called type I membrane protein.

This receptor protein had a highest homology to human CD40 precursor protein among known proteins, but the homology was 29% based on the amino acid level. The region from the 30th amino acid (Ile) to the 210th amino acid (Tyr) of this protein was deduced to be the extracellular region and contained cysteine-rich repeats which are a common characteristic region to members of TNF receptor family. Then, it has been defined that the receptor protein is a novel protein of TNF receptor family proteins.

The plasmid pTB1984 having the DNA encoding the receptor protein of the present invention derived from human dendritic cell (hereinafter sometimes abbreviated as DC2) was introduced into *Escherichia coli* to obtain the transformant, *Escherichia coli* DH10B/pTB1984.

Example 2

Preparation of anti-DC2 antiserum

The peptide represented by SEQ ID NO: 13 (A-789) composed of the amino acid sequence from the 185th amino acid (His) to the 206th amino acid (Glu) of the protein of the present invention represented by SEQ ID NO: 1 and added to the carboxyl group of cysteine, and the peptide represented by SEQ ID NO: 14 (A-782) composed of the amino acid sequence from the 64th amino acid (Ser) to the 85th amino acid (Lys) of SEQ ID NO: 1 and added to the carboxyl group of cysteine were chemically synthesized according to a per se known method, respectively. The peptide was bound to KLH (keyhole limpet hemocyanin) and then it (corresponding to 1 mg of the antigen peptide) was injected to two rabbits (SPF, Japanese White Rabbit) subcutaneously together with Freund's complete adjuvant (FCA) to provide primary immunization. Immunization was repeated once every 2 weeks, in total 6 times. After 3 months, whole blood was collected and the serum fraction was obtained by a per se known method to prepare anti-DC2 antiserum. The antibody titer was confirmed by a dot-blotting analysis to the synthesized peptide used as the immunogen (the secondary antibody was alkaline phosphatase-labeled anti-rabbit IgG antibody) and ELISA (the secondary antibody was peroxidase-labeled anti-rabbit IgG antibody).

The results of the dot-blotting analysis obtain by each antiserum (1,000-fold dilution) are shown in Table 1.

Table 1

| Peptide sample/antiserum | 50ng peptide/dot | 5ng peptide/dot | 0.5ng peptide/dot |
|---|---|---|---|
| A-789/anti-A-789 | +++ | + | - |
| A-782/anti-A-789 | - | - | - |
| A-789/anti-A-782 | - | - | - |
| A-782/anti-A-782 | +++ | + | - |

As seen from Table 1, each synthesized immunogen peptide of as little as 5 ng was specifically detected by respective samples.

A-789 CHGTEKSDVVCSSSLPARKPPNE (SEQ ID NO: 13)

A-782 CSDSVCLPCGPDEYLDSWNEEDK (SEQ ID NO: 14)

Example 3

Expression of the recombinant receptor protein and soluble recombinant receptor protein of the present invention in COS cells

For expression of the receptor protein encoded by the DNA obtained in Example 1 in animal cells, an expression vector plasmid, pcDNA3.1(-)/Myc-His B (Invitrogen) is used. This vector has an enhancer promoter of cytomegalovirus (CMV) together with a multi-cloning site at the downstream therefrom and a nucleotide sequence encoding a histidine tag at the downstream from the multi-cloning site. Then, the desired receptor protein can be produced as a fused protein having a histidine tag. A high expression level of the desired receptor protein in COS cells is expected by using this plasmid.

The plasmid is recovered from the transformant, *Escherichia coli* DH10B/pTB1984 obtained by introduction of the plasmid pTB1984 having the cDNA encoding the receptor protein of the present invention according to a known method and PCR is carried out by using this plasmid as a template to isolate a DNA fragment encoding the entire receptor protein of the present invention and a DNA fragment encoding the soluble receptor protein, respectively.

First, the DNA fragment encoding the entire receptor protein is amplified as follows. PCR is carried out by using the plasmid pTB1983 as a template, by using two synthetic oligonucleotides, that is, (a) a synthetic oligonucleotide corresponding to the amino terminal region of the open reading frame having a sequence which can be cleaved with EcoRI at the upstream from its initiation codon:

5'-GAATTCATGGCCCCGCGCGCC-3' (SEQ ID NO: 9)

and, (b) a synthetic oligonucleotide complementary to the carboxy terminal region of the open reading frame having a sequence which can be cleaved with XbaI in the sequence :

5'-TTCTAGAGCCTTGGCCCCGCC-3' (SEQ ID NO: 10)

as primers, and by using Takara LA PCR™ Kit Ver. 2 (Takara Shuzo) and Thermalcycler GeneAmp™ PCR system 2400 (Perkin Elmer). The temperature cycle program was first 1 minute at 94°C; and then 20 seconds at 98°C and 15 minutes at 68°C, for 30 cycles to amplify the DNA fragment. After digesting the amino terminus and carboxy terminus of the DNA fragment obtained with the restriction enzymes EcoRI and XbaI, the reaction mixture was subjected to electrophoresis with 1% agarose to identify the desired DNA fragment. Then, the DNA fragment is recovered and purified by Qia Quick Gel Extraction Kit (Qiagen). The DNA fragment thus obtained is named F1.

The DNA fragment encoding the soluble receptor protein is amplified as follows. PCR is carried out by using the plasmid pTB1983 as a template, by using a synthetic oligonucleotide complementary to the nucleotide sequence encoding the carboxyl terminus of the extracellular region of the receptor protein of the present invention to which a sequence which can be cleaved by XbaI is joined:

5'-TTCTAGAAAGTAAACATGGGG-3' (SEQ ID NO: 11)

and, the above-described synthetic oligonucleotide (a) (SEQ ID NO: 9) as primers under the same conditions as described above to amplify the DNA fragment. After digesting the amino terminus and carboxy terminus of the DNA fragment obtained with the restriction enzymes EcoRI and XbaI, the reaction mixture was subjected to electrophoresis with 1% agarose to identify the desired DNA fragment. Then, the DNA fragment is recovered and purified by Qia Quick Gel Extraction Kit (Qiagen). The DNA fragment thus obtained is named F2.

On the other hand, pcDNA3.1(-)/Myc-His B is linearized with the restriction enzymes EcoRI and XbaI and then treated with alkaline phosphatase to dephosphorylate the terminal phosphate group. After the reaction, the plasmid DNA is subjected to electrophoresis with 1% agarose to identify the desired DNA fragment. The DNA fragment is recovered and purified with Qia Quick Gel Extraction Kit (Qiagen). The vector DNA thus obtained is named V1.

The DNA fragments F1 or F2 is inserted and ligated to the vector by T4 DNA ligase according to a known method and then introduced into *Escherichia coli* DH5α to obtain ampicillin resistant strains and the desired plasmid DNA is selected and isolated. The nucleotide sequence of the cloned DNA fragment is identified by carrying out the DNA sequencing according to the same manner as described in Example 1. The resultant recombinant plasmid into which the DNA fragment F1 is inserted is named P1. The resultant recombinant plasmid into which the DNA fragment F2 is inserted is named P2.

COS cells (IFO, Osaka) are cultivated in DMEM medium (BIOWHITTAKER) containing 10% fetal bovine serum (FBS) until they are reached 50% confluent. The recombinant plasmid P1 or P2 is introduced into the COS cells by using Transfectum (Nippon Gene) . After incubating the COS cells in the absence of FBS at 37°C for 4 hours in 5% $CO_2$, the FBS is added to the COS cells at the final concentration of 10%. After incubating for additional 20 hours, the medium is changed to serum free DMEM medium. After cultivation for 3 days, the culture supernatant is collected and the cells are lysed with RIPA buffer (150 mM NaCl, 1% NP-40, 0.1% SDS, 0.5% DOC, 40 mM Tris-HCl (pH 7.5)) . The resultant cell lysate is centrifuged at 10,000 × g for 10 minutes and the supernatant is recovered as a crude extract. The expression of the desired protein in the culture supernatant or the crude extract is confirmed by a western blot analysis using the anti-DC2 antiserum obtained in Example 2, an antibody specific to the histidine tag, and Anti-His (C-term) (Invitrogen). The purification of the desired protein is carried out by affinity chromatography using a nickel chelate column.

Example 4

Expression of the recombinant receptor protein of the present invention using a baculovirus expression system

The expression of the receptor protein of the present invention in insect cells can be carried out by using Bac-To-Bac baculovirus expression system (Gibco BRL). This system comprises the following four steps.

(1) The desired gene is inserted into the donor plasmid, pFastBac1 and the resultant recombinant plasmid is introduced into *Escherichia coli* DH10Bac.
(2) The desired gene inserted into the recombinant plasmid is transferred into the baculovirus vector (bacmid) contained in DH10Bac.
(3) After selection of an *Escherichia coli* strain containing the recombinant bacmid into which the desired gene is inserted, the bacmid DNA is isolated from the cells.
(4) Insect cells are infected with the resultant bacmid DNA to express the desired gene.

The DNA fragment encoding the receptor protein of the present invention is amplified as follows. The DNA fragment encoding the soluble receptor protein is amplified as follows. PCR is carried out by using the recombinant plasmid P1 constructed in Example 3 as a template, by using the synthetic oligonucleotide (a) used in Example 3 (SEQ ID NO: 9) and a synthetic oligonucleotide designed in such a manner that it is complementary to the carboxyl terminal region of the open reading frame of the gene and has a sequence which can be cleaved by HindIII in the sequence:

5'-AAGCTTTCAATGATGATGATG-3' (SEQ ID NO: 12)

as primers, and by using Takara LA PCR™ Kit Ver. 2 (Takara Shuzo) and Thermalcycler GeneAmp™ PCR system 2400 (Perkin Elmer). The temperature cycle program was first 1 minute at 94°C; and then 20 seconds at 98°C and 15 minutes at 68°C, for 30 cycles to amplify the DNA fragment. After digesting the amino terminus and carboxy terminus of the DNA fragment obtained with the restriction enzymes EcoRI and HindIII, the reaction mixture was subjected to electrophoresis with 1% agarose to identify the desired DNA fragment. Then, the DNA fragment is recovered and purified by Qia Quick Gel Extraction Kit (Qiagen). The DNA fragment thus obtained is named F3.

On the other hand, pFastBacI to be used as a donor plasmid is changed to a linear strand with the restriction enzymes EcoRI and HindIII and then treated with alkaline phosphatase to dephosphorylate the terminal phosphate group. After the reaction, the plasmid DNA is subjected to electrophoresis with 1% agarose to identify the desired DNA fragment. The DNA fragment is recovered and purified by Qia Quick Gel Extraction Kit (Qiagen) . The vector DNA thus obtained is named V2.

The DNA fragment F3 is inserted and joined to the vector by T4 DNA ligase according to a known method and then inserted into *Escherichia coli* DH5α to obtain ampicillin resistant strains and the desired plasmid DNA is selected and isolated. The nucleotide sequence of the cloned DNA fragment and the insertion form are identified by carrying out the DNA sequencing according to the same manner as described in Example 1. The resultant recombinant plasmid into which the DNA encoding the receptor protein of the present invention is inserted is named P3.

Next, for transferring F3 DNA inserted into the plasmid P3 into a bacmid, the plasmid is introduced into *Escherichia coli* DH10Bac. First, 1 ng of the plasmid P3 is added to 100 μl of DH10Bac competent cell suspension. The suspension is allowed to stand for 30 minutes in ice, warmed at 42°C for 45 seconds and then allowed to stand for additional 2 minutes on ice. Then, 900 μl of SOC medium is added and the mixture is incubated at 37°C for 4 hours. After cultivation, *Escherichia coli* is diluted to each concentration and applied to Luria agar medium containing 50 μg/ml of kanamycin, 7 μg/ml of gentamicin, 10 μg/ml of tetracycline, 100 μg/ml of Bluo-gal (Gibco BRL) and 40 μg/ml of IPTG and incubated at 37° C overnight. Among the colonies appeared, white colonies are selected. The resultant clone having the recombinant bacmid is inoculated into 2 ml of LB medium containing 50 μg/ml of kanamycin, 7 μg/ml of gentamicin, 10 μg/ml of tetracycline and incubated at 37°C overnight.

Then, the bacmid is isolated from the cells and purified according to the following manner. First, to the cells recovered from the culture broth by centrifugation are added 0.3 ml of Solution I (15 mM Tris-HCl buffer pH 8.0, 10 mM EDTA, 100 μg/ml Rnase A) and 0.3 ml of Solution II (0.2 N sodium hydroxide, 1% SDS) and the mixture is mixed gently and allowed to stand at room temperature for 5 minutes. Then, 0.3 ml of 3 M potassium acetate (pH 5.5) is added and the mixture is stirred and allowed to stand for 10 minutes on ice. After centrifugation at room temperature at 14,000 × g for 15 minutes, the supernatant is recovered and 0.8 ml of isopropanol is added thereto. After the mixture is stirred several times and allowed to stand for 10 minutes, the mixture is subjected to centrifugation at room temperature at 14,000 × g for 15 minutes. The supernatant is removed and 0.5 ml of ethanol is added. The mixture is stirred several times and further subjected to 50 μg/ml of kanamycin, 7 μg/ml of gentamicin, 10 μg/ml of tetracycline. The supernatant is removed and the pellet is dried by allowing to stand at room temperature for 10 minutes. Then, it is dissolved in 40 μl of TE buffer to obtain a recombinant bacmid DNA solution.

The recombinant bacmid DNA is transduced into the insect cell line Sf9 according to the following method to express the desired protein. First, cellfectin reagent (Gibco BRL) diluted with 100 μl of SF-900II SFM medium (Gibco BRL) free from an antibiotic (50 units/ml penicillin, 50 μg/ml streptomycin) is thoroughly mixed and allowed to stand at room temperature for 45 hours. To this is added 0.8 ml of SF-900II SFM medium to obtain 1 ml of a lipid-DNA solution. On the other hand, $9 \times 10^5$ cells of Sf9 cell is seeded on a plastic 35 mm culture dish and incubated in 2 ml of Sf-900II SFM medium containing the antibiotic (50 units/ml penicillin, 50 μg/ml streptomycin) at 27°C for 1 hour and washed once with 2 ml of Sf-900II SFM medium free from the antibiotic. To the cells are added 1 ml of the above-prepared lipid-DNA solution and the mixture is incubated at 27°C for 5 hours. The solution was removed and 2 ml of Sf-900II SFM medium containing the antibiotic (50 units/ml penicillin, 50 μg/ml streptomycin). The cells are incubated for 2 days.

The cells thus obtained are recovered by centrifugation and lysed with 5 ml of a lysis solution (50 mM Tris-HCl (pH 8.5), 10 mM 2-mercaptoethanol, 1 mM PMSF, 1% NP-40). The resultant mixture is subjected to centrifugation at 10,000 × g for 10 minutes and the supernatant is recovered as a crude extract. The expression of the desired protein in the resultant crude extract is identified by a western blot analysis using the anti-DC2 antiserum obtained in Example 2, an antibody specific to the histidine tag, and Anti-HIs (C-term) (Invitorgen). The desired protein is purified by affinity chromatography using a nickel chelate column.

Example 5

Expression of the recombinant protein of the present invention in CHO cells

(1) Construction of expression vector

A DNA fragment encoding the entire receptor protein of the present invention is obtained by the following protocol. First, PCR is carried out by using the plasmid pTB1984 containing the DNA encoding the receptor protein obtained in Example 1 as a template, by using two synthetic oligonucleotides represented by SEQ ID NO: 15 and SEQ ID NO: 16:

5'-AGGATCCCCTGTCCCGCGCCATGGCC-3' (SEQ ID NO: 15)

and,

5'-AAAGCTTAAGCCTTGGCCCCGCCTTGCTCC-3' (SEQ ID NO: 16)

as primers, and by using Takara LA PCR™ Kit Ver. 2 (Takara Shuzo) and Thermalcycler GeneAmp™ PCR system 2400 (Perkin Elmer). The temperature cycle program was first 1 minute at 94°C; and then 20 seconds at 98°C, 20 seconds at 55°C and 4 minutes at 72°C, for 30 cycles to obtain an amplified product. After digesting with the restriction enzymes BamHI and HindIII, the reaction mixture was subjected to electrophoresis with 1% agarose to identify the desired DNA fragment. Then, the DNA fragment is recovered and purified by Qia Quick Gel Extraction Kit (Qiagen).

On the other hand, pcDNA3.1(-) (Invitorogen) to be used as the vector DNA is also digested and linearized with the restriction enzymes BamHI and HindIII and then the above purified DNA is introduced into and ligated to the vector with T4 DNA ligase. The vector is introduced into into *Escherichia coli* DH5α to obtain ampicillin resistant strains and the desired plasmid DNA is selected and isolated. The nucleotide sequence of the cloned DNA fragment is identified by carrying out the DNA sequencing according to the same manner as described in Example 1.

(2) Obtaining recombinant receptor protein-expressing CHO cells

CHS cells are cultivated on a 60 mm culture dish containing α-MEM medium (NIKKEN BIOMEDICAL LABORATORY) containing 10% FBS until they are reached $3 \times 10^5$ cells. The expression vector plasmid obtained in the above (1) is introduced into the CHO cells by using Transfectum used in Example 3. After incubating the CHO cells at 37°C for 24 hours in 5% $CO_2$, the cells are collected and distributed into a 96-well flat bottom culture plate. The CHO cells are further incubated at 37°C for 24 hours in 5% $CO_2$ and then G418 is added at the final concentration of 500 μg/ml. Incubation is further continued to select viable resistant strains. After cloning the resistant strains to obtain a single clone by limiting dilution, respective chromosomal DNAs are prepared. Genomic PCR is carried out by using the chromosomal DNA as a template, by using two synthetic oligonucleotides represented by SEQ ID NO: 17 and SEQ ID NO: 18:

5'-TTGCCCGGTTTAATAATTCTGCTTCTCTTC-3' (SEQ ID NO: 17)

and

5'-TGTATTCATCTTCTGTGGGCATCTGTCTG-3' (SEQ ID NO: 18)

as primers to select a candidate strain having the amplified DNA fragment of 418 base pairs derived from the DNA encoding the receptor protein. The expression of the desired protein in a crude extract or its membrane fraction is confirmed by a western blot analysis using the anti-DC2 antibody (e.g., the antiserum obtained in Example 2, etc.). The cell strain thus obtained is useful for investigating ligands of the receptor protein and screening of substances which promotes or inhibits the functions of the receptor protein.

As described hereinabove, the receptor protein, its partial peptide and their salts of the present invention are useful as a reagent for screening ligands, agonists, antagonists and the like. The against is useful as a prophylactic or therapeutic medicine of cancer, AIDS, infections and the like and the antagonist is useful as a prophylactic or therapeutic medicine of allergic immunological diseases, inflammatory, autoimmune disease, AIDS, glomerulonephritis, bronchial asthma and the like.

By using the antibody of the present invention, concentration of the receptor protein, its partial peptide or their salts in a specimen fluid can be assayed quantitatively.

## Claims

1. A receptor protein which comprises the same or substantially the same amino acid sequence as that represented by SEQ ID NO: 1, or its salt.

2. The receptor protein of claim 1, wherein the substantially the same amino acid sequence as that represented by SEQ ID NO: 1 is an amino acid sequence having at least about 40% homology to that represented by SEQ ID NO: 1, or its salt.

3. The receptor protein of claim 1, wherein the substantially the same amino acid sequence as that represented by SEQ ID NO: 1 is a variant of the amino acid sequence having a deletion, addition or substitution of 1 to 20 amino acids, its salt.

4. The receptor protein of claim 1 which is a receptor protein derived from dendritic cell and belonging to TNF receptor family.

5. A partial peptide of the protein of claim 1, or its salt.

6. A process for producing the receptor protein which comprises cultivating a transformant transformed with a recombinant vector containing a DNA encoding the receptor protein of claim 1 to form and accumulate the receptor protein and collecting the same.

7. An antibody against the receptor protein of claim 1, the partial peptide of claim 5 of their salts.

8. A method for determination of a ligand to the receptor protein of claim 1 or its salt which comprises using the receptor protein of claim 1, the partial peptide of claim 5 or their salts.

9. A method for screening a compound which alters binding properties between a ligand and the receptor protein of claim 1 or its salt, or a salt thereof, said method comprising using the receptor protein of claim 1, the partial peptide of claim 5 or their salts.

10. A reagent kit for screening a compound which alters binding properties between a ligand and the receptor protein of claim 1 or its salt, or a salt thereof, said kit comprising as an essential component the receptor protein of claim 1, the partial peptide of claim 5 or their salts.

11. A compound which alters binding properties between a ligand and the receptor protein of claim 1 or its salt, or a salt thereof whenever obtained by using the screening method of claim 9 or the screening kit of claim 10.

12. A pharmaceutical composition which comprises a compound which alters binding properties between a ligand and the receptor protein of claim 1 or its salt, or a salt thereof whenever obtained by using the screening method of claim 9 or the screening kit of claim 10.

13. The receptor protein or its salt of claim 1 for using determination of a ligand thereto.

14. The receptor protein or its salt of claim 1 for screening a compound which alters binding properties between a ligand and the receptor protein of claim 1.

15. The partial peptide or its salt of claim 5 for using determination of a ligand to the receptor protein of claim 1.

16. The partial peptide or its salt of claim 5 for screening a compound which alters binding properties between a ligand and the receptor protein of claim 1.

Fig. 1

GCCAGCCTGTCCCCGCCCATGGCCCCGCGCGCCCCGCGGCGCCGCCCCGCTGTTCGCGCTGCTGCTGCTCTGCGCGCTGCTCGCCCCGGCTGCAGGTGGCTTTGCAGATCGCTCCTCCATGT 120
Met Ala Pro Arg Ala Arg Arg Arg Arg Pro Leu Phe Ala Leu Leu Leu Leu Cys Ala Leu Leu Ala Arg Leu Gln Val Ala Leu Gln Ile Ala Pro Pro Cys

ACCAGTGACAAGCATTATGAGCATCTGGGACGGTGCTGTAACAAATGTGAACCAGGAAAGTACATGTCTTCTAAATGCACTACTACCTCTGACAGTGTATGTCTGCCCTGTGGCCCGGAT 240
Thr Ser Glu Lys His Tyr Glu His Leu Gly Arg Cys Cys Asn Lys Cys Glu Pro Gly Lys Tyr Met Ser Ser Lys Cys Thr Thr Thr Ser Asp Ser Val Cys Leu Pro Cys Gly Pro Asp

GAATACTTGGATAGCTGGAATGAAGAAGATAAATGCTTGCTGCATAAAGTTTGTGATACAGGCAAGGCCCTGGTGGCCGTGGTCGCCGGCAACAGCACGACCCCCCGGCGCTGCGCGTGC 360
Glu Tyr Leu Asp Ser Trp Asn Glu Glu Asp Lys Cys Leu Leu His Lys Val Cys Asp Thr Gly Lys Ala Leu Val Ala Val Val Ala Gly Asn Ser Thr Thr Pro Arg Arg Cys Ala Cys

ACGGCTGGGTACCACTGGAGCCAGGACTGCGAGTGCTGCCGCCGCAACACCGAGTGCGCGCCGGGCCTGGGCGCCCAGCACCCGTTGCAGCTCAACAAGGACACAGTGTGCAAACCTTGC 480
Thr Ala Gly Tyr His Trp Ser Gln Asp Cys Glu Cys Cys Arg Arg Asn Thr Glu Cys Ala Pro Gly Leu Gly Ala Gln His Pro Leu Gln Leu Asn Lys Asp Thr Val Cys Lys Pro Cys

CTTGCAGGCTACTTCTCTGATGCCTTTTCCTCCACGGACAAATGCAGACCCTGGACCAACTGTACCTTCCTTGGAAAGAGAGTAGAACATCATGGGACAGAGAAATCCGATGTGGTTTGC 600
Leu Ala Gly Tyr Phe Ser Asp Ala Phe Ser Ser Thr Asp Lys Cys Arg Pro Trp Thr Asn Cys Thr Phe Leu Gly Lys Arg Val Glu His His Gly Thr Glu Lys Ser Asp Val Val Cys

AGTTCTTCTCTGCCAGCTAGAAAACCACCAAATGAACCCCATGTTTACTTGCCCGGTTTAATAATTCTGCTTCTCTTCGCGTCTGTGGCCCTGGTGGCTGCCATCATCTTTGGCGTTTGC 720
Ser Ser Ser Leu Pro Ala Arg Lys Pro Pro Asn Glu Pro His Val Tyr Leu Pro Gly Leu Ile Ile Leu Leu Leu Phe Ala Ser Val Ala Leu Val Ala Ala Ile Ile Phe Gly Val Cys

TATAGGAAAAAAGGGAAAGCACTCACAGCTAATTTGTGGCACTGGATCAATGAGGCTTGTGGCCGCCTAAGTGGAGATAAGGAGTCCTCAGGTGACAGTTGTGTCAGTACACACACGGCA 840
Tyr Arg Lys Lys Gly Lys Ala Leu Thr Ala Asn Leu Trp His Trp Ile Asn Glu Ala Cys Gly Arg Leu Ser Gly Asp Lys Glu Ser Ser Gly Asp Ser Cys Val Ser Thr His Thr Ala

AACTTTGGTCAGCAGGGAGCATGTGAAGGTGTCTTACTGCTGACTCTGGAGGAGAAGACATTTCCAGAAGATATGTGCTACCCAGATCAAGGTGGTGTCTGTCAGGGCACGTGTGTAGGA 960
Asn Phe Gly Gln Gln Gly Ala Cys Glu Gly Val Leu Leu Leu Thr Leu Glu Glu Lys Thr Phe Pro Glu Asp Met Cys Tyr Pro Asp Gln Gly Gly Val Cys Gln Gly Thr Cys Val Gly

GGTGGTCCCTACGCACAAGGCGAAGATGCCAGGATGCTCTCATTGGTCAGCAAGACCGAGATAGAGGAAGACAGCTTCAGACAGATGCCCACAGAAGATGAATACATGGACAGGCCCTCC 1080
Gly Gly Pro Tyr Ala Gln Gly Glu Asp Ala Arg Met Leu Ser Leu Val Ser Lys Thr Glu Ile Glu Glu Asp Ser Phe Arg Gln Met Pro Thr Glu Asp Glu Tyr Met Asp Arg Pro Ser

CAGCCCACAGACCAGTTACTGTTCCTCACTGAGCCTGGAAGCAAATCCACACCTCCTTTCTCTGAACCCCTGGAGGTGGGGGAGAATGACAGTTTAAGCCAGTGCTTCACGGGGACACAG 1200
Gln Pro Thr Asp Gln Leu Leu Phe Leu Thr Glu Pro Gly Ser Lys Ser Thr Pro Pro Phe Ser Glu Pro Leu Glu Val Gly Glu Asn Asp Ser Leu Ser Gln Cys Phe Thr Gly Thr Gln

AGCACAGTGGGTTCAGAAAGCTGCAACTGCACTGAGCCCCTGTGCAGGACTGATTGGACTCCCATGTCCTCTGAAAACTACTTGCAAAAAGAGGTGGACAGTGGCCATTGCCCGCACTGG 1320
Ser Thr Val Gly Ser Glu Ser Cys Asn Cys Thr Glu Pro Leu Cys Arg Thr Asp Trp Thr Pro Met Ser Ser Glu Asn Tyr Leu Gln Lys Glu Val Asp Ser Gly His Cys Pro His Trp

GCAGCCAGCCCCAGCCCCAACTGGGCAGATGTCTGCACAGGCTGCCGGAACCCTCCTGGGGAGGACTGTGAACCCCTCGTGGGTTCCCCAAAACGTGGACCCTTGCCCCAGTGCGCCTAT 1440
Ala Ala Ser Pro Ser Pro Asn Trp Ala Asp Val Cys Thr Gly Cys Arg Asn Pro Pro Gly Glu Asp Cys Glu Pro Leu Val Gly Ser Pro Lys Arg Gly Pro Leu Pro Gln Cys Ala Tyr

Fig. 1 continued

```
GGCATGGGCCTTCCCCCTGAAGAAGAAGCCAGCAGGACGGAGGCCAGAGACCAGCCCGAGGATGGGGCTGATGGGAGCCTCCCAAGCTCAGCGAGGGCAGGTGCCGGGTCTGGAAGCTCC 1560
Gly Met Gly Leu Pro Pro Glu Glu Glu Ala Ser Arg Thr Glu Ala Arg Asp Gln Pro Glu Asp Gly Ala Asp Gly Arg Leu Pro Ser Ser Ala Arg Ala Gly Ala Gly Ser Gly Ser Ser

CCTGGTGGCCAGTCCCCTGCATCTGGAAATGTGACTGGAAACAGTAACTCCACGTTCATCTCCAGCGGGCAGGTGATGAACTTCAAGGGCGACATCATCGTGGTCTACGTCAGCCAGACC 1680
Pro Gly Gly Gln Ser Pro Ala Ser Gly Asn Val Thr Gly Asn Ser Asn Ser Thr Phe Ile Ser Ser Gly Gln Val Met Asn Phe Lys Gly Asp Ile Ile Val Val Tyr Val Ser Gln Thr

TCGCAGGAGGGCGCGGCGGCGGCTGCGGAGCCCATGGGCCGCCCGGTGCAGGAGGAGACCCTGGCGCGCCGAGACTCCTTCGCGGGGAACGGCCCGCGCTTCCCGGACCCGTGCGGCGGC 1800
Ser Gln Glu Gly Ala Ala Ala Ala Ala Glu Pro Met Gly Arg Pro Val Gln Glu Glu Thr Leu Ala Arg Arg Asp Ser Phe Ala Gly Asn Gly Pro Arg Phe Pro Asp Pro Cys Gly Gly

CCCGAGGGGCTGCGGGAGCCGGAGAAGGCCTCGAGGCCGGTGCAGGAGCAAGGCGGGGCCAAGGCTTGAGCGCCCCCCATGGCTGGGAGCCCGAAGCTCGGAGCCAGGGCTCGCGAGGGC 1920
Pro Glu Gly Leu Arg Glu Pro Glu Lys Ala Ser Arg Pro Val Gln Glu Gln Gly Gly Ala Lys Ala

AGCACCGCAGCCTCTGCCCCAGCCCCGGCCACCCAGGGATCGATCGGTACAGTCGAGGAAGACCACCCGGCATTCTCTGCCCACTTTGCCTTCCAGGAAATGGGCTTTTCAGCAAGTGAA 2040

TTGATGAGGACTGTCCCCATGCCCACGGATGCTCAGCAGCCCGCCGCACTGGGGCAGATGTCTCCCCTGCCACTCCTCAAACTCGCAGCAGTAATTTGTGGCACTATGACAGCTATTTTT 2160

ATGACTATCCTGTTCTGTGGGGGGGGGGTCTATGTTTTCCCCCCATATTTGTATTCCTTTTCATAACTTTTCTTGATATCTTTCCTCCCTCTTTTTTAATGTAAAGGTTTTCTCAAAAAT 2280

TCTCCTAAAGGTGAGGGTCTCTTTCTTTTCTCTTTTCCTTTTTTTTTTCTTTTTTTGGCAACCTGGCTCTGGCCCAGGCTAGAGTGCAGTGGTGCGATTATAGCCCCGCTGCAGCCTCTAA 2400

CTCCTGGGCTCAAGCAATCCAAGTGATCCTCCCACCTCAACCTTCGGAGTAGCTGGGATCACAGCTGCAGGCCACGCCCAGCTTCCTCCCCCCGACTCCCCCCCCAGACACACGGTCCCA 2520

CCATGTTACCCAGCCTGGTCTCAAACTCCCCAGCTAAAGCAGTCCTCCAGCCTCGGCCTCCCAAAGTACTGGGATTACAGGCGTGAGCCCCCACGCTGGCCTGCTTTACGTATTTTCTTT 2640

TGTGCCCCCTGCTCACAGTGTTTTAGAGATGGCTTTCCCAGTGTGTGTTCATTGTAAACACTTTTGGGAAAGGGCTAAACATGTGAGCCCTGGAGATAGTTGCTAAGTTGCTAGGAACATG 2760

TGGTGGGACTTTCATATTCTG 2781
```

Fig. 2

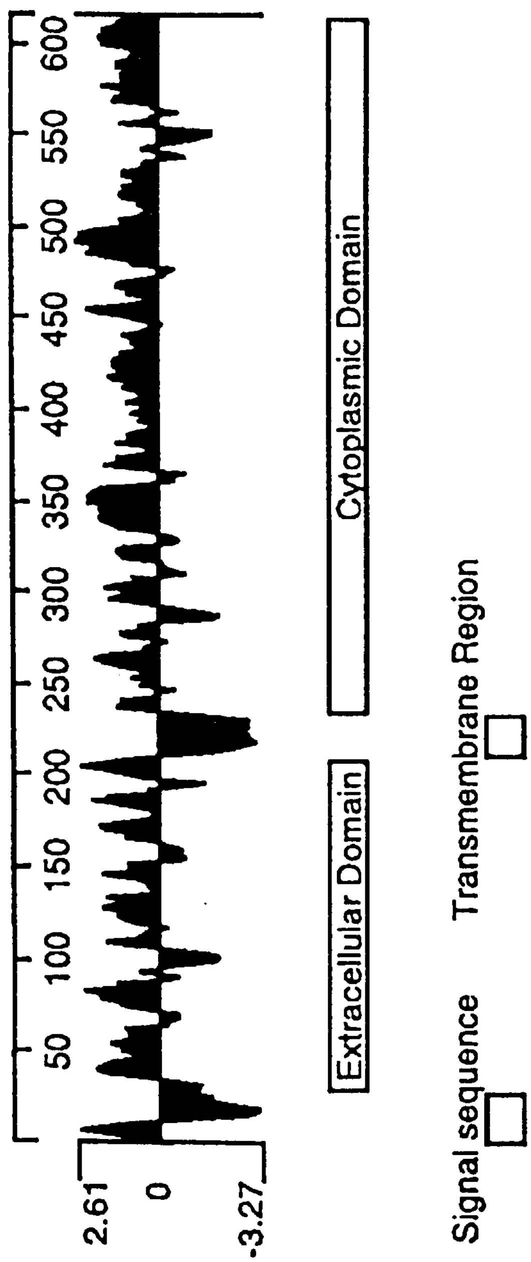

2.61
0
-3.27
50
100
150
200
250
300
350
400
450
500
550
600
Extracellular Domain
Cytoplasmic Domain
Signal sequence
Transmembrane Region